# EUROPEAN PATENT SPECIFICATION

(11) **EP 1 987 836 B1**
(45) Date of publication and mention of the grant of the patent: **21.12.2011**
(21) Application number: 07107512.1
(22) Date of filing: 04.05.2007
(51) Int. Cl.: A61K 38/16, A61K 39/085, C07K 14/31

(54) **Protective staphylococcus aureus vaccine based on cell wall-associated proteins**
Impfstoff zum Schutz gegen staphylococcus aureus auf der Basis zellwandassoziierter Proteine
Vaccin de protection contre le staphylococcus aureus basé sur des protéines liées à la paroi cellulaire

(43) Date of publication of application: 05.11.2008
(60) Divisional application: 11169342.0
(73) Proprietor: Krönke, Martin, 50968 Köln (DE); Krut, Oleg, 50354 Hürth (DE)
(72) Inventor: Krönke, Martin, 50968 Köln (DE); Krut, Oleg, 50354 Hürth (DE); Glowalla, Eva, 50937 Köln (DE); Tosetti, Bettina, 50733 Köln (DE)
(74) Representative: von Kreisler Selting Werner

(56) References cited:
- WO-A-2005/009379
- WO-A-2006/032500
- GARCIA-LARA J ET AL: "Staphylococcus aureus: the search for novel targets" DRUG DISCOVERY TODAY, ELSEVIER, RAHWAY, NJ, US, vol. 10, no. 9, 1 May 2005 (2005-05-01), pages 643-651, XP004890888 ISSN: 1359-6446
- PEREZ-CASAL ET AL: "Immune responses to a Staphylococcus aureus GapC/B chimera and its potential use as a component of a vaccine for S. aureus mastitis" VETERINARY IMMUNOLOGY AND IMMUNOPATHOLOGY, AMSTERDAM, NL, vol. 109, no. 1-2, 15 January 2006 (2006-01-15), pages 85-97, XP005196452 ISSN: 0165-2427
- GOTZ F: "Staphylococci in colonization and disease: prospective targets for drugs and vaccines" CURRENT OPINION IN MICROBIOLOGY, CURRENT BIOLOGY LTD, GB, vol. 7, no. 5, October 2004 (2004-10), pages 477-487, XP004586484 ISSN: 1369-5274
- DATABASE UniProt [Online] 21 March 2006 (2006-03-21), "Esterase-like protein. DVTQNN" XP002464954 retrieved from EBI accession no. UNIPROT:Q2FEE7 Database accession no. Q2FEE7
- MODUN B ET AL: "The staphylococcal transferrin-binding protein is a cell wall glyceraldehyde-3-phosphate dehydrogenase." INFECTION AND IMMUNITY MAR 1999, vol. 67, no. 3, March 1999 (1999-03), pages 1086-1092, XP009094534 ISSN: 0019-9567
- TAVERNA ET AL: "Characterization of cell wall associated proteins of a Staphylococcus aureus isolated from bovine mastitis case by a proteomic approach" VETERINARY MICROBIOLOGY, AMSTERDAM, NL, vol. 119, no. 2-4, 4 January 2007 (2007-01-04), pages 240-247, XP005821859 ISSN: 0378-1135

### SEQUENCE LISTING

<110> Kroenke, Martin Krut, Oleg
<120> Protective Staphylococcus Aureus Vaccine Based on Cell Wall-Associated Proteins
<130> 070570ep
<150> EP 06101431.2
   <151> 2006-02-08
<160> 84
<170> PatentIn version 3.3
<210> 1
   <211> 306
   <212> PRT
   <213> Staphylococcus aureus
<400> 1
   Met Ile Arg Asn Arg Val Met Asn Ser Val Val Asn Lys Tyr Leu Leu
   1               5                   10                  15
   His Asn Arg Ser Ile Met Phe Lys Asn Asp Gln Asp Val Glu Arg Phe
               20                  25                  30
   Phe Tyr Lys Arg Glu Ile Glu Asn Arg Lys Lys His Lys Gln Pro Ser
           35                  40                  45
   Thr Leu Asn Val Lys Ala Asn Leu Glu Lys Leu Ser Leu Asp Asp Met
       50                  55                  60
   Gln Val Phe Arg Phe Asn Phe Arg His Gln Ile Asp Lys Lys Ile Leu
   65                  70                  75                  80
   Tyr Ile His Gly Gly Phe Asn Ala Leu Gln Pro Ser Pro Phe His Trp
                   85                  90                  95
   Arg Leu Leu Asp Lys Ile Thr Leu Ser Thr Leu Tyr Glu Val Val Leu
               100                 105                 110
   Pro Ile Tyr Pro Lys Thr Pro Glu Phe His Ile Asp Asp Thr Phe Gln
           115                 120                 125
   Ala Ile Gln Arg Val Tyr Asp Gln Leu Val Ser Glu Val Gly His Gln
       130                 135                 140
   Asn Val Val Val Met Gly Asp Gly Ser Gly Gly Ala Leu Ala Leu Ser
   145                 150                 155                 160
   Phe Val Gln Ser Leu Leu Asp Asn Gln Gln Pro Leu Pro Asn Lys Leu
   165 170 175
   Tyr Leu Ile Ser Pro Ile Leu Asp Ala Thr Leu Ser Asn Lys Asp Ile
   180 185 190
   Ser Asp Ala Leu Ile Glu Gln Asp Ala Val Leu Ser Gln Phe Gly Val
   195 200 205
   Asn Glu Ile Met Lys Lys Trp Ala Asn Gly Leu Pro Leu Thr Asp Lys
   210 215 220
   Arg Ile Ser Pro Ile Asn Gly Thr Ile Glu Gly Leu Pro Pro Val Tyr
   225 230 235 240
   Met Phe Gly Gly Gly Arg Glu Met Thr His Pro Asp Met Lys Leu Phe
   245 250 255
   Glu Gln Met Met Leu Gln His His Gln Tyr Ile Glu Phe Tyr Asp Tyr
   260 265 270
   Pro Lys Met Val His Asp Phe Pro Ile Tyr Pro Ile Arg Gln Ser His
   275 280 285
   Lys Ala Ile Lys Gln Ile Ala Lys Ser Ile Asp Glu Asp Val Thr Gln
   290 295 300
   Asn Asn
   305
<210> 2
   <211> 434
   <212> PRT
   <213> Staphylococcus aureus
<400> 2
<210> 3
   <211> 246
   <212> PRT
   <213> Staphylococcus aureus
<400> 3
   Met Lys Met Thr Lys Ser Ala Leu Val Thr Gly Ala Ser Arg Gly Ile
   1               5                   10                  15
   Gly Arg Ser Ile Ala Leu Gln Leu Ala Glu Glu Gly Tyr Asn Val Ala
               20                  25                  30
   Val Asn Tyr Ala Gly Ser Lys Glu Lys Ala Glu Ala Val Val Glu Glu
           35                  40                  45
   Ile Lys Ala Lys Gly Val Asp Ser Phe Ala Ile Gln Ala Asn Val Ala
       50                  55                  60
   Asp Ala Asp Glu Val Lys Ala Met Ile Lys Glu Val Val Ser Gln Phe
   65                  70                  75                  80
   Gly Ser Leu Asp Val Leu Val Asn Asn Ala Gly Ile Thr Arg Asp Asn
                   85                  90                  95
   Leu Leu Met Arg Met Lys Glu Gln Glu Trp Asp Asp Val Ile Asp Thr
               100                 105                 110
   Asn Leu Lys Gly Val Phe Asn Cys Ile Gln Lys Ala Thr Pro Gln Met
           115                 120                 125
   Leu Arg Gln Arg Ser Gly Ala Ile Ile Asn Leu Ser Ser Val Val Gly
       130                 135                 140
   Ala Val Gly Asn Pro Gly Gln Ala Asn Tyr Val Ala Thr Lys Ala Gly
   145                 150                 155                 160
   Val Ile Gly Leu Thr Lys Ser Ala Ala Arg Glu Leu Ala Ser Arg Gly
                   165                 170                 175
   Ile Thr Val Asn Ala Val Ala Pro Gly Phe Ile Val Ser Asp Met Thr
               180                 185                 190
   Asp Ala Leu Ser Asp Glu Leu Lys Glu Gln Met Leu Thr Gln Ile Pro
           195                 200                 205
   Leu Ala Arg Phe Gly Gln Asp Thr Asp Ile Ala Asn Thr Val Ala Phe
       210                 215                 220
   Leu Ala Ser Asp Lys Ala Lys Tyr Ile Thr Gly Gln Thr Ile His Val
   225                 230                 235                 240
   Asn Gly Gly Met Tyr Met
                   245
<210> 4
   <211> 402
   <212> PRT
   <213> Staphylococcus aureus
<400> 4
   Met Ala Gln Asp Arg Lys Lys Val Leu Val Leu Gly Ala Gly Tyr Ala
   1               5                   10                  15
   Gly Leu Gln Thr Val Thr Lys Leu Gln Lys Ala Ile Ser Thr Glu Glu
               20                  25                  30
   Ala Glu Ile Thr Leu Ile Asn Lys Asn Glu Tyr His Tyr Glu Ala Thr
           35                  40                  45
   Trp Leu His Glu Ala Ser Ala Gly Thr Leu Asn Tyr Glu Asp Val Leu
       50                  55                  60
   Tyr Pro Val Glu Ser Val Leu Lys Lys Asp Lys Val Asn Phe Val Gln
   65 70 75 80
   Ala Glu Val Thr Lys Ile Asp Arg Asp Ala Lys Lys Val Glu Thr Asn
   85 90 95
   Gln Gly Ile Tyr Asp Phe Asp Ile Leu Val Val Ala Leu Gly Phe Val
   100 105 110
   Ser Glu Thr Phe Gly Ile Glu Gly Met Lys Asp His Ala Phe Gln Ile
   115 120 125
   Glu Asn Val Ile Thr Ala Arg Glu Leu Ser Arg His Ile Glu Asp Lys
   130 135 140
   Phe Ala Asn Tyr Ala Ala Ser Lys Glu Lys Asp Asp Asn Asp Leu Ser
   145 150 155 160
   Ile Leu Val Gly Gly Ala Gly Phe Thr Gly Val Glu Phe Leu Gly Glu
   165 170 175
   Leu Thr Asp Arg Ile Pro Glu Leu Cys Ser Lys Tyr Gly Val Asp Gln
   180 185 190
   Asn Lys Val Lys Ile Thr Cys Val Glu Ala Ala Pro Lys Met Leu Pro
   195 200 205
   Met Phe Ser Glu Glu Leu Val Asn His Ala Val Ser Tyr Leu Glu Asp
   210 215 220
   Arg Gly Val Glu Phe Lys Ile Ala Thr Pro Ile Val Ala Cys Asn Glu
   225 230 235 240
   Lys Gly Phe Val Val Glu Val Asp Gly Glu Lys Gln Gln Leu Asn Ala
   245 250 255
   Gly Thr Ser Val Trp Ala Ala Gly Val Arg Gly Ser Lys Leu Met Glu
   260 265 270
   Glu Ser Phe Glu Gly Val Lys Arg Gly Arg Ile Val Thr Lys Gln Asp
   275 280 285
   Leu Thr Ile Asn Gly Tyr Asp Asn Ile Phe Val Ile Gly Asp Cys Ser
   290 295 300
   Ala Phe Ile Pro Ala Gly Glu Glu Arg Pro Leu Pro Thr Thr Ala Gln
   305                 310                 315                 320
   Ile Ala Met Gln Gln Gly Glu Ser Val Ala Lys Asn Ile Lys Arg Ile
                   325                 330                 335
   Leu Asn Gly Glu Ser Thr Glu Glu Phe Glu Tyr Val Asp Arg Gly Thr
               340                 345                 350
   Val Cys Ser Leu Gly Ser His Asp Gly Val Gly Met Val Phe Gly Lys
           355                 360                 365
   Pro Ile Ala Gly Lys Lys Ala Ala Phe Met Lys Lys Val Ile Asp Thr
       370                 375                 380
   Arg Ala Val Phe Lys Ile Gly Gly Ile Gly Leu Ala Phe Lys Lys Gly
   385                 390                 395                 400
   Lys Phe
<210> 5
   <211> 646
   <212> PRT
   <213> Staphylococcus aureus
<400> 5
   Met Ser Ser Gln Lys Lys Lys Ile Ser Leu Phe Ala Phe Phe Leu Leu
   1               5                   10                  15
   Thr Val Ile Thr Ile Thr Leu Lys Thr Tyr Phe Ser Tyr Tyr Val Asp
               20                  25                  30
   Phe Ser Leu Gly Val Lys Gly Leu Val Gln Asn Leu Ile Leu Leu Met
           35                  40                  45
   Asn Pro Tyr Ser Leu Val Ala Leu Val Leu Ser Val Phe Leu Phe Phe
       50                  55                  60
   Lys Gly Lys Lys Ala Phe Trp Phe Met Phe Ile Gly Gly Phe Leu Leu
   65                  70                  75                  80
   Thr Phe Leu Leu Tyr Ala Asn Val Val Tyr Phe Arg Phe Phe Ser Asp
                   85                  90                  95
   Phe Leu Thr Phe Ser Thr Leu Asn Gln Val Gly Asn Val Glu Ser Met
               100                 105                 110
   Gly Gly Ala Val Ser Ala Ser Phe Lys Trp Tyr Asp Phe Val Tyr Phe
           115                 120                 125
   Ile Asp Thr Leu Val Tyr Leu Phe Ile Leu Ile Phe Lys Thr Lys Trp
       130                 135                 140
   Leu Asp Thr Lys Ala Phe Ser Lys Lys Phe Val Pro Val Val Met Ala
   145                 150                 155                 160
   Ala Ser Val Ala Leu Phe Phe Leu Asn Leu Ala Phe Ala Glu Thr Asp
                   165                 170                 175
   Arg Pro Glu Leu Leu Thr Arg Thr Phe Asp His Lys Tyr Leu Val Lys
               180                 185                 190
   Tyr Leu Gly Pro Tyr Asn Phe Thr Val Tyr Asp Gly Val Lys Thr Ile
           195                 200                 205
   Glu Asn Asn Gln Gln Lys Ala Leu Ala Ser Glu Asp Asp Leu Thr Lys
       210                 215                 220
   Val Leu Asn Tyr Thr Lys Gln Arg Gln Thr Glu Pro Asn Pro Glu Tyr
   225                 230                 235                 240
   Tyr Gly Val Ala Lys Lys Lys Asn Ile Ile Lys Ile His Leu Glu Ser
                   245                 250                 255
   Phe Gln Thr Phe Leu Ile Asn Lys Lys Val Asn Gly Lys Glu Val Thr
               260                 265                 270
   Pro Phe Leu Asn Lys Leu Ser Ser Gly Lys Glu Gln Phe Thr Tyr Phe
           275                 280                 285
   Pro Asn Phe Phe His Gln Thr Gly Gln Gly Lys Thr Ser Asp Ser Glu
       290                 295                 300
   Phe Thr Met Asp Asn Ser Leu Tyr Gly Leu Pro Gln Gly Ser Ala Phe
   305                 310                 315                 320
   Ser Leu Lys Gly Asp Asn Thr Tyr Gln Ser Leu Pro Ala Ile Leu Asp
                   325                 330                 335
   Gln Lys Gln Gly Tyr Lys Ser Asp Val Met His Gly Asp Tyr Lys Thr
               340                 345                 350
   Phe Trp Asn Arg Asp Gln Val Tyr Lys His Phe Gly Ile Asp Lys Phe
           355                 360                 365
   Tyr Asp Ala Thr Tyr Tyr Asp Met Ser Asp Lys Asn Val Val Asn Leu
   370 375 380
   Gly Leu Lys Asp Lys Ile Phe Phe Lys Asp Ser Ala Asn Tyr Gln Ala
   385 390 395 400
   Lys Met Lys Ser Pro Phe Tyr Ser His Leu Ile Thr Leu Thr Asn His
   405 410 415
   Tyr Pro Phe Thr Leu Asp Glu Lys Asp Ala Thr Ile Glu Lys Ser Asn
   420 425 430
   Thr Gly Asp Ala Thr Val Asp Gly Tyr Ile Gln Thr Ala Arg Tyr Leu
   435 440 445
   Asp Glu Ala Leu Glu Glu Tyr Ile Asn Asp Leu Lys Lys Lys Gly Leu
   450 455 460
   Tyr Asp Asn Ser Val Ile Met Ile Tyr Gly Asp His Tyr Gly Ile Ser
   465 470 475 480
   Glu Asn His Asn Asn Ala Met Glu Lys Leu Leu Gly Glu Lys Ile Thr
   485 490 495
   Pro Ala Lys Phe Thr Asp Leu Asn Arg Thr Gly Phe Trp Ile Lys Ile
   500 505 510
   Pro Gly Lys Ser Gly Gly Ile Asn Asn Glu Tyr Ala Gly Gln Val Asp
   515 520 525
   Val Met Pro Thr Ile Leu His Leu Ala Gly Ile Asp Thr Lys Asn Tyr
   530 535 540
   Leu Met Phe Gly Thr Asp Leu Phe Ser Lys Gly His Asn Gln Val Val
   545 550 555 560
   Pro Phe Arg Asn Gly Asp Phe Ile Thr Lys Asp Tyr Lys Tyr Val Asn
   565 570 575
   Gly Lys Ile Tyr Ser Asn Lys Asn Asn Glu Leu Ile Thr Thr Gln Pro
   580 585 590
   Ala Asp Phe Glu Lys Asn Lys Lys Gln Val Glu Lys Asp Leu Glu Met
   595 600 605
   Ser Asp Asn Val Leu Asn Gly Asp Leu Phe Arg Phe Tyr Lys Asn Pro
   610 615 620
   Asp Phe Lys Lys Val Asn Pro Ser Lys Tyr Lys Tyr Glu Thr Gly Pro
   625                 630                 635                 640
   Lys Ala Asn Ser Lys Lys
                   645
<210> 6
   <211> 322
   <212> PRT
   <213> Staphylococcus aureus
<400> 6
   Met Glu Asp Val Leu Tyr Met Lys Lys Leu Thr Ala Ala Ala Ile Ala
   1               5                   10                  15
   Thr Met Gly Phe Ala Thr Phe Thr Met Ala His Gln Ala Asp Ser Ala
               20                  25                  30
   Glu Thr Thr Asn Thr Gln Gln Ala His Thr Gln Met Ser Thr Gln Ser
           35                  40                  45
   Gln Asp Val Ser Tyr Gly Thr Tyr Tyr Thr Ile Asp Ser Asn Gly Asp
       50                  55                  60
   Tyr His His Thr Pro Asp Gly Asn Trp Asn Gln Ala Met Phe Asp Asn
   65                  70                  75                  80
   Lys Glu Tyr Ser Tyr Thr Phe Val Asp Ala Gln Gly His Thr His Tyr
                   85                  90                  95
   Phe Tyr Asn Cys Tyr Pro Lys Asn Ala Asn Ala Asn Gly Ser Gly Gln
               100                 105                 110
   Thr Tyr Val Asn Pro Ala Thr Ala Gly Asp Asn Asn Asp Tyr Thr Ala
           115                 120                 125
   Ser Gln Ser Gln Gln His Ile Asn Gln Tyr Gly Tyr Gln Ser Asn Val
       130                 135                 140
   Gly Pro Asp Ala Ser Tyr Tyr Ser His Ser Asn Asn Asn Gln Ala Tyr
   145                 150                 155                 160
   Asn Ser His Asp Gly Asn Gly Lys Val Asn Tyr Pro Asn Gly Thr Ser
                   165                 170                 175
   Asn Gln Asn Gly Gly Ser Ala Ser Lys Ala Thr Arg Ser Gly His Ala
               180                 185                 190
   Lys Asp Ala Ser Trp Leu Thr Ser Arg Lys Gln Leu Gln Pro Tyr Gly
           195                 200                 205
   Gln Tyr His Gly Gly Gly Ala His Tyr Gly Val Asp Tyr Ala Met Pro
       210                 215                 220
   Glu Asn Ser Pro Val Tyr Ser Leu Thr Asp Gly Thr Val Val Gln Ala
   225                 230                 235                 240
   Gly Trp Ser Asn Tyr Gly Gly Gly Asn Gln Val Thr Ile Lys Glu Ala
                   245                 250                 255
   Asn Ser Asn Asn Tyr Gln Trp Tyr Met His Asn Asn Arg Leu Thr Val
               260                 265                 270
   Ser Ala Gly Asp Lys Val Lys Ala Gly Asp Gln Ile Ala Tyr Ser Gly
           275                 280                 285
   Ser Thr Gly Asn Ser Thr Ala Pro His Val His Phe Gln Arg Met Ser
       290                 295                 300
   Gly Gly Ile Gly Asn Gln Tyr Ala Val Asp Pro Thr Ser Tyr Leu Gln
   305                 310                 315                 320
   Ser Arg
<210> 7
   <211> 274
   <212> PRT
   <213> Staphylococcus aureus
<400> 7
   Met Gln Pro His Leu Ile Cys Leu Asp Leu Asp Gly Thr Leu Leu Asn
   1               5                   10                  15
   Asp Asn Lys Glu Ile Ser Ser Tyr Thr Lys Gln Val Leu Asn Glu Leu
               20                  25                  30
   Gln Gln Arg Gly His Gln Ile Met Ile Ala Thr Gly Arg Pro Tyr Arg
           35                  40                  45
   Ala Ser Gln Met Tyr Tyr His Glu Leu Asn Leu Thr Thr Pro Ile Val
       50                  55                  60
   Asn Phe Asn Gly Ala Tyr Val His His Pro Lys Asp Lys Asn Phe Lys
   65                  70                  75                  80
   Thr Cys His Glu Ile Leu Asp Leu Gly Ile Ala Gln Asn Ile Ile Gln
                   85                  90                  95
   Gly Leu Gln Gln Tyr Gln Val Ser Asn Ile Ile Ala Glu Val Lys Asp
               100                 105                 110
   Tyr Val Phe Ile Asn Asn His Asp Pro Arg Leu Phe Glu Gly Phe Ser
           115                 120                 125
   Met Gly Asn Pro Arg Ile Gln Thr Gly Asn Leu Leu Val His Leu Lys
       130                 135                 140
   Glu Ser Pro Thr Ser Ile Leu Ile Glu Ala Glu Glu Ser Lys Ile Pro
   145                 150                 155                 160
   Glu Ile Lys Asn Met Leu Thr His Phe Tyr Ala Asp His Ile Glu His
                   165                 170                 175
   Arg Arg Trp Gly Ala Pro Phe Pro Val Ile Glu Ile Val Lys Leu Gly
               180                 185                 190
   Ile Asn Lys Ala Arg Gly Ile Glu Gln Val Arg Gln Phe Leu Asn Ile
           195                 200                 205
   Asp Arg Asn Asn Ile Ile Ala Phe Gly Asp Glu Asp Asn Asp Ile Glu
       210                 215                 220
   Met Ile Glu Tyr Ala Arg His Gly Val Ala Met Glu Asn Gly Leu Gln
   225                 230                 235                 240
   Glu Leu Lys Asp Val Ala Asn Asn Ile Thr Phe Asn Asn Asn Glu Asp
                   245                 250                 255
   Gly Ile Gly Arg Tyr Leu Asn Asp Phe Phe Asn Leu Asn Ile Arg Tyr
               260                 265                 270
   Tyr Cys
<210> 8
   <211> 233
   <212> PRT
   <213> Staphylococcus aureus
<400> 8
   Met Lys Lys Thr Ile Met Ala Ser Ser Leu Ala Val Ala Leu Gly Val
   1               5                   10                  15
   Thr Gly Tyr Ala Ala Gly Thr Gly His Gln Ala His Ala Ala Glu Val
               20                  25                  30
   Asn Val Asp Gln Ala His Leu Val Asp Leu Ala His Asn His Gln Asp
           35                  40                  45
   Gln Leu Asn Ala Ala Pro Ile Lys Asp Gly Ala Tyr Asp Ile His Phe
       50                  55                  60
   Val Lys Asp Gly Phe Gln Tyr Asn Phe Thr Ser Asn Gly Thr Thr Trp
   65                  70                  75                  80
   Ser Trp Ser Tyr Glu Ala Ala Asn Gly Gln Thr Ala Gly Phe Ser Asn
                   85                  90                  95
   Val Ala Gly Ala Asp Tyr Thr Thr Ser Tyr Asn Gln Gly Ser Asp Val
               100                 105                 110
   Gln Ser Val Ser Tyr Asn Ala Gln Ser Ser Asn Ser Asn Val Glu Ala
           115                 120                 125
   Val Ser Ala Pro Thr Tyr His Asn Tyr Ser Thr Ser Thr Thr Ser Ser
       130                 135                 140
   Ser Val Arg Leu Ser Asn Gly Asn Thr Ala Gly Ala Thr Gly Ser Ser
   145                 150                 155                 160
   Ala Ala Gln Ile Met Ala Gln Arg Thr Gly Val Ser Ala Ser Thr Trp
                   165                 170                 175
   Ala Ala Ile Ile Ala Arg Glu Ser Asn Gly Gln Val Asn Ala Tyr Asn
               180                 185                 190
   Pro Ser Gly Ala Ser Gly Leu Phe Gln Thr Met Pro Gly Trp Gly Pro
           195                 200                 205
   Thr Asn Thr Val Asp Gln Gln Ile Asn Ala Ala Val Lys Ala Tyr Lys
       210                 215                 220
   Ala Gln Gly Leu Gly Ala Trp Gly Phe
   225                 230
<210> 9
   <211> 459
   <212> PRT
   <213> Staphylococcus aureus
<400> 9
   Met Asn Ile Ile Glu Gln Lys Phe Tyr Asp Ser Lys Ala Phe Phe Asn
   1               5                   10                  15
   Thr Gln Gln Thr Lys Asp Ile Ser Phe Arg Lys Asp Gln Leu Lys Lys
               20                  25                  30
   Leu Ser Lys Ala Ile Lys Ser Tyr Glu Ser Asp Ile Leu Glu Ala Leu
           35                  40                  45
   Tyr Thr Asp Leu Gly Lys Asn Lys Val Glu Ala Tyr Ala Thr Glu Ile
       50                  55                  60
   Gly Ile Thr Leu Lys Ser Ile Lys Asn Ala Arg Lys Glu Leu Lys Asn
   65                  70                  75                  80
   Trp Thr Lys Thr Lys Asn Val Asp Thr Pro Leu Tyr Leu Phe Pro Thr
                   85                  90                  95
   Lys Ser Tyr Ile Lys Lys Glu Pro Tyr Gly Thr Val Leu Ile Ile Ala
               100                 105                 110
   Pro Phe Asn Tyr Pro Phe Gln Leu Val Phe Glu Pro Leu Ile Gly Ala
           115                 120                 125
   Ile Ala Ala Gly Asn Thr Ala Ile Ile Lys Pro Ser Glu Leu Thr Pro
       130                 135                 140
   Asn Val Ala Arg Val Ile Lys Arg Leu Ile Asn Glu Thr Phe Asp Ala
   145                 150                 155                 160
   Asn Tyr Ile Glu Val Ile Glu Gly Gly Ile Glu Glu Thr Gln Thr Leu
                   165                 170                 175
   Ile His Leu Pro Phe Asp Tyr Val Phe Phe Thr Gly Ser Glu Asn Val
               180                 185                 190
   Gly Lys Ile Val Tyr Gln Ala Ala Ser Glu Asn Leu Val Pro Val Thr
           195                 200                 205
   Leu Glu Met Gly Gly Lys Ser Pro Val Ile Val Asp Glu Thr Ala Asn
       210                 215                 220
   Ile Lys Val Ala Ser Glu Arg Ile Cys Phe Gly Lys Phe Thr Asn Ala
   225                 230                 235                 240
   Gly Gln Thr Cys Val Ala Pro Asp Tyr Ile Leu Val His Glu Ser Val
                   245                 250                 255
   Lys Asp Asp Leu Ile Thr Ala Leu Ser Lys Thr Leu Arg Glu Phe Tyr
               260                 265                 270
   Gly Gln Asn Ile Gln Gln Ser Pro Asp Tyr Gly Arg Ile Val Asn Leu
           275                 280                 285
   Lys His Tyr His Arg Leu Thr Ser Leu Leu Asn Ser Ala Gln Met Asn
       290                 295                 300
   Ile Val Phe Gly Gly His Ser Asp Glu Asp Glu Arg Tyr Ile Glu Pro
   305                 310                 315                 320
   Thr Leu Leu Asp His Val Thr Ser Asp Ser Ala Ile Met Gln Glu Glu
                   325                 330                 335
   Ile Phe Gly Pro Ile Leu Pro Ile Leu Thr Tyr Gln Ser Leu Asp Glu
               340                 345                 350
   Ala Ile Ala Phe Ile His Gln Arg Pro Lys Pro Leu Ser Leu Tyr Leu
           355                 360                 365
   Phe Ser Glu Asp Glu Asn Ala Thr Gln Arg Val Ile Asn Glu Leu Ser
       370                 375                 380
   Phe Gly Gly Gly Ala Ile Asn Asp Thr Leu Met His Leu Ala Asn Pro
   385                 390                 395                 400
   Lys Leu Pro Phe Gly Gly Val Gly Ala Ser Gly Met Gly Arg Tyr His
                   405                 410                 415
   Gly Lys Tyr Ser Phe Asp Thr Phe Thr His Glu Lys Ser Tyr Ile Phe
               420                 425                 430
   Lys Ser Thr Arg Leu Glu Ser Gly Val His Leu Pro Pro Tyr Lys Gly
           435                 440                 445
   Lys Phe Lys Tyr Ile Lys Ala Phe Phe Lys Asn
       450                 455
<210> 10
   <211> 274
   <212> PRT
   <213> Staphylococcus aureus
<400> 10
   Met Tyr Pro Asn Trp Gly Gln Tyr Lys Arg Ala Asp Leu Ile Gly Gln
   1               5                   10                  15
   Ser Ser Tyr Ile Lys Asn Asn Asp Val Val Ile Phe Asn Glu Ala Phe
               20                  25                  30
   Asp Asn Gly Ala Ser Asp Lys Leu Leu Ser Asn Val Lys Lys Glu Tyr
           35                  40                  45
   Pro Tyr Gln Thr Pro Val Leu Gly Arg Ser Gln Ser Gly Trp Asp Lys
       50                  55                  60
   Thr Glu Gly Ser Tyr Ser Ser Thr Val Ala Glu Asp Gly Gly Val Ala
   65                  70                  75                  80
   Ile Val Ser Lys Tyr Pro Ile Lys Glu Lys Ile Gln His Val Phe Lys
                   85                  90                  95
   Ser Gly Cys Gly Phe Asp Asn Asp Ser Asn Lys Gly Phe Val Tyr Thr
               100                 105                 110
   Lys Ile Glu Lys Asn Gly Lys Asn Val His Val Ile Gly Thr His Thr
           115                 120                 125
   Gln Ser Glu Asp Ser Arg Cys Gly Ala Gly His Asp Arg Lys Ile Arg
       130                 135                 140
   Ala Glu Gln Met Lys Glu Ile Ser Asp Phe Val Lys Lys Lys Asn Ile
   145                 150                 155                 160
   Pro Lys Asp Glu Thr Val Tyr Ile Gly Gly Asp Leu Asn Val Asn Lys
                   165                 170                 175
   Gly Thr Pro Glu Phe Lys Asp Met Leu Lys Asn Leu Asn Val Asn Asp
               180                 185                 190
   Val Leu Tyr Ala Gly His Asn Ser Thr Trp Asp Pro Gln Ser Asn Ser
           195                 200                 205
   Ile Ala Lys Tyr Asn Tyr Pro Asn Gly Lys Pro Glu His Leu Asp Tyr
       210                 215                 220
   Ile Phe Thr Asp Lys Asp His Lys Gln Pro Lys Gln Leu Val Asn Glu
   225                 230                 235                 240
   Val Val Thr Glu Lys Pro Lys Pro Trp Asp Val Tyr Ala Phe Pro Tyr
                   245                 250                 255
   Tyr Tyr Val Tyr Asn Asp Phe Ser Asp His Tyr Pro Ile Lys Ala Tyr
   260 265 270
   Ser Lys
<210> 11
   <211> 267
   <212> PRT
   <213> Staphylococcus aureus
<400> 11
   Met Lys Lys Ile Ala Thr Ala Thr Ile Ala Thr Ala Gly Phe Ala Thr
   1 5 10 15
   Ile Ala Ile Ala Ser Gly Asn Gln Ala His Ala Ser Glu Gln Asp Asn
   20 25 30
   Tyr Gly Tyr Asn Pro Asn Asp Pro Thr Ser Tyr Ser Tyr Thr Tyr Thr
   35 40 45
   Ile Asp Ala Gln Gly Asn Tyr His Tyr Thr Trp Lys Gly Asn Trp His
   50 55 60
   Pro Ser Gln Leu Asn Gln Asp Asn Gly Tyr Tyr Ser Tyr Tyr Tyr Tyr
   65 70 75 80
   Asn Gly Tyr Asn Asn Tyr Asn Asn Tyr Asn Asn Gly Tyr Ser Tyr Asn
   85 90 95
   Asn Tyr Ser Arg Tyr Asn Asn Tyr Ser Asn Asn Asn Gln Ser Tyr Asn
   100 105 110
   Tyr Asn Asn Tyr Asn Ser Tyr Asn Thr Asn Ser Tyr Arg Thr Gly Gly
   115 120 125
   Leu Gly Ala Ser Tyr Ser Thr Ser Ser Asn Asn Val Gln Val Thr Thr
   130 135 140
   Thr Met Ala Pro Ser Ser Asn Gly Arg Ser Ile Ser Ser Gly Tyr Thr
   145 150 155 160
   Ser Gly Arg Asn Leu Tyr Thr Ser Gly Gln Cys Thr Tyr Tyr Val Phe
   165 170 175
   Asp Arg Val Gly Gly Lys Ile Gly Ser Thr Trp Gly Asn Ala Ser Asn
   180 185 190
   Trp Ala Asn Ala Ala Ala Arg Ala Gly Tyr Thr Val Asn Asn Thr Pro
           195                 200                 205
   Lys Ala Gly Ala Ile Met Gln Thr Thr Gln Gly Ala Tyr Gly His Val
       210                 215                 220
   Ala Tyr Val Glu Ser Val Asn Ser Asn Gly Ser Val Arg Val Ser Glu
   225                 230                 235                 240
   Met Asn Tyr Gly Tyr Gly Pro Gly Val Val Thr Ser Arg Thr Ile Ser
                   245                 250                 255
   Ala Ser Gln Ala Ala Gly Tyr Asn Phe Ile His
               260                 265
<210> 12.
   <211> 145
   <212> PRT
   <213> Staphylococcus aureus
<400> 12
   Met Arg Gln Thr Phe Met Ala Asn Glu Ser Asn Ile Glu Arg Lys Trp
   1               5                   10                  15
   Tyr Val Ile Asp Ala Glu Gly Gln Thr Leu Gly Arg Leu Ser Ser Glu
               20                  25                  30
   Val Ala Ser Ile Leu Arg Gly Lys Asn Lys Val Thr Tyr Thr Pro His
           35                  40                  45
   Val Asp Thr Gly Asp Tyr Val Ile Val Ile Asn Ala Ser Lys Ile Glu
       50                  55                  60
   Phe Thr Gly Asn Lys Glu Thr Asp Lys Val Tyr Tyr Arg His Ser Asn
   65                  70                  75                  80
   His Pro Gly Gly Ile Lys Ser Ile Thr Ala Gly Glu Leu Arg Arg Thr
                   85                  90                  95
   Asn Pro Glu Arg Leu Ile Glu Asn Ser Ile Lys Gly Met Leu Pro Ser
               100                 105                 110
   Thr Arg Leu Gly Glu Lys Gln Gly Lys Lys Leu Phe Val Tyr Gly Gly
           115                 120                 125
   Ala Glu His Pro His Ala Ala Gln Gln Pro Glu Asn Tyr Glu Leu Arg
       130                 135                 140
   Gly
    145
<210> 13
   <211> 305
   <212> PRT
   <213> Staphylococcus aureus
<400> 13
   Met Glu Thr Tyr Glu Phe Asn Ile Thr Asp Lys Glu Gln Thr Gly Met
   1               5                   10                  15
   Arg Val Asp Lys Leu Leu Pro Glu Leu Asn Asn Asp Trp Ser Arg Asn
               20                  25                  30
   Gln Ile Gln Asp Trp Ile Lys Ala Gly Leu Val Val Ala Asn Asp Lys
           35                  40                  45
   Val Val Lys Ser Asn Tyr Lys Val Lys Leu Asn Asp His Ile Val Val
       50                  55                  60
   Thr Glu Lys Glu Val Val Glu Ala Asp Ile Leu Pro Glu Asn Leu Asn
   65                  70                  75                  80
   Leu Asp Ile Tyr Tyr Glu Asp Asp Asp Val Ala Val Val Tyr Lys Pro
                   85                  90                  95
   Lys Gly Met Val Val His Pro Ser Pro Gly His Tyr Thr Asn Thr Leu
               100                 105                 110
   Val Asn Gly Leu Met Tyr Gln Ile Lys Asn Leu Ser Gly Ile Asn Gly
           115                 120                 125
   Glu Ile Arg Pro Gly Ile Val His Arg Ile Asp Met Asp Thr Ser Gly
       130                 135                 140
   Leu Leu Met Val Ala Lys Asn Asp Ile Ala His Arg Gly Leu Val Glu
   145                 150                 155                 160
   Gln Leu Met Asp Lys Ser Val Lys Arg Lys Tyr Ile Ala Leu Val His
                   165                 170                 175
   Gly Asn Ile Pro His Asp Tyr Gly Thr Ile Asp Ala Pro Ile Gly Arg
               180                 185                 190
   Asn Lys Asn Asp Arg Gln Ser Met Ala Val Val Asp Asp Gly Lys Glu
           195                 200                 205
   Ala Val Thr His Phe Asn Val Leu Glu His Phe Lys Asp Tyr Thr Leu
   210 215 220
   Val Glu Cys Gln Leu Glu Thr Gly Arg Thr His Gln Ile Arg Val His
   225 230 235 240
   Met Lys Tyr Ile Gly Phe Pro Leu Val Gly Asp Pro Lys Tyr Gly Pro
   245 250 255
   Lys Lys Thr Leu Asp Ile Gly Gly Gln Ala Leu His Ala Gly Leu Ile
   260 265 270
   Gly Phe Glu His Pro Val Thr Gly Glu Tyr Ile Glu Arg His Ala Glu
   275 280 285
   Leu Pro Gln Asp Phe Glu Asp Leu Leu Asp Thr Ile Arg Lys Arg Asp
   290 295 300
   Ala
   305
<210> 14
   <211> 394
   <212> PRT
   <213> Staphylococcus aureus
<400> 14
   Met Ala Lys Glu Lys Phe Asp Arg Ser Lys Glu His Ala Asn Ile Gly
   1               5                   10                  15
   Thr Ile Gly His Val Asp His Gly Lys Thr Thr Leu Thr Ala Ala Ile
               20                  25                  30
   Ala Thr Val Leu Ala Lys Asn Gly Asp Ser Val Ala Gln Ser Tyr Asp
           35                  40                  45
   Met Ile Asp Asn Ala Pro Glu Glu Lys Glu Arg Gly Ile Thr Ile Asn
       50                  55                  60
   Thr Ser His Ile Glu Tyr Gln Thr Asp Lys Arg His Tyr Ala His Val
   65                  70                  75                  80
   Asp Cys Pro Gly His Ala Asp Tyr Val Lys Asn Met Ile Thr Gly Ala
                   85                  90                  95
   Ala Gln Met Asp Gly Gly Ile Leu Val Val Ser Ala Ala Asp Gly Pro
               100                 105                 110
   Met Pro Gln Thr Arg Glu His Ile Leu Leu Ser Arg Asn Val Gly Val
   115 120 125
   Pro Ala Leu Val Val Phe Leu Asn Lys Val Asp Met Val Asp Asp Glu
   130 135 140
   Glu Leu Leu Glu Leu Val Glu Met Glu Val Arg Asp Leu Leu Ser Glu
   145 150 155 160
   Tyr Asp Phe Pro Gly Asp Asp Val Pro Val Ile Ala Gly Ser Ala Leu
   165 170 175
   Lys Ala Leu Glu Gly Asp Ala Gln Tyr Glu Glu Lys Ile Leu Glu Leu
   180 185 190
   Met Glu Ala Val Asp Thr Tyr Ile Pro Thr Pro Glu Arg Asp Ser Asp
   195 200 205
   Lys Pro Phe Met Met Pro Val Glu Asp Val Phe Ser Ile Thr Gly Arg
   210 215 220
   Gly Thr Val Ala Thr Gly Arg Val Glu Arg Gly Gln Ile Lys Val Gly
   225 230 235 240
   Glu Glu Val Glu Ile Ile Gly Leu His Asp Thr Ser Lys Thr Thr Val
   245 250 255
   Thr Gly Val Glu Met Phe Arg Lys Leu Leu Asp Tyr Ala Glu Ala Gly
   260 265 270
   Asp Asn Ile Gly Ala Leu Leu Arg Gly Val Ala Arg Glu Asp Val Gln
   275 280 285
   Arg Gly Gln Val Leu Ala Ala Pro Gly Ser Ile Thr Pro His Thr Glu
   290 295 300
   Phe Lys Ala Glu Val Tyr Val Leu Ser Lys Asp Glu Gly Gly Arg His
   305 310 315 320
   Thr Pro Phe Phe Ser Asn Tyr Arg Pro Gln Phe Tyr Phe Arg Thr Thr
   325 330 335
   Asp Val Thr Gly Val Val His Leu Pro Glu Gly Thr Glu Met Val Met
   340 345 350
   Pro Gly Asp Asn Val Glu Met Thr Val Glu Leu Ile Ala Pro Ile Ala
   355 360 365
   Ile Glu Asp Gly Thr Arg Phe Ser Ile Arg Glu Gly Gly Arg Thr Val
       370                 375                     380
   Gly Ser Gly Val Val Thr Glu Ile Ile Lys
   385                 390
<210> 15
   <211> 943
   <212> PRT
   <213> Staphylococcus aureus
<400> 15
   Met Leu Gly Val Ile Asn Arg Met Ala Lys Lys Phe Asn Tyr Lys Leu
   1               5                   10                  15
   Pro Ser Met Val Ala Leu Thr Leu Val Gly Ser Ala Val Thr Ala His
               20                  25                  30
   Gln Val Gln Ala Ala Glu Thr Thr Gln Asp Gln Thr Thr Asn Lys Asn
           35                  40                  45
   Val Leu Asp Ser Asn Lys Val Lys Ala Thr Thr Glu Gln Ala Lys Ala
       50                  55                  60
   Glu Val Lys Asn Pro Thr Gln Asn Ile Ser Gly Thr Gln Val Tyr Gln
   65                  70                  75                  80
   Asp Pro Ala Ile Val Gln Pro Lys Thr Ala Asn Asn Lys Thr Gly Asn
                   85                  90                  95
   Ala Gln Val Ser Gln Lys Val Asp Thr Ala Gln Val Asn Gly Asp Thr
               100                 105                 110
   Arg Ala Asn Gln Ser Ala Thr Thr Asn Asn Thr Gln Pro Val Ala Lys
           115                 120                 125
   Ser Thr Ser Thr Thr Ala Pro Lys Thr Asn Thr Asn Val Thr Asn Ala
       130                 135                 140
   Gly Tyr Ser Leu Val Asp Asp Glu Asp Asp Asn Ser Glu His Gln Ile
   145                 150                 155                 160
   Asn Pro Glu Leu Ile Lys Ser Ala Ala Lys Pro Ala Ala Leu Glu Thr
                   165                 170                 175
   Gln Tyr Lys Ala Ala Ala Pro Lys Ala Lys Thr Glu Ala Thr Pro Lys
               180                 185                 190
   Val Thr Thr Phe Ser Ala Ser Ala Gln Pro Arg Ser Val Ala Ala Thr
           195                 200                 205
   Pro Lys Thr Ser Leu Pro Lys Tyr Lys Pro Gln Val Asn Ser Ser Ile
       210                 215                 220
   Asn Asp Tyr Ile Arg Lys Asn Asn Leu Lys Ala Pro Lys Ile Glu Glu
   225                 230                 235                 240
   Asp Tyr Thr Ser Tyr Phe Pro Lys Tyr Ala Tyr Arg Asn Gly Val Gly
                   245                 250                 255
   Arg Pro Glu Gly Ile Val Val His Asp Thr Ala Asn Asp Arg Ser Thr
               260                 265                 270
   Ile Asn Gly Glu Ile Ser Tyr Met Lys Asn Asn Tyr Gln Asn Ala Phe
           275                 280                 285
   Val His Ala Phe Val Asp Gly Asp Arg Ile Ile Glu Thr Ala Pro Thr
       290                 295                 300
   Asp Tyr Leu Ser Trp Gly Val Gly Ala Val Gly Asn Pro Arg Phe Ile
   305                 310                 315                 320
   Asn Val Glu Ile Val His Thr His Asp Tyr Ala Ser Phe Ala Arg Ser
                   325                 330                 335
   Met Asn Asn Tyr Ala Asp Tyr Ala Ala Thr Gln Leu Gln Tyr Tyr Gly
               340                 345                 350
   Leu Lys Pro Asp Ser Ala Glu Tyr Asp Gly Asn Gly Thr Val Trp Thr
           355                 360                 365
   His Tyr Ala Val Ser Lys Tyr Leu Gly Gly Thr Asp His Ala Asp Pro
       370                 375                 380
   His Gly Tyr Leu Arg Ser His Asn Tyr Ser Tyr Asp Gln Leu Tyr Asp
   385                 390                 395                 400
   Leu Ile Asn Glu Lys Tyr Leu Ile Lys Met Gly Lys Val Ala Pro Trp
                   405                 410                 415
   Gly Thr Gln Phe Thr Thr Thr Pro Thr Thr Pro Ser Lys Pro Thr Thr
               420                 425                 430
   Pro Ser Lys Pro Ser Thr Gly Lys Leu Thr Val Ala Ala Asn Asn Gly
           435                 440                 445
   Val Ala Gln Ile Lys Pro Thr Asn Ser Gly Leu Tyr Thr Thr Val Tyr
       450                 455                 460
   Asp Lys Thr Gly Lys Ala Thr Asn Glu Val Gln Lys Thr Phe Ala Val
   465                 470                 475                 480
   Ser Lys Thr Ala Thr Leu Gly Asn Gln Lys Phe Tyr Leu Val Gln Asp
                   485                 490                 495
   Tyr Asn Ser Gly Asn Lys Phe Gly Trp Val Lys Glu Gly Asp Val Val
               500                 505                 510
   Tyr Asn Thr Ala Lys Ser Pro Val Asn Val Asn Gln Ser Tyr Ser Ile
           515                 520                 525
   Lys Ser Gly Thr Lys Leu Tyr Thr Val Pro Trp Gly Thr Ser Lys Gln
       530                 535                 540
   Val Ala Gly Ser Val Ser Gly Ser Gly Asn Gln Thr Phe Lys Ala Ser
   545                 550                 555                 560
   Lys Gln Gln Gln Ile Asp Lys Ser Ile Tyr Leu Tyr Gly Ser Val Asn
                   565                 570                 575
   Gly Lys Ser Gly Trp Val Ser Lys Ala Tyr Leu Val Asp Thr Ala Lys
               580                 585                 590
   Pro Thr Pro Thr Pro Ile Pro Lys Pro Ser Thr Pro Thr Thr Asn Asn
           595                 600                 605
   Lys Leu Thr Val Ser Ser Leu Asn Gly Val Ala Gln Ile Asn Ala Lys
       610                 615                 620
   Asn Asn Gly Leu Phe Thr Thr Val Tyr Asp Lys Thr Gly Lys Pro Thr
   625                 630                 635                 640
   Lys Glu Val Gln Lys Thr Phe Ala Val Thr Lys Glu Ala Ser Leu Gly
                   645                 650                 655
   Gly Asn Lys Phe Tyr Leu Val Lys Asp Tyr Asn Ser Pro Thr Leu Ile
               660                 665                 670
   Gly Trp Val Lys Gln Gly Asp Val Ile Tyr Asn Asn Ala Lys Ser Pro
           675                 680                 685
   Val Asn Val Met Gln Thr Tyr Thr Val Lys Pro Gly Thr Lys Leu Tyr
       690                 695                 700
   Ser Val Pro Trp Gly Thr Tyr Lys Gln Glu Ala Gly Ala Val Ser Gly
   705                 710                 715                 720
   Thr Gly Asn Gln Thr Phe Lys Ala Thr Lys Gln Gln Gln Ile Asp Lys
                   725                 730                 735
   Ser Ile Tyr Leu Phe Gly Thr Val Asn Gly Lys Ser Gly Trp Val Ser
               740                 745                 750
   Lys Ala Tyr Leu Ala Val Pro Ala Ala Pro Lys Lys Ala Val Ala Gln
           755                 760                 765
   Pro Lys Thr Ala Val Lys Ala Tyr Thr Val Thr Lys Pro Gln Thr Thr
       770                 775                 780
   Gln Thr Val Ser Lys Ile Ala Gln Val Lys Pro Asn Asn Thr Gly Ile
   785                 790                 795                 800
   Arg Ala Ser Val Tyr Glu Lys Thr Ala Lys Asn Gly Ala Lys Tyr Ala
                   805                 810                 815
   Asp Arg Thr Phe Tyr Val Thr Lys Glu Arg Ala His Gly Asn Glu Thr
               820                 825                 830
   Tyr Val Leu Leu Asn Asn Thr Ser His Asn Ile Pro Leu Gly Trp Phe
           835                 840                 845
   Asn Val Lys Asp Leu Asn Val Gln Asn Leu Gly Lys Glu Val Lys Thr
       850                 855                 860
   Thr Gln Lys Tyr Thr Val Asn Lys Ser Asn Asn Gly Leu Ser Met Val
   865                 870                 875                 880
   Pro Trp Gly Thr Lys Asn Gln Val Ile Leu Thr Gly Asn Asn Ile Ala
                   885                 890                 895
   Gln Gly Thr Phe Asn Ala Thr Lys Gln Val Ser Val Gly Lys Asp Val
               900                 905                 910
   Tyr Tyr Thr Val Leu Leu Ile Thr Ala Leu Val Gly Lys Ala Lys Asp
           915                 920                 925
   Leu Pro His Gln Leu Gly Asn Gln Leu His Gln Leu Pro Lys Ile
       930                 935                 940
<210> 16
   <211> 564
   <212> PRT
   <213> Staphylococcus aureus
<400> 16
   Met Thr Asp Tyr Val Thr Lys Tyr Ala Lys Lys Val Val Ser Gly Glu
   1               5                   10                  15
   Ile Leu Ala Ser Leu Lys Asn Ile Gln Val Cys Lys Arg His Leu Ser
               20                  25                  30
   Phe Met Glu Asn Pro Pro Asn Gly Cys His Trp Asp Asn His Leu Ser
           35                  40                  45
   Asn Lys Ala Ile Lys Phe Val Glu Met Leu Pro Asp Pro Lys Thr Asn
       50                  55                  60
   Gln Pro Met Pro Leu Met Glu Phe Gln Lys Phe Ile Val Gly Ser Leu
   65                  70                  75                  80
   Tyr Gly Trp Arg Arg Gly Gln Tyr Arg Met Phe Thr Lys Ala Tyr Ile
                   85                  90                  95
   Ser Met Ala Arg Lys Gln Gly Lys Ser Leu Ile Val Ser Gly Met Ser
               100                 105                 110
   Val Asn Glu Leu Leu Phe Gly Gln Tyr Pro Lys Phe Asn Arg Gln Ile
           115                 120                 125
   Tyr Val Ala Ser Ser Thr Tyr Lys Gln Ala Gln Thr Ile Phe Lys Met
       130                 135                 140
   Ala Ser Gln Gln Val Asn Leu Met Arg Ser Lys Ser Lys Phe Ile Arg
   145                 150                 155                 160
   Glu Lys Thr Asp Val Arg Lys Thr Asp Ile Glu Asp Val Leu Ser Ser
                   165                 170                 175
   Ser Val Phe Ala Pro Leu Ser Asn Asn Pro Asp Ala Val Asp Gly Lys
               180                 185                 190
   Asp Pro Thr Val Ala Ile Leu Asp Glu Leu Ala Ser Met Pro Asp Asp
           195                 200                 205
   Glu Met Tyr Ser Arg Phe Lys Thr Gly Met Thr Leu Gln Lys Asn Pro
       210                 215                 220
   Leu Thr Leu Leu Val Ser Thr Ala Gly Asp Asn Leu Asn Ser Gln Met
   225                 230                 235                 240
   Tyr Gln Glu Tyr Lys Tyr Ile Lys Arg Ile Leu Asn Glu Glu Val Arg
                   245                 250                 255
   Ala Asp Asn Tyr Phe Val Tyr Cys Ala Glu Met Asp Ser Gln Glu Glu
               260                 265                 270
   Val Gln Asp Glu Thr Lys Trp Ile Lys Ala Met Pro Leu Leu Glu Ser
           275                 280                 285
   Lys Glu His Arg Lys Thr Ile Leu Gln Asn Val Lys Ala Asp Ile Gln
       290                 295                 300
   Asp Glu Leu Glu Lys Gly Thr Ser Tyr His Lys Ile Leu Ile Lys Asn
   305                 310                 315                 320
   Phe Asn Leu Trp Gln Ala Gln Arg Glu Asp Ser Leu Leu Asp Ile Ser
                   325                 330                 335
   Asp Trp Glu Gln Val Ile Thr Pro Met Pro Asn Ile Asn Gly Lys Asp
               340                 345                 350
   Val Tyr Ile Gly Val Asp Leu Ser Arg Leu Asp Asp Leu Thr Ser Val
           355                 360                 365
   Gly Phe Ile Phe Pro Asn Asp Asp Lys Lys Val Phe Leu His Ser His
       370                 375                 380
   Ser Phe Ile Gly Leu Arg Thr Asn Leu Glu Gln Lys Ser Lys Arg Asp
   385                 390                 395                 400
   Lys Ile Asn Tyr Glu Leu Ala Ile Glu Arg Gly Glu Ala Glu Thr Thr
                   405                 410                 415
   Gln Ser Asp Ser Gly Met Ile Asp Tyr Lys Gln Val Ile Asp Phe Ile
               420                 425                 430
   Val Lys Phe Ile Thr Thr His Asp Leu Asn Val Gln Ala Val Cys Tyr
           435                 440                 445
   Asp Pro Trp Asn Ala Gln Ser Phe Ile Thr Thr Ile Glu Ser Met Ala
       450                 455                 460
   Leu Asp Trp Pro Leu Ile Glu Val Gly Gln Ser Phe Lys Ala Leu Ser
   465                 470                 475                 480
   Gln Ser Ile Lys Glu Phe Arg Met Trp Val Ala Asp Glu Arg Ile Gln
   485 490 495
   His Asn Asp Asn Met Leu Leu Thr Thr Ser Val Asn Asn Ala Val Leu
   500 505 510
   Ile Arg Asp Gly Glu Asp Asn Val Lys Ile Asn Lys Lys Met Asn Arg
   515 520 525
   Gln Lys Ile Asp Pro Ile Ile Ser Ile Ile Thr Ala Phe Thr Glu Ala
   530 535 540
   Arg Met His Glu Phe Gln Glu Asn Trp Thr Glu Lys Tyr Glu Ser Glu
   545 550 555 560
   Glu Phe Gly Phe
<210> 17
   <211> 466
   <212> PRT
   <213> Staphylococcus aureus
<400> 17
   Met Thr Lys Ser Val Ala Ile Ile Gly Ala Gly Ile Thr Gly Leu Ser
   1               5                   10                  15
   Ser Ala Tyr Phe Leu Lys Gln Gln Asp Pro Asn Ile Asp Val Thr Ile
               20                  25                  30
   Phe Glu Ala Ser Asn Arg Pro Gly Gly Lys Ile Gln Ser Tyr Arg Lys
           35                  40                  45
   Asp Gly Tyr Met Ile Glu Leu Gly Pro Glu Ser Tyr Leu Gly Arg Lys
       50                  55                  60
   Thr Ile Met Thr Glu Leu Ala Lys Asp Ile Gly Leu Glu Gln Asp Ile
   65                  70                  75                  80
   Val Thr Asn Thr Thr Gly Gln Ser Tyr Ile Phe Ala Lys Asn Lys Leu
                   85                  90                  95
   Tyr Pro Ile Pro Gly Gly Ser Ile Met Gly Ile Pro Thr Asp Ile Lys
               100                 105                 110
   Pro Phe Val Thr Thr Lys Leu Ile Ser Pro Leu Gly Lys Leu Arg Ala
           115                 120                 125
   Gly Leu Asp Leu Ile Lys Lys Pro Ile Gln Met Gln Asp Gly Asp Ile
       130                 135                 140
   Ser Val Gly Ala Phe Phe Arg Ala Arg Leu Gly Asn Glu Val Leu Glu
   145                 150                 155                 160
   Asn Leu Ile Glu Pro Leu Met Gly Gly Ile Tyr Gly Thr Asp Ile Asp
                   165                 170                 175
   Lys Leu Ser Leu Met Ser Thr Phe Pro Asn Phe Lys Glu Lys Glu Glu
               180                 185                 190
   Ala Phe Gly Ser Leu Ile Lys Gly Met Lys Asp Glu Lys Asn Lys Arg
           195                 200                 205
   Leu Lys Gln Arg Gln Leu Tyr Pro Gly Ala Pro Lys Gly Gln Phe Lys
       210                 215                 220
   Gln Phe Lys His Gly Leu Ser Ser Phe Ile Glu Ala Leu Glu Gln Asp
   225                 230                 235                 240
   Val Lys Asn Lys Gly Val Thr Ile Arg Tyr Asn Thr Ser Val Asp Asp
                   245                 250                 255
   Ile Ile Thr Ser Gln Lys Gln Tyr Lys Ile Val Tyr Ser Asn Gln Gln
               260                 265                 270
   Glu Asp Val Phe Asp Gly Val Leu Val Thr Thr Pro His Gln Val Phe
           275                 280                 285
   Leu Asn Trp Phe Gly Gln Asp Pro Ala Phe Asp Tyr Phe Lys Thr Met
       290                 295                 300
   Asp Ser Thr Thr Val Ala Thr Val Val Leu Ala Phe Asp Glu Lys Asp
   305                 310                 315                 320
   Ile Glu Asn Thr Tyr Asp Gly Thr Gly Phe Val Ile Ala Arg Thr Ser
                   325                 330                 335
   Asp Thr Asp Ile Thr Ala Cys Thr Trp Thr Ser Lys Lys Trp Pro Phe
               340                 345                 350
   Thr Thr Pro Glu Gly Lys Val Leu Ile Arg Ala Tyr Val Gly Lys Pro
           355                 360                 365
   Gly Asp Thr Val Val Asp Asp His Thr Asp Asn Glu Leu Val Ser Ile
       370                 375                 380
   Val Arg Arg Asp Leu Ser Gln Met Met Thr Phe Lys Gly Asp Pro Glu
   385 390 395 400
   Phe Thr Ile Val Asn Arg Leu Pro Lys Ser Met Pro Gln Tyr His Val
   405 410 415
   Gly His Ile Gln Gln Ile Arg Gln Ile Gln Ala His Ile Lys Gln Thr
   420 425 430
   Tyr Pro Arg Leu Arg Val Thr Gly Ala Ser Phe Glu Ala Val Gly Leu
   435 440 445
   Pro Asp Cys Ile Thr Gln Gly Lys Val Ala Ala Glu Glu Val Ile Ala
   450 455 460
   Glu Leu
   465
<210> 18
   <211> 338
   <212> PRT
   <213> Staphylococcus aureus
<400> 18
   Met Ile Lys Gln Leu Tyr Lys Asn Ile Thr Ile Cys Ser Leu Ala Ile
   1               5                   10                  15
   Ser Thr Ala Leu Thr Val Phe Pro Ala Thr Ser Tyr Ala Lys Ile Asn
               20                  25                  30
   Ser Glu Ile Lys Ala Val Ser Glu Lys Asn Leu Asp Gly Asp Thr Lys
           35                  40                  45
   Met Tyr Thr Arg Thr Ala Thr Thr Ser Asp Ser Gln Lys Asn Ile Thr
       50                  55                  60
   Gln Ser Leu Gln Phe Asn Phe Leu Thr Glu Pro Asn Tyr Asp Lys Glu
   65                  70                  75                  80
   Thr Val Phe Ile Lys Ala Lys Gly Thr Ile Gly Ser Gly Leu Arg Ile
                   85                  90                  95
   Leu Asp Pro Asn Gly Tyr Trp Asn Ser Thr Leu Arg Trp Pro Gly Ser
               100                 105                 110
   Tyr Ser Val Ser Ile Gln Asn Val Asp Asp Asn Asn Asn Thr Asn Val
           115                 120                 125
   Thr Asp Phe Ala Pro Lys Asn Gln Asp Glu Ser Arg Glu Val Lys Tyr
       130                 135                 140
   Thr Tyr Gly Tyr Lys Thr Gly Gly Asp Phe Ser Ile Asn Arg Gly Gly
   145                 150                 155                 160
   Leu Thr Gly Asn Ile Thr Lys Glu Ser Asn Tyr Ser Glu Thr Ile Ser
                   165                 170                 175
   Tyr Gln Gln Pro Ser Tyr Arg Thr Leu Leu Asp Gln Ser Thr Ser His
               180                 185                 190
   Lys Gly Val Gly Trp Lys Val Glu Ala His Leu Ile Asn Asn Met Gly
           195                 200                 205
   His Asp His Thr Arg Gln Leu Thr Asn Asp Ser Asp Asn Arg Thr Lys
       210                 215                 220
   Ser Glu Ile Phe Ser Leu Thr Arg Asn Gly Asn Leu Trp Ala Lys Asp
   225                 230                 235                 240
   Asn Phe Thr Pro Lys Asp Lys Met Pro Val Thr Val Ser Glu Gly Phe
                   245                 250                 255
   Asn Pro Glu Phe Leu Ala Val Met Ser His Asp Lys Lys Asp Lys Gly
               260                 265                 270
   Lys Ser Gln Phe Val Val His Tyr Lys Arg Ser Met Asp Glu Phe Lys
           275                 280                 285
   Ile Asp Trp Asn Arg His Gly Phe Trp Gly Tyr Trp Ser Gly Glu Asn
       290                 295                 300
   His Val Asp Lys Lys Glu Glu Lys Leu Ser Ala Leu Tyr Glu Val Asp
   305                 310                 315                 320
   Trp Lys Thr His Asp Val Lys Phe Val Lys Val Leu Asn Asp Asn Glu
                   325                 330                 335
   Lys Lys
<210> 19
   <211> 485
   <212> PRT
   <213> Staphylococcus aureus
<400> 19
   Met Ser Ile Arg Tyr Glu Ser Val Glu Asn Leu Leu Thr Leu Ile Lys
   1               5                   10                  15
   Asp Lys Lys Ile Lys Pro Ser Asp Val Val Lys Asp Ile Tyr Asp Ala
               20                  25                  30
   Ile Glu Glu Thr Asp Pro Thr Ile Lys Ser Phe Leu Ala Leu Asp Lys
           35                  40                  45
   Glu Asn Ala Ile Lys Lys Ala Gln Glu Leu Asp Glu Leu Gln Ala Lys
       50                  55                  60
   Asp Gln Met Asp Gly Lys Leu Phe Gly Ile Pro Met Gly Ile Lys Asp
   65                  70                  75                  80
   Asn Ile Ile Thr Asn Gly Leu Glu Thr Thr Cys Ala Ser Lys Met Leu
                   85                  90                  95
   Glu Gly Phe Val Pro Ile Tyr Glu Ser Thr Val Met Glu Lys Leu His
               100                 105                 110
   Asn Glu Asn Ala Val Leu Ile Gly Lys Leu Asn Met Asp Glu Phe Ala
           115                 120                 125
   Met Gly Gly Ser Thr Glu Thr Ser Ile Ser Lys Lys Thr Val Asn Pro
       130                 135                 140
   Phe Asp His Lys Ala Val Pro Gly Gly Ser Ser Gly Gly Ser Ala Ala
   145                 150                 155                 160
   Ala Val Ala Ala Gly Leu Val Pro Phe Ser Leu Gly Ser Asp Thr Gly
                   165                 170                 175
   Gly Ser Ile Arg Gln Pro Ala Ala Tyr Cys Gly Val Val Gly Met Lys
               180                 185                 190
   Pro Thr Tyr Gly Arg Val Ser Arg Phe Gly Leu Val Ala Phe Ala Ser
           195                 200                 205
   Ser Leu Asp Gln Ile Gly Pro Leu Thr Arg Asn Val Lys Asp Asn Ala
       210                 215                 220
   Ile Val Leu Glu Ala Ile Ser Gly Ala Asp Ala Asn Asp Ser Thr Ser
   225                 230                 235                 240
   Ala Pro Val Asp Asp Val Asp Phe Thr Ser Glu Ile Gly Lys Asp Ile
                   245                 250                 255
   Lys Gly Leu Lys Val Ala Leu Pro Lys Glu Tyr Leu Gly Glu Gly Val
               260                 265                 270
   Ala Asp Asp Val Lys Glu Ala Val Gln Asn Ala Val Glu Thr Leu Lys
           275                 280                 285
   Ser Leu Gly Ala Val Val Glu Glu Val Ser Leu Pro Asn Thr Lys Phe
       290                 295                 300
   Gly Ile Pro Ser Tyr Tyr Val Ile Ala Ser Ser Glu Ala Ser Ser Asn
   305                 310                 315                 320
   Leu Ser Arg Phe Asp Gly Ile Arg Tyr Gly Tyr His Ser Lys Glu Ala
                   325                 330                 335
   His Ser Leu Glu Glu Leu Tyr Lys Met Ser Arg Ser Glu Gly Phe Gly
               340                 345                 350
   Lys Glu Val Lys Arg Arg Ile Phe Leu Gly Thr Phe Ala Leu Ser Ser
           355                 360                 365
   Gly Tyr Tyr Asp Ala Tyr Tyr Lys Lys Ser Gln Lys Val Arg Thr Leu
       370                 375                 380
   Ile Lys Asn Asp Phe Asp Lys Val Phe Glu Asn Tyr Asp Val Val Val
   385                 390                 395                 400
   Gly Pro Thr Ala Pro Thr Thr Ala Phe Asn Leu Gly Glu Glu Ile Asp
                   405                 410                 415
   Asp Pro Leu Thr Met Tyr Ala Asn Asp Leu Leu Thr Thr Pro Val Asn
               420                 425                 430
   Leu Ala Gly Leu Pro Gly Ile Ser Val Pro Cys Gly Gln Ser Asn Gly
           435                 440                 445
   Arg Pro Ile Gly Leu Gln Phe Ile Gly Lys Pro Phe Asp Glu Lys Thr
       450                 455                 460
   Leu Tyr Arg Val Ala Tyr Gln Tyr Glu Thr Gln Tyr Asn Leu His Asp
   465                 470                 475                 480
   Val Tyr Glu Lys Leu
                   485
<210> 20.
   <211> 413
   <212> PRT
   <213> Staphylococcus aureus
<400> 20
   Met Ala Glu His Ser Phe Asp Val Asn Glu Val Ile Lys Asp Phe Pro
   1               5                   10                  15
   Ile Leu Asp Gln Lys Val Asn Gly Lys Arg Leu Ala Tyr Leu Asp Ser
               20                  25                  30
   Thr Ala Thr Ser Gln Thr Pro Met Gln Val Leu Asn Val Leu Glu Asp
           35                  40                  45
   Tyr Tyr Lys Arg Tyr Asn Ser Asn Val His Arg Gly Val His Thr Leu
       50                  55                  60
   Gly Ser Leu Ala Thr Asp Gly Tyr Glu Asn Ala Arg Glu Thr Val Arg
   65                  70                  75                  80
   Arg Phe Ile Asn Ala Lys Tyr Phe Glu Glu Ile Ile Phe Thr Arg Gly
                   85                  90                  95
   Thr Thr Ala Ser Ile Asn Leu Val Ala His Ser Tyr Gly Asp Ala Asn
               100                 105                 110
   Val Glu Glu Gly Asp Glu Ile Val Val Thr Glu Met Glu His His Ala
           115                 120                 125
   Asn Ile Val Pro Trp Gln Gln Leu Ala Lys Arg Lys Asn Ala Thr Leu
       130                 135                 140
   Lys Phe Ile Pro Met Thr Ala Asp Gly Glu Leu Asn Ile Glu Asp Ile
   145                 150                 155                 160
   Lys Gln Thr Ile Asn Asp Lys Thr Lys Ile Val Ala Ile Ala His Ile
                   165                 170                 175
   Ser Asn Val Leu Gly Thr Ile Asn Asp Val Lys Thr Ile Ala Glu Ile
               180                 185                 190
   Ala His Gln His Gly Ala Ile Ile Ser Val Asp Gly Ala Gln Ala Ala
           195                 200                 205
   Pro His Met Lys Leu Asp Met Gln Glu Met Asn Ala Asp Phe Tyr Ser
       210                 215                 220
   Phe Ser Gly His Lys Met Leu Gly Pro Thr Gly Ile Gly Val Leu Phe
   225                 230                 235                 240
   Gly Lys Arg Glu Leu Leu Gln Lys Met Glu Pro Ile Glu Phe Gly Gly
                   245                 250                 255
   Asp Met Ile Asp Phe Val Ser Lys Tyr Asp Ala Thr Trp Ala Asp Leu
               260                 265                 270
   Pro Thr Lys Phe Glu Ala Gly Thr Pro Leu Ile Ala Gln Ala Ile Gly
           275                 280                 285
   Leu Ala Glu Ala Ile Arg Tyr Leu Glu Arg Ile Gly Phe Asp Ala Ile
       290                 295                 300
   His Lys Tyr Glu Gln Glu Leu Thr Ile Tyr Ala Tyr Glu Gln Met Ser
   305                 310                 315                 320
   Ala Ile Glu Gly Ile Glu Ile Tyr Gly Pro Pro Lys Asp Arg Arg Ala
                   325                 330                 335
   Gly Val Ile Thr Phe Asn Leu Gln Asp Val His Pro His Asp Val Ala
               340                 345                 350
   Thr Ala Val Asp Thr Glu Gly Val Ala Val Arg Ala Gly His His Cys
           355                 360                 365
   Ala Gln Pro Leu Met Lys Trp Leu Asn Val Ser Ser Thr Ala Arg Ala
       370                 375                 380
   Ser Phe Tyr Ile Tyr Asn Thr Lys Glu Asp Ile Asp Gln Leu Ile Asn
   385                 390                 395                 400
   Ala Leu Lys Gln Thr Lys Glu Phe Phe Ser Tyr Glu Phe
                   405                 410
<210> 21
   <211> 394
   <212> PRT
   <213> Staphylococcus aureus
<400> 21
   Met Ala Lys Glu Lys Phe Asp Arg Ser Lys Glu His Ala Asn Ile Gly
   1               5                   10                  15
   Thr Ile Gly His Val Asp His Gly Lys Thr Thr Leu Thr Ala Ala Ile
               20                  25                  30
   Ala Thr Val Leu Ala Lys Asn Gly Asp Ser Val Ala Gln Ser Tyr Asp
           35                  40                  45
   Met Ile Asp Asn Ala Pro Glu Glu Lys Glu Arg Gly Ile Thr Ile Asn
       50                  55                  60
   Thr Ser His Ile Glu Tyr Gln Thr Asp Lys Arg His Tyr Ala His Val
   65                  70                  75                  80
   Asp Cys Pro Gly His Ala Asp Tyr Val Lys Asn Met Ile Thr Gly Ala
                   85                  90                  95
   Ala Gln Met Asp Gly Gly Ile Leu Val Val Ser Ala Ala Asp Gly Pro
               100                 105                 110
   Met Pro Gln Thr Arg Glu His Ile Leu Leu Ser Arg Asn Val Gly Val
           115                 120                 125
   Pro Ala Leu Val Val Phe Leu Asn Lys Val Asp Met Val Asp Asp Glu
       130                 135                 140
   Glu Leu Leu Glu Leu Val Glu Met Glu Val Arg Asp Leu Leu Ser Glu
   145                 150                 155                 160
   Tyr Asp Phe Pro Gly Asp Asp Val Pro Val Ile Ala Gly Ser Ala Leu
                   165                 170                 175
   Lys Ala Leu Glu Gly Asp Ala Gln Tyr Glu Glu Lys Ile Leu Glu Leu
               180                 185                 190
   Met Glu Ala Val Asp Thr Tyr Ile Pro Thr Pro Glu Arg Asp Ser Asp
           195                 200                 205
   Lys Pro Phe Met Met Pro Val Glu Asp Val Phe Ser Ile Thr Gly Arg
       210                 215                 220
   Gly Thr Val Ala Thr Gly Arg Val Glu Arg Gly Gln Ile Lys Val Gly
   225                 230                 235                 240
   Glu Glu Val Glu Ile Ile Gly Leu His Asp Thr Ser Lys Thr Thr Val
                   245                 250                 255
   Thr Gly Val Glu Met Phe Arg Lys Leu Leu Asp Tyr Ala Glu Ala Gly
               260                 265                 270
   Asp Asn Ile Gly Ala Leu Leu Arg Gly Val Ala Arg Glu Asp Val Gln
           275                 280                 285
   Arg Gly Gln Val Leu Ala Ala Pro Gly Ser Ile Thr Pro His Thr Glu
       290                     295                     300
   Phe Lys Ala Glu Val Tyr Val Leu Ser Lys Asp Glu Gly Gly Arg His
   305                     310                     315                     320
   Thr Pro Phe Phe Ser Asn Tyr Arg Pro Gln Phe Tyr Phe Arg Thr Thr
                   325                     330                     335
   Asp Val Thr Gly Val Val His Leu Pro Glu Gly Thr Glu Met Val Met
               340                     345                     350
   Pro Gly Asp Asn Val Glu Met Thr Val Glu Leu Ile Ala Pro Ile Ala
           355                     360                     365
   Ile Glu Asp Gly Thr Arg Phe Ser Ile Arg Glu Gly Gly Arg Thr Val
       370                     375                     380
   Gly Ser Gly Val Val Thr Glu Ile Ile Lys
   385                     390
<210> 22
   <211> 259
   <212> PRT
   <213> Staphylococcus aureus
<400> 22
   Met Gln Ala Leu Gln Thr Phe Asn Phe Glu Glu Leu Pro Val Arg Thr
   1               5                   10                  15
   Leu Glu Val Asp Gly Glu Pro Tyr Phe Ile Gly Lys Asp Val Ala Asp
               20                  25                  30
   Ile Leu Gly Tyr Ala Asn Gly Arg Asp Ala Leu Ser Lys His Val Asp
           35                  40                  45
   Glu Asp Asp Lys Lys Val Leu Thr Ser Arg Asn Thr Thr Leu Glu Asn
       50                  55                  60
   Leu Pro Asn Arg Gly Leu Thr Ala Val Asn Glu Ser Gly Leu Tyr Ser
   65                  70                  75                  80
   Leu Ile Phe Ser Ser Lys Leu Glu Ser Ala Lys Arg Phe Lys Arg Trp
                   85                  90                  95
   Val Thr Ser Asp Val Leu Pro Ala Ile Arg Lys Tyr Gly Ile Tyr Ala
               100                 105                 110
   Thr Asp Asn Val Ile Glu Gln Thr Leu Lys Asp Pro Asp Tyr Ile Ile
           115                 120                 125
   Thr Val Leu Thr Glu Tyr Lys Lys Glu Lys Glu Gln Asn Leu Leu Leu
       130                 135                 140
   Gln Gln Glu Ile Gly Glu Leu Lys Pro Lys Ala Asp Tyr Val Asp Glu
   145                 150                 155                 160
   Ile Leu Lys Ser Thr Gly Thr Leu Ala Thr Thr Gln Ile Ala Ala Asp
                   165                 170                 175
   Tyr Gly Ile Ser Ala Gln Lys Leu Asn Lys Leu Leu His Glu Ala Arg
               180                 185                 190
   Leu Gln Arg Lys Val Asn Lys Gln Trp Val Leu Tyr Ser Glu His Met
           195                 200                 205
   Gly Lys Ser Tyr Thr Asp Ser Asp Thr Ile Thr Ile Val Arg Ser Asp
       210                 215                 220
   Gly Arg Glu Asp Thr Val Leu Gln Thr Arg Trp Thr Gln Lys Gly Arg
   225                 230                 235                 240
   Leu Lys Ile His Glu Ile Met Thr Glu Phe Gly Tyr Glu Ala Asn Leu
                   245                 250                 255
   Gly Gly Ala
<210>
   <211>
   <212> PRT
   <213> Staphylococcus aureus
<400> 23
<210> 24
   <211> 470
   <212> PRT
   <213> Staphylococcus aureus
<400> 24
   Met Gly Ile Gly Arg Val Thr Gln Val Met Gly Pro Val Ile Asp Val
   1               5                   10                  15
   Arg Phe Glu His Asn Glu Val Pro Lys Ile Asn Asn Ala Leu Val Ile
               20                  25                  30
   Asp Val Pro Lys Glu Glu Gly Thr Ile Gln Leu Thr Leu Glu Val Ala
           35                  40                  45
   Leu Gln Leu Gly Asp Asp Val Val Arg Thr Ile Ala Met Asp Ser Thr
       50                  55                  60
   Asp Gly Val Gln Arg Gly Met Asp Val Lys Asp Thr Gly Lys Glu Ile
   65                  70                  75                  80
   Ser Val Pro Val Gly Asp Glu Thr Leu Gly Arg Val Phe Asn Val Leu
                   85                  90                  95
   Gly Glu Thr Ile Asp Leu Lys Glu Glu Ile Ser Asp Ser Val Arg Arg
               100                 105                 110
   Asp Pro Ile His Arg Gln Ala Pro Ala Phe Asp Glu Leu Ser Thr Glu
           115                 120                 125
   Val Gln Ile Leu Glu Thr Gly Ile Lys Val Val Asp Leu Leu Ala Pro
       130                 135                 140
   Tyr Ile Lys Gly Gly Lys Ile Gly Leu Phe Gly Gly Ala Gly Val Gly
   145                 150                 155                 160
   Lys Thr Val Leu Ile Gln Glu Leu Ile Asn Asn Ile Ala Gln Glu His
                   165                 170                 175
   Gly Gly Ile Ser Val Phe Ala Gly Val Gly Glu Arg Thr Arg Glu Gly
               180                 185                 190
   Asn Asp Leu Tyr Phe Glu Met Ser Asp Ser Gly Val Ile Lys Lys Thr
           195                 200                 205
   Ala Met Val Phe Gly Gln Met Asn Glu Pro Pro Gly Ala Arg Met Arg
       210                 215                 220
   Val Ala Leu Ser Gly Leu Thr Met Ala Glu Tyr Phe Arg Asp Glu Gln
   225                 230                 235                 240
   Gly Gln Asp Val Leu Leu Phe Ile Asp Asn Ile Phe Arg Phe Thr Gln
                   245                 250                 255
   Ala Gly Ser Glu Val Ser Ala Leu Leu Gly Arg Met Pro Ser Ala Val
               260                 265                 270
   Gly Tyr Gln Pro Thr Leu Ala Thr Glu Met Gly Gln Leu Gln Glu Arg
           275                 280                 285
   Ile Thr Ser Thr Thr Lys Gly Ser Val Thr Ser Ile Gln Ala Val Phe
       290                 295                 300
   Val Pro Ala Asp Asp Tyr Thr Asp Pro Ala Pro Ala Thr Ala Phe Ala
   305                 310                 315                 320
   His Leu Asp Ala Thr Thr Asn Leu Glu Arg Lys Leu Thr Glu Met Gly
                   325                 330                 335
   Ile Tyr Pro Ala Val Asp Pro Leu Ala Ser Thr Ser Arg Ala Leu Glu
               340                 345                 350
   Pro Ser Ile Val Gly Gln Glu His Tyr Glu Val Ala Arg Asp Val Gln
           355                 360                 365
   Ser Thr Leu Gln Lys Tyr Arg Glu Leu Gln Asp Ile Ile Ala Ile Leu
       370                 375                 380
   Gly Met Asp Glu Leu Ser Asp Glu Asp Lys Gln Thr Val Glu Arg Ala
   385                 390                 395                 400
   Arg Arg Ile Gln Phe Phe Leu Ser Gln Asn Phe His Val Ala Glu Gln
                   405                 410                 415
   Phe Thr Gly Gln Lys Gly Ser Tyr Val Pro Val Lys Thr Thr Val Ala
               420                 425                 430
   Asn Phe Lys Asp Ile Leu Asp Gly Lys Tyr Asp His Ile Pro Glu Asp
           435                 440                 445
   Ala Phe Arg Leu Val Gly Ser Met Asp Asp Val Ile Ala Lys Ala Lys
       450                 455                 460
   Asp Met Gly Val Glu Val
   465                 470
<210> 25
   <211> 372
   <212> PRT
   <213> Staphylococcus aureus
<400> 25
   Met Lys Ile Gly Ile Pro Arg Glu Ile Lys Asn Asn Glu Asn Arg Val
   1               5                   10                  15
   Gly Leu Ser Pro Ser Gly Val His Ala Leu Val Glu Ser Gly His Thr
               20                  25                  30
   Val Leu Val Glu Thr Asn Ala Gly Ser Gly Ser Phe Phe Glu Asp Val
           35                  40                  45
   Asp Tyr Lys Glu Ala Gly Ala Glu Ile Val Ala Glu Gln Ala Lys Val
   50 55 60
   Trp Asp Val Asp Met Val Ile Lys Val Lys Glu Pro Leu Glu Ser Glu
   65 70 75 80
   Tyr Pro Tyr Phe Lys Glu Gly Leu Val Leu Phe Thr Tyr Leu His Leu
   85 90 95
   Ala Asn Glu Glu Lys Leu Thr Gln Ala Leu Ile Asp Arg Lys Val Ile
   100 105 110
   Ser Ile Ala Tyr Glu Thr Val Gln Leu Pro Asp Arg Ser Leu Pro Leu
   115 120 125
   Leu Ser Pro Met Ser Glu Val Ala Gly Arg Met Ser Ala Gln Val Gly
   130 135 140
   Ala Glu Phe Leu Gln Lys Leu Asn Gly Gly Met Gly Ile Leu Leu Gly
   145 150 155 160
   Gly Val Pro Gly Val Pro Lys Gly Lys Val Thr Ile Ile Gly Gly Gly
   165 170 175
   Gln Ala Gly Thr Asn Ala Ala Lys Ile Ala Leu Gly Leu Gly Ala Asp
   180 185 190
   Val Thr Ile Leu Asp Val Asn Pro Lys Arg Leu Gln Gln Leu Asp Asp
   195 200 205
   Leu Phe Gly Gly Arg Val His Thr Ile Met Ser Asn Pro Leu Asn Ile
   210 215 220
   Glu Leu Tyr Val Lys Gln Ser Asp Leu Val Ile Gly Ala Val Leu Ile
   225 230 235 240
   Pro Gly Ala Lys Ala Pro Arg Leu Val Thr Glu Asp Met Ile Lys Gln
   245 250 255
   Met Lys Asn Gly Ser Val Ile Ile Asp Ile Ala Ile Asp Gln Gly Gly
   260 265 270
   Ile Phe Glu Thr Thr Asp Lys Ile Thr Thr His Asp Asp Pro Thr Tyr
   275 280 285
   Ile Lys His Gly Val Val His Tyr Ala Val Ala Asn Met Pro Gly Ala
   290 295 300
   Val Pro Arg Thr Ser Thr Leu Ala Leu Asn Asn Ala Thr Leu Pro Tyr
   305 310 315 320
   Ala Leu Met Leu Ala Asn Lys Gly Tyr Arg Glu Ala Phe Lys Ser Asn
   325 330 335
   Gln Pro Leu Ser Leu Gly Leu Asn Thr Tyr Lys Gly His Val Thr Asn
   340 345 350
   Lys Gly Val Ala Glu Ala Phe Glu Met Glu Tyr Lys Ser Val Glu Glu
   355 360 365
   Ala Leu Gln Leu
   370
<210> 26
   <211> 392
   <212> PRT
   <213> Staphylococcus aureus
<400> 26
   Met Thr Arg Pro Phe Asn Arg Val His Leu Ile Val Met Asp Ser Val
   1               5                   10                  15
   Gly Ile Gly Glu Ala Pro Asp Ala Ala Asp Phe Lys Asp Glu Gly Ser
               20                  25                  30
   His Thr Leu Arg His Thr Leu Glu Gly Phe Asp Gln Thr Leu Pro Asn
           35                  40                  45
   Leu Glu Lys Leu Gly Leu Gly Asn Ile Asp Lys Leu Pro Val Val Asn
       50                  55                  60
   Ala Val Glu Gln Pro Glu Ala Tyr Tyr Thr Lys Leu Ser Glu Ala Ser
   65                  70                  75                  80
   Val Gly Lys Asp Thr Met Thr Gly His Trp Glu Ile Met Gly Leu Asn
                   85                  90                  95
   Ile Met Gln Pro Phe Lys Val Tyr Pro Asn Gly Phe Pro Glu Glu Leu
               100                 105                 110
   Ile Gln Gln Ile Glu Glu Met Thr Gly Arg Lys Val Val Ala Asn Lys
           115                 120                 125
   Pro Ala Ser Gly Thr Gln Ile Ile Asp Glu Trp Gly Glu His Gln Met
       130                 135                 140
   Lys Thr Gly Asp Leu Ile Val Tyr Thr Ser Ala Asp Pro Val Leu Gln
   145                 150                 155                 160
   Ile Ala Ala His Glu Asp Ile Ile Pro Leu Glu Glu Leu Tyr Asp Ile
                   165                 170                 175
   Cys Glu Lys Val Arg Glu Leu Thr Lys Asp Pro Lys Tyr Leu Ile Gly
               180                 185                 190
   Arg Ile Ile Ala Arg Pro Tyr Val Gly Glu Pro Gly Asn Phe Thr Arg
           195                 200                 205
   Thr Ser Asn Arg His Asp Tyr Ala Leu Lys Pro Phe Gly Lys Thr Val
       210                 215                 220
   Leu Asp His Leu Lys Asp Gly Gly Tyr Asp Val Ile Ala Ile Gly Lys
   225                 230                 235                 240
   Ile Asn Asp Ile Tyr Asp Gly Glu Gly Val Thr Glu Ala Val Arg Thr
                   245                 250                 255
   Lys Ser Asn Met Asp Gly Met Asp Gln Leu Met Lys Ile Val Lys Lys
               260                 265                 270
   Asp Phe Thr Gly Ile Ser Phe Leu Asn Leu Val Asp Phe Asp Ala Leu
           275                 280                 285
   Tyr Gly His Arg Arg Asp Lys Pro Gly Tyr Ala Gln Ala Ile Lys Asp
       290                 295                 300
   Phe Asp Asp Arg Leu Pro Glu Leu Phe Ser Asn Leu Lys Glu Asp Asp
   305                 310                 315                 320
   Leu Val Ile Ile Thr Ala Asp His Gly Asn Asp Pro Thr Ala Pro Gly
                   325                 330                 335
   Thr Asp His Thr Arg Glu Tyr Ile Pro Val Ile Met Tyr Ser Pro Lys
               340                 345                 350
   Phe Lys Gly Gly His Ala Leu Glu Ser Asp Thr Thr Phe Ser Ser Ile
           355                 360                 365
   Gly Ala Thr Ile Ala Asp Asn Phe Asn Val Thr Leu Pro Glu Phe Gly
       370                 375                 380
   Lys Ser Tyr Leu Lys Glu Leu Lys
   385 390
<210> 27
   <211> 414
   <212> PRT
   <213> Staphylococcus aureus
<400> 27
   Met Thr Glu Asn Asn Asn Leu Val Thr Ser Thr Gln Gly Ile Ile Lys
   1               5                   10                  15
   Glu Ala Leu His Lys Leu Gly Phe Asp Glu Gly Met Tyr Asp Leu Ile
               20                  25                  30
   Lys Glu Pro Leu Arg Met Leu Gln Val Arg Ile Pro Val Arg Met Asp
           35                  40                  45
   Asp Gly Thr Val Lys Thr Phe Thr Gly Tyr Arg Ala Gln His Asn Asp
       50                  55                  60
   Ala Val Gly Pro Thr Lys Gly Gly Val Arg Phe His Pro Asp Val Asp
   65                  70                  75                  80
   Glu Glu Glu Val Lys Ala Leu Ser Met Trp Met Thr Leu Lys Cys Gly
                   85                  90                  95
   Ile Val Asn Leu Pro Tyr Gly Gly Gly Lys Gly Gly Ile Val Cys Asp
               100                 105                 110
   Pro Arg Gln Met Ser Ile His Glu Val Glu Arg Leu Ser Arg Gly Tyr
           115                 120                 125
   Val Arg Ala Ile Ser Gln Phe Val Gly Pro Asn Lys Asp Ile Pro Ala
       130                 135                 140
   Pro Asp Val Phe Thr Asn Ser Gln Ile Met Ala Trp Met Met Asp Glu
   145                 150                 155                 160
   Tyr Ser Ala Leu Asp Lys Phe Asn Ser Pro Gly Phe Ile Thr Gly Lys
                   165                 170                 175
   Pro Ile Val Leu Gly Gly Ser His Gly Arg Asp Arg Ser Thr Ala Leu
               180                 185                 190
   Gly Val Val Ile Ala Ile Glu Gln Ala Ala Lys Arg Arg Asn Met Gln
           195                 200                 205
   Ile Glu Gly Ala Lys Val Val Ile Gln Gly Phe Gly Asn Ala Gly Ser
   210 215 220
   Phe Leu Ala Lys Phe Leu Tyr Asp Leu Gly Ala Lys Ile Val Gly Ile
   225 230 235 240
   Ser Asp Ala Tyr Gly Ala Leu His Asp Pro Asn Gly Leu Asp Ile Asp
   245 250 255
   Tyr Leu Leu Asp Arg Arg Asp Ser Phe Gly Thr Val Thr Asn Leu Phe
   260 265 270
   Glu Glu Thr Ile Ser Asn Lys Glu Leu Phe Glu Leu Asp Cys Asp Ile
   275 280 285
   Leu Val Pro Ala Ala Ile Ser Asn Gln Ile Thr Glu Asp Asn Ala His
   290 295 300
   Asp Ile Lys Ala Ser Ile Val Val Glu Ala Ala Asn Gly Pro Thr Thr
   305 310 315 320
   Pro Glu Ala Thr Arg Ile Leu Thr Glu Arg Gly Ile Leu Leu Val Pro
   325 330 335
   Asp Val Leu Ala Ser Ala Gly Gly Val Thr Val Ser Tyr Phe Glu Trp
   340 345 350
   Val Gln Asn Asn Gln Gly Tyr Tyr Trp Ser Glu Glu Glu Val Asn Glu
   355 360 365
   Lys Leu Arg Glu Lys Leu Glu Ala Ala Phe Asp Thr Ile Tyr Glu Leu
   370 375 380
   Ser Gln Asn Arg Lys Ile Asp Met Arg Leu Ala Ala Tyr Ile Ile Gly
   385 390 395 400
   Ile Lys Arg Thr Ala Glu Ala Ala Arg Tyr Arg Gly Trp Ala
   405 410
<210> 28
   <211> 619
   <212> PRT
   <213> Staphylococcus aureus
<400> 28
   Met Pro Lys Asn Lys Ile Leu Ile Tyr Leu Leu Ser Thr Thr Leu Val
   1 5 10 15
   Leu Pro Thr Leu Val Ser Pro Thr Ala Tyr Ala Asp Thr Pro Gln Lys
   20 25 30
   Asp Thr Thr Ala Lys Thr Thr Ser His Asp Ser Lys Lys Ser Asn Asp
   35 40 45
   Asp Glu Thr Ser Lys Asp Thr Thr Ser Lys Asp Thr Asp Lys Ala Asp
   50 55 60
   Asn Asn Asn Thr Ser Asn Gln Asp Asn Asn Asp Lys Lys Phe Lys Thr
   65 70 75 80
   Ile Asp Asp Ser Thr Ser Asp Ser Asn Asn Ile Ile Asp Phe Ile Tyr
   85 90 95
   Lys Asn Leu Pro Gln Thr Asn Ile Asn Gln Leu Leu Thr Lys Asn Lys
   100 105 110
   Tyr Asp Asp Asn Tyr Ser Leu Thr Thr Leu Ile Gln Asn Leu Phe Asn
   115 120 125
   Leu Asn Ser Asp Ile Ser Asp Tyr Glu Gln Pro Arg Asn Gly Glu Lys
   130 135 140
   Ser Thr Asn Asp Ser Asn Lys Asn Ser Asp Asn Ser Ile Lys Asn Asp
   145 150 155 160
   Thr Asp Thr Gln Ser Ser Lys Gln Asp Lys Ala Asp Asn Gln Lys Ala
   165 170 175
   Pro Lys Ser Asn Asn Thr Lys Pro Ser Thr Ser Asn Lys Gln Pro Asn
   180 185 190
   Ser Pro Lys Pro Thr Gln Pro Asn Gln Ser Asn Ser Gln Pro Ala Ser
   195 200 205
   Asp Asp Lys Ala Asn Gln Lys Ser Ser Ser Lys Asp Asn Gln Ser Met
   210 215 220
   Ser Asp Ser Ala Leu Asp Ser Ile Leu Asp Gln Tyr Ser Glu Asp Ala
   225 230 235 240
   Lys Lys Thr Gln Lys Asp Tyr Ala Ser Gln Ser Lys Lys Asp Lys Asn
   245 250 255
   Glu Lys Ser Asn Thr Lys Asn Pro Gln Leu Pro Thr Gln Asp Glu Leu
   260 265 270
   Lys His Lys Ser Lys Pro Ala Gln Ser Phe Asn Asn Asp Val Asn Gln
           275                 280                 285
   Lys Asp Thr Arg Ala Thr Ser Leu Phe Glu Thr Asp Pro Ser Ile Ser
       290                 295                 300
   Asn Asn Asp Asp Ser Gly Gln Phe Asn Val Val Asp Ser Lys Asp Thr
   305                 310                 315                 320
   Arg Gln Phe Val Lys Ser Ile Ala Lys Asp Ala His Arg Ile Gly Gln
                   325                 330                 335
   Asp Asn Asp Ile Tyr Ala Ser Val Met Ile Ala Gln Ala Ile Leu Glu
               340                 345                 350
   Ser Asp Ser Gly Arg Ser Ala Leu Ala Lys Ser Pro Asn His Asn Leu
           355                 360                 365
   Phe Gly Ile Lys Gly Ala Phe Glu Gly Asn Ser Val Pro Phe Asn Thr
       370                 375                 380
   Leu Glu Ala Asp Gly Asn Lys Leu Tyr Ser Ile Asn Ala Gly Phe Arg
   385                 390                 395                 400
   Lys Tyr Pro Ser Thr Lys Glu Ser Leu Lys Asp Tyr Ser Asp Leu Ile
                   405                 410                 415
   Lys Asn Gly Ile Asp Gly Asn Arg Thr Ile Tyr Lys Pro Thr Trp Lys
               420                 425                 430
   Ser Glu Ala Asp Ser Tyr Lys Asp Ala Thr Ser His Leu Ser Lys Thr
           435                 440                 445
   Tyr Ala Thr Asp Pro Asn Tyr Ala Lys Lys Leu Asn Ser Ile Ile Lys
       450                 455                 460
   His Tyr Gln Leu Thr Gln Phe Asp Asp Glu Arg Met Pro Asp Leu Asp
   465                 470                 475                 480
   Lys Tyr Glu Arg Ser Ile Lys Asp Tyr Asp Asp Ser Ser Asp Glu Phe
                   485                 490                 495
   Lys Pro Phe Arg Glu Val Ser Asp Ser Met Pro Tyr Pro His Gly Gln
               500                 505                 510
   Cys Thr Trp Tyr Val Tyr Asn Arg Met Lys Gln Phe Gly Thr Ser Ile
           515                 520                 525
   Ser Gly Asp Leu Gly Asp Ala His Asn Trp Asn Asn Arg Ala Gln Tyr
       530                 535                 540
   Arg Asp Tyr Gln Val Ser His Thr Pro Lys Arg His Ala Ala Val Val
   545                 550                 555                 560
   Phe Glu Ala Gly Gln Phe Gly Ala Asp Gln His Tyr Gly His Val Ala
                   565                 570                 575
   Phe Val Glu Lys Val Asn Ser Asp Gly Ser Ile Val Ile Ser Glu Ser
               580                 585                 590
   Asn Val Lys Gly Leu Gly Ile Ile Ser His Arg Thr Ile Asn Ala Ala
           595                 600                 605
   Ala Ala Glu Glu Leu Ser Tyr Ile Thr Gly Lys
       610                 615
<210> 29
   <211> 363
   <212> PRT
   <213> Staphylococcus aureus
<400> 29
   Met Ser Asn Lys Leu Glu Ser Tyr Arg Ser Glu Ile Val Ser Leu Asn
   1 5 10 15
   His Gln Ile Leu Asp Leu Leu Ser Lys Arg Gly Glu Leu Ala Gln Lys
   20 25 30
   Ile Gly Glu Glu Lys Leu Lys Gln Gly Thr Arg Ile Tyr Asp Pro Gln
   35 40 45
   Arg Glu Lys Glu Met Leu Asn Asp Leu Ile Asp Ser Asn Lys Gly Pro
   50 55 60
   Phe Asn Asp Asn Thr Ile Lys Gln Leu Phe Lys Glu Ile Phe Lys Ala
   65 70 75 80
   Ser Thr Asp Leu Gln Lys Ser Glu Asn Glu Lys His Leu Tyr Val Ser
   85 90 95
   Arg Lys Leu Lys Pro Glu Asp Thr Ile Val Thr Phe Asp Asn Gly Gly
   100 105 110
   Ile Ile Gly Asp Gly Asn Lys Ser Phe Val Phe Gly Pro Cys Ser Val
   115 120 125
   Glu Ser Phe Glu Gln Val Glu Ala Val Ala Lys Asn Leu His Ala Lys
       130                 135                 140
   Gly Glu Lys Phe Ile Arg Gly Gly Ala Phe Lys Pro Arg Thr Ser Pro
   145                 150                 155                 160
   Tyr Asp Phe Gln Gly Leu Gly Val Glu Gly Leu Lys Ile Leu Lys Gln
                   165                 170                 175
   Ile Lys Asp Lys Tyr Asp Leu Asn Val Val Ser Glu Ile Val Asn Pro
               180                 185                 190
   Asn Asp Phe Glu Val Ala Asp Glu Tyr Leu Asp Val Phe Gln Ile Gly
           195                 200                 205
   Ala Arg Asn Met Gln Asn Phe Glu Leu Leu Lys Glu Ala Gly Arg Thr
       210                 215                 220
   Lys Lys Pro Ile Leu Leu Lys Arg Gly Leu Ser Ala Thr Ile Glu Glu
   225                 230                 235                 240
   Phe Val Tyr Ala Ala Glu Tyr Ile Ala Ser Gln Gly Asn Gln Asn Ile
                   245                 250                 255
   Ile Leu Cys Glu Arg Gly Ile Arg Thr Tyr Glu Lys Ala Thr Arg Asn
               260                 265                 270
   Thr Leu Asp Ile Ser Ala Val Pro Ile Leu Lys Gln Gly Thr His Leu
           275                 280                 285
   Pro Val Met Val Asp Val Thr His Ser Thr Gly Arg Lys Asp Ile Met
       290                 295                 300
   Leu Pro Thr Ala Lys Ala Ala Leu Ala Val Gly Ala Asp Gly Val Met
   305                 310                 315                 320
   Ala Glu Val His Pro Asp Pro Ser Val Ala Leu Ser Asp Ala Gly Gln
                   325                 330                 335
   Gln Met Asp Leu Asp Glu Phe Gln Ala Phe Tyr Asp Glu Leu Lys Pro
               340                 345                 350
   Leu Ala Asp Leu Tyr Asn Ala Lys Lys Leu Lys
           355                 360
<210> 30
   <211> 231
   <212> PRT
   <213> Staphylococcus aureus
<400> 30
   Met Lys Lys Thr Leu Leu Ala Ser Ser Leu Ala Val Gly Leu Gly Ile
   1               5                   10                  15
   Val Ala Gly Asn Ala Gly His Glu Ala His Ala Ser Glu Ala Asp Leu
               20                  25                  30
   Asn Lys Ala Ser Leu Ala Gln Met Ala Gln Ser Asn Asp Gln Thr Leu
           35                  40                  45
   Asn Gln Lys Pro Ile Glu Ala Gly Ala Tyr Asn Tyr Thr Phe Asp Tyr
       50                  55                  60
   Glu Gly Phe Thr Tyr His Phe Glu Ser Asp Gly Thr His Phe Ala Trp
   65                  70                  75                  80
   Asn Tyr His Ala Thr Gly Ala Asn Gly Ala Asn Met Ser Ala Gln Ala
                   85                  90                  95
   Pro Ala Thr Asn Asn Val Glu Pro Ser Ala Val Gln Ala Asn Gln Val
               100                 105                 110
   Gln Ser Gln Glu Val Glu Ala Pro Gln Asn Ala Gln Thr Gln Gln Pro
           115                 120                 125
   Gln Ala Ser Thr Ser Asn Asn Ser Gln Val Thr Ala Thr Pro Thr Glu
       130                 135                 140
   Ser Lys Ala Ser Glu Gly Ser Ser Val Asn Val Asn Ala His Leu Lys
   145                 150                 155                 160
   Gln Ile Ala Gln Arg Glu Ser Gly Gly Asn Ile His Ala Val Asn Pro
                   165                 170                 175
   Thr Ser Gly Ala Ala Gly Lys Tyr Gln Phe Leu Gln Ser Thr Trp Asp
               180                 185                 190
   Ser Val Ala Pro Ala Lys Tyr Lys Gly Val Ser Pro Ala Asn Ala Pro
           195                 200                 205
   Glu Ser Val Gln Asp Ala Ala Ala Val Lys Leu Tyr Asn Thr Gly Gly
       210                 215                 220
   Ala Gly His Trp Val Thr Ala
   225                 230
<210> 31
   <211> 315
   <212> PRT
   <213> Staphylococcus aureus
<400> 31
   Met Asp Ile Glu Leu Thr Lys Lys Asp Gly Thr Val Ile Lys Leu Ser
   1               5                   10                  15
   Glu Tyr Gly Phe Ile Val Asn Asp Ile Val Ile Asp Ser Met Gln Ile
               20                  25                  30
   Asn Thr Lys Tyr Gln Asp Lys Glu Asn Met Asn Gly Arg Ile Leu Met
           35                  40                  45
   Gly Ser Asn Tyr Ile Ser Arg Asp Ile Val Val Pro Cys Phe Cys Val
       50                  55                  60
   Val Lys Asn Arg Ser Asp Ile Ala Tyr Met Arg Asp Met Leu Tyr Ser
   65                  70                  75                  80
   Leu Thr Thr Asp Ile Glu Pro Met Tyr Leu Arg Glu Ile Arg Arg Lys
                   85                  90                  95
   Glu Glu Leu Asn Tyr Arg Phe Thr Gln Pro Ile Ser Asp Asp Tyr Val
               100                 105                 110
   Lys Leu Asp Lys Asn Asn Phe Pro Asp Tyr Glu Tyr Ser Arg His Asp
           115                 120                 125
   Gln Gln Asn Tyr Val Asn Gly Lys Gln Tyr Lys Val Ile Phe Asn Gly
       130                 135                 140
   Val Ile Asn Pro Lys Gln Lys Gly Asn Lys Val Ser Phe Glu Leu Lys
   145                 150                 155                 160
   Phe Glu Thr Thr Glu Leu Pro Tyr Gly Glu Ser Ile Gly Thr Ser Leu
                   165                 170                 175
   Glu Leu Glu Glu Asn Lys Lys Val Gly Leu Trp Ser Phe Asp Phe Asn
               180                 185                 190
   Ile Asp Trp His Ala Gly Gly Asp Lys Arg Lys Tyr Thr Phe Glu Asn
           195                 200                 205
   Leu Ser Lys Gly Thr Val Tyr Tyr His Gly Ser Ala Pro Asn Asp Gln
       210                 215                 220
   Phe Asn Met Tyr Lys Lys Ile Thr Ile Ile Leu Gly Glu Asp Thr Glu
   225                 230                 235                 240
   Ser Phe Val Trp Asn Leu Thr His Ala Glu Ile Met Lys Ile Glu Gly
                   245                 250                 255
   Ile Lys Leu Lys Thr Gly Asp Arg Ile Val Tyr Asp Ser Phe Arg Val
               260                 265                 270
   Tyr Lys Asn Gly Val Glu Ile Ser Thr Glu Thr Asn Ile Ala Gln Pro
           275                 280                 285
   Lys Phe Lys Tyr Gly Ala Asn Lys Phe Glu Phe Asn Gln Thr Val Gln
       290                 295                 300
   Lys Val Gln Phe Asp Leu Lys Phe Tyr Tyr Lys
   305                 310                 315
<210> 32
   <211> 240
   <212> PRT
   <213> Staphylococcus aureus
<400> 32
   Met Ala Gln Ile Ser Lys Tyr Lys Arg Val Val Leu Lys Leu Ser Gly
   1               5                   10                  15
   Glu Ala Leu Ala Gly Glu Lys Gly Phe Gly Ile Asn Pro Val Ile Ile
               20                  25                  30
   Lys Ser Val Ala Glu Gln Val Ala Glu Val Ala Lys Met Asp Cys Glu
           35                  40                  45
   Ile Ala Val Ile Val Gly Gly Gly Asn Ile Trp Arg Gly Lys Thr Gly
       50                  55                  60
   Ser Asp Leu Gly Met Asp Arg Gly Thr Ala Asp Tyr Met Gly Met Leu
   65                  70                  75                  80
   Ala Thr Val Met Asn Ala Leu Ala Leu Gln Asp Ser Leu Glu Gln Leu
                   85                  90                  95
   Asp Cys Asp Thr Arg Val Leu Thr Ser Ile Glu Met Lys Gln Val Ala
               100                 105                 110
   Glu Pro Tyr Ile Arg Arg Arg Ala Ile Arg His Leu Glu Lys Lys Arg
           115                 120                 125
   Val Val Ile Phe Ala Ala Gly Ile Gly Asn Pro Tyr Phe Ser Thr Asp
       130                 135                 140
   Thr Thr Ala Ala Leu Arg Ala Ala Glu Val Glu Ala Asp Val Ile Leu
   145                 150                 155                 160
   Met Gly Lys Asn Asn Val Asp Gly Val Tyr Ser Ala Asp Pro Lys Val
                   165                 170                 175
   Asn Lys Asp Ala Val Lys Tyr Glu His Leu Thr His Ile Gln Met Leu
               180                 185                 190
   Gln Glu Gly Leu Gln Val Met Asp Ser Thr Ala Ser Ser Phe Cys Met
           195                 200                 205
   Asp Asn Asn Ile Pro Leu Thr Val Phe Ser Ile Met Glu Glu Gly Asn
       210                 215                 220
   Ile Lys Arg Ala Val Met Gly Glu Lys Ile Gly Thr Leu Ile Thr Lys
   225                 230                 235                 240
<210> 33
   <211> 257
   <212> PRT
   <213> Staphylococcus aureus
<400> 33
   Met Ser Leu Leu Ser Lys Thr Arg Glu Leu Asn Thr Leu Leu Gln Lys
   1               5                   10                  15
   His Lys Gly Ile Ala Val Asp Phe Lys Asp Val Ala Gln Thr Ile Ser
               20                  25                  30
   Ser Val Thr Val Thr Asn Val Phe Ile Val Ser Arg Arg Gly Lys Ile
           35                  40                  45
   Leu Gly Ser Ser Leu Asn Glu Leu Leu Lys Ser Gln Arg Ile Ile Gln
       50                  55                  60
   Met Leu Glu Glu Arg His Ile Pro Ser Glu Tyr Thr Glu Arg Leu Met
   65                  70                  75                  80
   Glu Val Lys Gln Thr Glu Ser Asn Ile Asp Ile Asp Asn Val Leu Thr
                   85                  90                  95
   Val Phe Pro Pro Glu Asn Arg Glu Leu Phe Ile Asp Ser Arg Thr Thr
               100                 105                 110
   Ile Phe Pro Ile Leu Gly Gly Gly Glu Arg Leu Gly Thr Leu Val Leu
           115                 120                 125
   Gly Arg Val His Asp Asp Phe Asn Glu Asn Asp Leu Val Leu Gly Glu
       130                 135                 140
   Tyr Ala Ala Thr Val Ile Gly Met Glu Ile Leu Arg Glu Lys His Ser
   145                 150                 155                 160
   Glu Val Glu Lys Glu Ala Arg Asp Lys Ala Ala Ile Thr Met Ala Ile
                   165                 170                 175
   Asn Ser Leu Ser Tyr Ser Glu Lys Glu Ala Ile Glu His Ile Phe Glu
               180                 185                 190
   Glu Leu Gly Gly Thr Glu Gly Leu Leu Ile Ala Ser Lys Val Ala Asp
           195                 200                 205
   Arg Val Gly Ile Thr Arg Ser Val Ile Val Asn Ala Leu Arg Lys Leu
       210                 215                 220
   Glu Ser Ala Gly Val Ile Glu Ser Arg Ser Leu Gly Met Lys Gly Thr
   225                 230                 235                 240
   Phe Ile Lys Val Lys Lys Glu Lys Phe Leu Asp Glu Leu Glu Lys Ser
                   245                 250                 255
   Lys
<210> 34
   <211> 239
   <212> PRT
   <213> Staphylococcus aureus
<400> 34
   Met Lys Arg Ile Leu Ile Ile Val Val Leu Leu Phe Cys Tyr Ser Gln
   1               5                   10                  15
   Asn His Ile Ala Thr Ala Asp Val Gly Val Leu Asn Leu Arg Asn Tyr
               20                  25                  30
   Tyr Gly Ser Tyr Pro Ile Glu Asp His Gln Ser Ile Asn Pro Glu Asn
           35                  40                  45
   Asn His Leu Ser His Gln Leu Val Phe Ser Met Asp Asn Ser Thr Val
       50                  55                  60
   Thr Ala Glu Phe Lys Asn Val Asp Asp Val Lys Lys Phe Lys Asn His
   65                  70                  75                  80
   Ala Val Asp Val Tyr Gly Leu Ser Tyr Ser Gly Tyr Cys Leu Lys Asn
                   85                  90                  95
   Lys Tyr Ile Tyr Gly Gly Val Thr Leu Ala Gly Asp Tyr Leu Glu Lys
               100                 105                 110
   Ser Arg Arg Ile Pro Ile Asn Leu Trp Val Asn Gly Glu His Gln Thr
           115                 120                 125
   Ile Ser Thr Asp Lys Val Ser Thr Asn Lys Lys Leu Val Thr Ala Gln
       130                 135                 140
   Glu Ile Asp Thr Lys Leu Arg Arg Tyr Leu Gln Glu Glu Tyr Asn Ile
   145                 150                 155                 160
   Tyr Gly Phe Asn Asp Thr Asn Lys Gly Arg Asn Tyr Gly Asn Lys Ser
                   165                 170                 175
   Lys Phe Ser Ser Gly Phe Asn Ala Gly Lys Ile Leu Phe His Leu Asn
               180                 185                 190
   Asp Gly Ser Ser Phe Ser Tyr Asp Leu Phe Asp Thr Gly Thr Gly Gln
           195                 200                 205
   Ala Glu Ser Phe Leu Lys Ile Tyr Asn Asp Asn Lys Thr Val Glu Thr
       210                 215                 220
   Glu Lys Phe His Leu Asp Val Glu Ile Ser Tyr Lys Asp Glu Ser
   225                 230                 235
<210> 35
   <211> 265
   <212> PRT
   <213> Staphylococcus aureus
<400> 35
   Met Asn Arg Leu His Gly Gln Gln Val Lys Ile Gly Tyr Gly Asp Asn
   1               5                   10                  15
   Thr Ile Ile Asn Lys Leu Asp Val Glu Ile Pro Asp Gly Lys Val Thr
               20                  25                  30
   Ser Ile Ile Gly Pro Asn Gly Cys Gly Lys Ser Thr Leu Leu Lys Ala
           35                  40                  45
   Leu Ser Arg Leu Leu Ala Val Lys Glu Gly Glu Val Phe Leu Asp Gly
       50                  55                  60
   Glu Asn Ile His Thr Gln Ser Thr Lys Glu Ile Ala Lys Lys Ile Ala
   65                  70                  75                  80
   Ile Leu Pro Gln Ser Pro Glu Val Ala Asp Gly Leu Thr Val Gly Glu
                   85                  90                  95
   Leu Val Ser Tyr Gly Arg Phe Pro His Gln Lys Gly Phe Gly Arg Leu
               100                 105                 110
   Thr Ala Glu Asp Lys Lys Glu Ile Asp Trp Ala Met Glu Val Thr Gly
           115                 120                 125
   Thr Asp Thr Phe Arg His Arg Ser Ile Asn Asp Leu Ser Gly Gly Gln
       130                 135                 140
   Arg Gln Arg Val Trp Ile Ala Met Ala Leu Ala Gln Arg Thr Asp Ile
   145                 150                 155                 160
   Ile Phe Leu Asp Glu Pro Thr Thr Tyr Leu Asp Ile Cys His Gln Leu
                   165                 170                 175
   Glu Ile Leu Glu Leu Val Gln Lys Leu Asn Gln Glu Gln Gly Cys Thr
               180                 185                 190
   Ile Val Met Val Leu His Asp Ile Asn Gln Ala Ile Arg Phe Ser Asp
           195                 200                 205
   His Leu Ile Ala Met Lys Glu Gly Asp Ile Ile Ala Thr Gly Ser Thr
       210                 215                 220
   Glu Asp Val Leu Thr Gln Glu Ile Leu Glu Lys Val Phe Asn Ile Asp
   225                 230                 235                 240
   Val Val Leu Ser Lys Asp Pro Lys Thr Gly Lys Pro Leu Leu Val Thr
                   245                 250                 255
   Tyr Asp Leu Cys Arg Arg Ala Tyr Ser
               260                 265
<210> 36
   <211> 318
   <212> PRT
   <213> Staphylococcus aureus
<400> 36
   Met Asn Tyr Gln Val Leu Leu Tyr Tyr Lys Tyr Thr Thr Ile Asp Asp
   1               5                   10                  15
   Pro Glu Gln Phe Ala Gln Asp His Leu Ala Phe Cys Lys Ala His His
               20                  25                  30
   Leu Lys Gly Arg Ile Leu Val Ser Thr Glu Gly Ile Asn Gly Thr Leu
           35                  40                  45
   Ser Gly Thr Lys Glu Glu Thr Glu Gln Tyr Met Ala His Met His Ala
       50                  55                  60
   Asp Glu Arg Phe Lys Asp Met Val Phe Lys Ile Asp Glu Ala Glu Gly
   65                  70                  75                  80
   His Ala Phe Lys Lys Met His Val Arg Pro Arg Lys Glu Ile Val Ala
                   85                  90                  95
   Leu Asp Leu Glu Asp Asp Val Asp Pro Arg His Thr Thr Gly Gln Tyr
               100                 105                 110
   Leu Ser Pro Val Glu Phe Arg Lys Ala Leu Glu Asp Asp Asp Thr Val
           115                 120                 125
   Ile Ile Asp Ala Arg Asn Asp Tyr Glu Phe Asp Leu Gly His Phe Arg
       130                 135                 140
   Gly Ala Ile Arg Pro Asp Ile Thr Arg Phe Arg Asp Leu Pro Asp Trp
   145                 150                 155                 160
   Ile Lys Glu Asn Lys Ala Leu Phe Thr Asp Lys Lys Val Val Thr Tyr
                   165                 170                 175
   Cys Thr Gly Gly Ile Arg Cys Glu Lys Phe Ser Gly Trp Leu Leu Lys
               180                 185                 190
   Glu Gly Phe Glu Asp Val Ala Gln Leu His Gly Gly Ile Ala Thr Tyr
           195                 200                 205
   Gly Lys Asp Pro Glu Thr Lys Gly Gln Tyr Trp Asp Gly Lys Met Tyr
       210                 215                 220
   Val Phe Asp Asp Arg Ile Ser Val Asp Ile Asn Gln Val Glu Lys Thr
   225                 230                 235                 240
   Ile Ile Gly Lys Asp Trp Phe Asp Gly Lys Pro Cys Glu Arg Tyr Ile
   245 250 255
   Asn Cys Ala Asn Pro Glu Cys Asn Lys Gln Ile Leu Val Ser Glu Glu
   260 265 270
   Asn Glu Thr Lys Tyr Leu Gly Ala Cys Ser Tyr Glu Cys Ala Lys His
   275 280 285
   Glu Arg Asn Arg Tyr Val Gln Ala Asn Asn Ile Ser Asp Asn Glu Trp
   290 295 300
   Gln Gln Arg Leu Thr Asn Phe Asp Asp Leu His Gln His Ala
   305 310 315
<210> 37
   <211> 317
   <212> PRT
   <213> Staphylococcus aureus
<400> 37
<210> 38
   <211> 253
   <212> PRT
   <213> Staphylococcus aureus
<400> 38
   Met Arg Thr Pro Ile Ile Ala Gly Asn Trp Lys Met Asn Lys Thr Val
   1               5                   10                  15
   Gln Glu Ala Lys Asp Phe Val Asn Ala Leu Pro Thr Leu Pro Asp Ser
               20                  25                  30
   Lys Glu Val Glu Ser Val Ile Cys Ala Pro Ala Ile Gln Leu Asp Ala
           35                  40                  45
   Leu Thr Thr Ala Val Lys Glu Gly Lys Ala Gln Gly Leu Glu Ile Gly
       50                  55                  60
   Ala Gln Asn Thr Tyr Phe Glu Asp Asn Gly Ala Phe Thr Gly Glu Thr
   65                  70                  75                  80
   Ser Pro Val Ala Leu Ala Asp Leu Gly Val Lys Tyr Val Val Ile Gly
                   85                  90                  95
   His Ser Glu Arg Arg Glu Leu Phe His Glu Thr Asp Glu Glu Ile Asn
               100                 105                 110
   Lys Lys Ala His Ala Ile Phe Lys His Gly Met Thr Pro Ile Ile Cys
           115                 120                 125
   Val Gly Glu Thr Asp Glu Glu Arg Glu Ser Gly Lys Ala Asn Asp Val
       130                 135                 140
   Val Gly Glu Gln Val Lys Lys Ala Val Ala Gly Leu Ser Glu Asp Gln
   145                 150                 155                 160
   Leu Lys Ser Val Val Ile Ala Tyr Glu Pro Ile Trp Ala Ile Gly Thr
                   165                 170                 175
   Gly Lys Ser Ser Thr Ser Glu Asp Ala Asn Glu Met Cys Ala Phe Val
               180                 185                 190
   Arg Gln Thr Ile Ala Asp Leu Ser Ser Lys Glu Val Ser Glu Ala Thr
           195                 200                 205
   Arg Ile Gln Tyr Gly Gly Ser Val Lys Pro Asn Asn Ile Lys Glu Tyr
       210                 215                 220
   Met Ala Gln Thr Asp Ile Asp Gly Ala Leu Val Gly Gly Ala Ser Leu
   225                 230                 235                 240
   Lys Val Glu Asp Phe Val Gln Leu Leu Glu Gly Ala Lys
                   245                 250
<210> 39
   <211> 353
   <212> PRT
   <213> Staphylococcus aureus
<400> 39
   Met Ser Arg Ile Thr Gln Val His Arg Ile Leu Glu Gln Lys His Leu
   1               5                   10                  15
   Asp Ala Ile Ile Ile Leu Ser Asp Tyr Asn Arg Arg Tyr Leu Ser Gly
               20                  25                  30
   Phe Thr Gly Thr Ser Gly Ala Leu Ile Ile Ser Lys Asp Lys Gln Tyr
           35                  40                  45
   Leu Ile Thr Asp Phe Arg Tyr Ile Asp Gln Ala Thr Lys Gln Ala Pro
       50                  55                  60
   Asn Tyr Glu Ile Ile Asn Arg Lys Ser Thr Ile Ile Gly Glu Ile Lys
   65                  70                  75                  80
   Glu Leu Leu His Gln Glu Asn Phe Glu Asn Ile Gly Phe Glu Gly His
                   85                  90                  95
   His Val Ser Tyr Asp Thr Tyr Leu Glu Leu Asn Lys Ser Arg Ile Ser
               100                 105                 110
   Leu Ile Ser Ile Ser Asn Thr Val Asp Lys Ile Arg Asp Val Lys Asp
           115                 120                 125
   Val Asp Glu Ile Ala Leu Ile Gln Lys Ala Ala Asn Ile Val Asp Glu
       130                 135                 140
   Thr Tyr Glu Tyr Ile Leu Thr Val Val Lys Ala Gly Met Thr Glu Lys
   145                 150                 155                 160
   Glu Leu Lys Ala Ile Leu Glu Ser Lys Met Leu Glu Leu Gly Ala Asp
                   165                 170                 175
   Gly Pro Ser Phe Asp Thr Ile Val Ala Ser Gly His Arg Gly Ala Leu
               180                 185                 190
   Pro His Gly Val Ala Ser Asp Lys Ile Ile Glu Lys Gly Asp Met Ile
           195                 200                 205
   Thr Leu Asp Phe Gly Ala Tyr Tyr Asn Gly Tyr Cys Ser Asp Ile Thr
       210                 215                 220
   Arg Thr Phe Ala Ile Gly Glu Pro Asp Pro Lys Leu Lys Glu Ile Tyr
   225                 230                 235                 240
   Gln Ile Val Leu Glu Ser Gln Met Lys Ala Ile Asn Glu Ile Arg Pro
                   245                 250                 255
   Gly Met Thr Gly Ala Glu Ala Asp Ala Ile Ser Arg Asn Tyr Leu Glu
               260                 265                 270
   Ser Lys Gly Tyr Gly Lys Glu Phe Gly His Ser Leu Gly His Gly Ile
           275                 280                 285
   Gly Leu Glu Ile His Glu Gly Pro Met Leu Ala Arg Thr Ile Gln Asp
       290                 295                 300
   Lys Leu Gln Val Asn Asn Cys Val Thr Val Glu Pro Gly Val Tyr Ile
   305                 310                 315                 320
   Glu Gly Leu Gly Gly Ile Arg Ile Glu Asp Asp Ile Leu Ile Thr Glu
                   325                 330                 335
   Asn Gly Cys Gln Val Phe Thr Lys Cys Thr Lys Asp Leu Ile Val Leu
               340                 345                 350
   Thr
<210> 40
   <211> 23
   <212> DNA
   <213> Synthetic
<400> 40
   caccatgcca attattacag atg 23
<210> 41
   <211> 28
   <212> DNA
   <213> Synthetic
<400> 41
   ttatttatct aagttataga atgatttg 28
<210> 42
   <211> 25
   <212> DNA
   <213> Synthetic
<400> 42
   caccatgaaa atgactaaga gtgct 25
<210> 43
   <211> 26
   <212> DNA
   <213> Synthetic
<400> 43
   tacatgtaca ttccaccatt tacatg 26
<210> 44
   <211> 25
   <212> DNA
   <213> Synthetic
<400> 44
   caccttgatt agaaaccgtg ttatg 25
<210> 45
   <211> 27
   <212> DNA
   <213> Synthetic
<400> 45
   ttagttattt tgtgttacat cctcatc 27
<210> 46
   <211> 921
   <212> DNA
   <213> Staphylococcus aureus
<400> 46
   ttgattagaa accgtgttat gaatagcgtt gtcaataaat atttgcttca caatcgctcg     60
   attatgttta aaaatgatca agatgttgaa agattttttt ataaaagaga aattgaaaat    120
   agaaaaaaac ataagcagcc ttcaacatta aatgttaaag caaatttaga aaaattatca    180
   ttagatgata tgcaagtctt tcgctttaat ttcagacatc aaattgataa gaaaatttta    240
   tatattcacg gtggattcaa tgcactacaa ccatcaccgt tccattggag attgttggat    300
   aaaatcactt taagtacatt atatgaggtt gtactgccta tctatccaaa gacaccagag    360
   tttcatattg acgatacttt ccaagcgata caacgtgttt atgatcaatt agtatctgaa    420
   gtaggacatc aaaatgtcgt agtcatgggt gatggttcag gtggtgcact ggcattatcc    480
   tttgtacaat ctcttttaga taatcaacag ccattaccga ataaattgta tttaatctca    540
   ccaattttag atgcaacact atctaataaa gatatttcgg acgccttaat cgaacaagat    600
   gcggttctaa gtcagtttgg tgtcaatgag attatgaaaa aatgggcgaa tggcctacca    660
   ttaacagata agcgcatatc gccaattaat ggcacaatag aaggattgcc accagtttat    720
   atgtttggtg gtggacgtga aatgacacat cctgatatga agctattcga acaaatgatg    780
   ttgcaacatc atcaatacat tgagttttat gattatccta agatggtaca tgattttcca    840
   atttatccaa ttagacaatc acataaagct attaaacaaa ttgccaaatc gatagatgag    900
   gatgtaacac aaaataacta a                                              921
<210> 47
   <211> 1305
   <212> DNA
   <213> Staphylococcus aureus
<400> 47
   atgccaatta ttacagatgt ttacgctcgc gaagtcttag actctcgtgg taacccaact      60
   gttgaagtag aagtattaac tgaaagtggc gcatttggtc gtgcattagt accatcaggt     120
   gcttcaactg gtgaacacga agctgttgaa ttacgtgatg gagacaaatc acgttattta     180
   ggtaaaggtg ttactaaagc agttgaaaac gttaatgaaa tcatcgcacc agaaattatt     240
   gaaggtgaat tttcagtatt agatcaagta tctattgata aaatgatgat cgcattagac     300
   ggtactccaa acaaaggtaa attaggtgca aatgctattt taggtgtatc tatcgcagta     360
   gcacgtgcag cagctgactt attaggtcaa ccactttaca aatatttagg tggatttaat     420
   ggtaagcagt taccagtacc aatgatgaac atcgttaatg gtggttctca ctcagatgct     480
   ccaattgcat tccaagaatt catgatttta cctgtaggtg ctacaacgtt caaagaatca     540
   ttacgttggg gtactgaaat tttccacaac ttaaaatcaa ttttaagcaa acgtggttta     600
   gaaactgcag taggtgacga aggtggtttc gctcctaaat ttgaaggtac tgaagatgct     660
   gttgaaacaa ttatccaagc aatcgaagca gctggttaca aaccaggtga agaagtattc     720
   ttaggatttg actgtgcatc atcagaattc tatgaaaatg gtgtatatga ctacagtaag     780
   ttcgaaggcg aacacggtgc aaaacgtaca gctgcagaac aagttgacta cttagaacaa     840
   ttagtagaca aatatcctat cattacaatt gaagacggta tggacgaaaa cgactgggat     900
   ggttggaaac aacttacaga acgtatcggt gaccgtgtac aattagtagg tgacgattta     960
   ttcgtaacaa acactgaaat tttagcaaaa ggtattgaaa acggaattgg taactcaatc    1020
   ttaattaaag ttaaccaaat cggtacatta actgaaacat ttgatgcaat cgaaatggct    1080
   caaaaagctg gttacacagc agtagtttct caccgttcag gtgaaacaga agatacaaca    1140
   attgctgata ttgctgttgc tacaaacgct ggtcaaatta aaactggttc attatcacgt    1200
   actgaccgta ttgctaaata caatcaatta ttacgtatcg aagatgaatt atttgaaact    1260
   gctaaatatg acggtatcaa atcattctat aacttagata aataa                    1305
<210> 48
   <211> 741
   <212> DNA
   <213> Staphylococcus aureus
<400> 48
   atgaaaatga ctaagagtgc tttagtaaca ggtgcatcaa gaggaattgg acgtagtatt 60
   gcgttacaat tagcagaaga aggatataat gtagcagtaa actatgcagg cagcaaagag 120
   aaagctgaag cagtagtcga agaaatcaaa gctaaaggtg ttgacagttt tgcgattcaa 180
   gcaaatgttg ccgatgctga tgaagttaaa gcaatgatta aagaagtagt tagccaattt 240
   ggttctttag atgttttagt aaataatgca ggtattactc gcgataattt attaatgcgt 300
   atgaaagaac aagagtggga tgatgttatt gacacaaact taaaaggtgt atttaactgt 360
   atccaaaaag caacaccaca aatgttaaga caacgtagtg gtgctatcat caatttatca 420
   agtgttgttg gagcagtagg taatccggga caagcaaact atgttgcaac aaaagcaggt 480
   gttattggtt taactaaatc tgcggcgcgt gaattagcat ctcgtggtat cactgtaaat 540
   gcagttgcac ctggttttat tgtttctgat atgacagatg ctttaagtga tgagcttaaa 600
   gaacaaatgt tgactcaaat tccgttagca cgttttggtc aagacacaga tattgctaat 660
   acagtagcgt tcttagcatc agacaaagca aaatatatta caggtcaaac aatccatgta 720
   aatggtggaa tgtacatgta a 741
<210> 49
   <211> 1209
   <212> DNA
   <213> Staphylococcus aureus
<400> 49
   atggctcaag atcgtaaaaa agtacttgta cttggtgctg gttatgcagg tttacaaact 60
   gtaactaaat tgcaaaaagc gatatcaaca gaagaagcag aaattacgct tattaataaa 120
   aatgaatatc actatgaagc aacatggtta catgaagcat cagcaggtac actaaactat 180
   gaagatgtat tatatcctgt ggaaagtgtc ttgaagaaag acaaagtgaa ctttgttcaa    240
   gcagaagtaa caaaaattga ccgtgatgct aaaaaggtag aaacaaatca aggtatttat    300
   gactttgata ttttagtagt agcattaggt ttcgttagtg aaacattcgg catcgaaggt    360
   atgaaagatc atgctttcca aattgaaaat gttatcacag cacgtgaatt atcacgtcat    420
   atcgaagaca aatttgctaa ctatgcagca tcaaaagaaa aagatgataa cgatttatct    480
   atcttagttg gtggtgctgg attcactggt gttgaattct taggtgaatt aacagacaga    540
   attcctgaat tatgtagcaa atatggtgtg gatcaaaata aagttaaaat cacttgtgtt    600
   gaagcagcac ctaaaatgtt accaatgttc tcagaagaat tagttaacca cgcagttagc    660
   tacttagaag accgcggtgt tgaatttaaa attgctacac caatcgttgc ttgtaacgaa    720
   aaaggttttg tagttgaagt agatggtgaa aaacaacaat taaatgcagg tacttcagta    780
   tgggcagctg gtgtacgtgg tagtaaatta atggaagaat catttgaagg cgttaaacgt    840
   ggacgtatcg ttacaaagca agatttaaca atcaatggtt acgacaacat ttttgttatt    900
   ggtgactgtt cagcgtttat cccagctgga gaagaacgtc cattaccaac tacagcacaa    960
   attgcaatgc aacaaggtga aagtgttgct aaaaacatta aacgcatctt aaacggtgaa   1020
   tcaactgaag aattcgaata tgttgatcgt ggaactgttt gttctttagg ttcacatgac   1080
   ggtgtaggta tggtatttgg taaacctatc gctggtaaaa aagcagcatt catgaaaaaa   1140
   gtgattgata cacgtgcggt attcaaaatc ggtggtatcg gtttagcatt caaaaaaggt   1200
   aaattctag                                                           1209
<210> 50
   <211> 194
   <222> DNA
   <213> Staphylococcus aureus
<400> 50
<210> 51
   <211> 969
   <212> DNA
   <213> Staphylococcus aureus
<400> 51
   atggaggatg ttttatacat gaaaaaatta acagcagcag cgattgcaac gatgggcttc     60
   gctacattta caatggcgca tcaagcagat gcagcagaaa cgacaaacac ccaacaagca    120
   catacacaaa tgtcaacaca atcacaagac gtatcttatg gtacttatta tacaattgat    180
   tctaatgggg attatcatca cacacctgat ggtaactgga atcaagcaat gtttgataat    240
   aaagaatata gctatacatt cgtagatgct caaggacata cgcattattt ttataactgt    300
   tatccaaaaa atgcaaatgc caatggaagc ggccaaacat atgtgaatcc agcaacagca    360
   ggagataaca atgactacac agcgagtcaa agccaacagc atattaatca atatggttat    420
   caatcaaatg taggtccaga cgcgagctat tattcacata gtaacaacaa ccaagcgtat    480
   aacagccatg atggtaatgg aaaggtcaat tatcctaatg gcacatctaa tcaaaatggt    540
   ggatcagcaa gtaaagcgac agctagtggt catgcgaaag acgcaagctg gttaacaagt    600
   cgtaaacaac tacaaccata tggacaatat cacggtggtg gtgcgcatta cggtgtcgac    660
   tatgcaatgc ctgaaaattc accagtttac tcattaactg atggtacagt agtacaagca    720
   ggttggagta actatggtgg cggcaatcaa gtaacgatta aagaagcgaa cagtaataac    780
   taccaatggt atatgcataa taatcgttta actgtttcag ctggtgataa agtcaaagct    840
   ggtgaccaaa ttgcatattc aggtagtacg ggtaattcaa cagcgcctca cgtacacttc    900
   caacgtatgt ctggtggcat cggtaatcaa tatgcagtag acccaacgtc atacttgcaa    960
   agtagataa                                                            969
<210> 52
   <211> 825
   <212> DNA
   <213> Staphylococcus aureus
<400> 52
   atgcaaccac atttaatatg tctagactta gacggaacat tattaaacga taacaaagaa 60
   atttcatcat atactaaaca agtattaaat gaattacaac aacgtggaca ccaaattatg 120
   attgcgactg gcagacctta tcgtgcaagt caaatgtatt atcatgaatt aaatttaacg 180
   acaccaattg ttaattttaa tggcgcttac gtacatcacc ctaaagataa aaacttcaaa 240
   acttgccatg aaattttaga tttaggcatc gcacaaaaca ttattcaagg attacaacaa 300
   tatcaagtat cgaatattat agcagaagtg aaagattatg ttttcattaa caatcatgat 360
   ccaagattat ttgaaggttt ttcaatgggt aatccaagaa ttcaaactgg taatttactt 420
   gtccacttga aagaatcccc tacctcaatt ttaattgaag ccgaagaaag taaaatacct 480
   gaaatcaaaa atatgcttac tcatttttat gccgatcata ttgagcatcg acgctggggc 540
   gcaccattcc ctgtcattga aattgtaaaa cttggtatta ataaagcaag aggcattgag 600
   caagttagac aatttttaaa tattgaccga aataatatta ttgcattcgg tgatgaagat 660
   aatgatattg aaatgattga gtacgcccgc catggtgttg ctatggaaaa tggtttgcaa 720
   gaacttaaag atgtagcgaa caatattaca ttcaacaata atgaagatgg cattggtcga 780
   tatttgaatg atttctttaa tttaaatatt agatattact gttaa 825
<210> 53
   <211> 702
   <212> DNA
   <213> Staphylococcus aureus
<400> 53
   atgaaaaaga caattatggc atcatcatta gcagtggcat taggtgtaac aggttacgca 60
   gcaggtacag gacatcaagc acacgctgct gaagtaaacg ttgatcaagc acacttagtt 120
   gacttagcgc ataatcacca agatcaatta aatgcagctc caatcaaaga tggtgcatat 180
   gacatccact ttgtaaaaga tggtttccaa tataacttta cttcaaatgg tactacatgg 240
   tcatggagct atgaagcagc taatggtcaa actgctggtt tctcaaacgt tgcaggtgca 300
   gactacacta cttcatacaa ccaaggttca gatgtacaat cagtaagcta caatgcacaa 360
   tcaagtaact caaacgttga agctgtttca gctccaactt accataacta cagcacttca 420
   actacttcaa gttcagtgag attaagcaat ggtaatactg caggtgctac tggttcatca 480
   gcagctcaaa tcatggctca acgtactggt gtttcagctt ctacatgggc tgcaatcatc 540
   gctcgtgaat caaatggtca agtaaatgct tacaacccat caggtgcttc aggtttattc 600
   caaactatgc caggttgggg tccgacaaac actgttgacc aacaaatcaa cgcagctgtt 660
   aaagcataca aagcacaagg tttaggtgct tggggattct aa 702
<210> 59
   <211> 1380
   <212> DNA
   <213> Staphylococcus aureus
<400> 54
<210> 55
   <211> 870
   <212> DNA
   <213> Staphylococcus aureus
<400> 55
   attagggact ccaaacccaa taaatactgt tgttacaagg tttctatgta tccaaactgg      60
   ggacaatata aacgcgctga tttaatcgga caatcttctt atattaaaaa taatgatgtc     120
   gtaatattca atgaagcatt tgataatggt gcatcagaca aattattaag taatgtgaaa     180
   aaagaatatc cttaccaaac acctgtactc ggtcgttctc aatcaggttg ggacaaaact     240
   gaaggtagct actcatcaac tgttgctgaa gatggtggcg tagcgattgt aagtaaatat     300
   cctattaaag aaaaaatcca gcatgttttc aaaagcggtt gtggattcga taatgatagc     360
   aacaaaggct ttgtttatac aaaaatagag aaaaatggta agaacgttca cgttatcggt     420
   acacatacac aatctgaaga ttcacgttgt ggtgctggac atgatcgaaa aattagagct     480
   gaacaaatga aagaaatcag tgactttgtt aaaaagaaaa atatccctaa agatgaaacg     540
   gtatatatag gtggcgacct taatgttaat aaaggcactc cagagttcaa agatatgctt     600
   aaaaacttga atgtaaatga tgttctatat gcaggtcata atagcacatg ggaccctcaa     660
   tcaaattcaa ttgcgaaata taattaccct aatggtaaac cagaacattt agactatata     720
   tttacagata aagatcataa acaaccaaaa caattagtca atgaagttgt gactgaaaaa     780
   cctaagccat gggatgtata tgcgttccca tattactacg tttacaatga tttttcagat     840
   cattacccaa tcaaagccta tagtaaatag                                      870
<210> 56
   <211> 804
   <212> DNA
   <213> Staphylococcus aureus
<400> 56
   atgaagaaaa tcgctacagc tactatcgca actgcaggat tcgctacaat cgcaattgca      60
   tcaggaaatc aagctcatgc ttctgagcaa gataactacg gttataatcc aaacgaccca   120
   acatcatata gctatactta cactattgat gcacaaggta actaccatta cacatggaaa   180
   ggtaactggc atccaagtca attaaaccaa gataatggct actacagcta ttactactac   240
   aatggctaca ataactacaa caattacaac aatggttata gctacaataa ttacagccgt   300
   tacaacaact actcaaataa taatcaatca tataactaca ataactataa tagttacaac   360
   acaaacagct accgtactgg tggtttaggt gcaagctaca gcacttcaag caacaatgtt   420
   caagtaacta caactatggc tccatcatca aatggccgtt caatctcaag tggttatact   480
   tcaggacgta acttatacac ttctggtcaa tgtacatact acgtatttga tcgtgtaggt   540
   ggtaaaatcg gttcaacttg gggcaatgca agtaactggg ctaacgcagc tgcaagagct   600
   ggttacacag tgaacaatac accaaaagct ggtgcaatta tgcaaacaac tcaaggtgca   660
   tacggtcacg ttgcatacgt tgaaagtgtt aacagcaatg gttcagtaag agtttcagaa   720
   atgaactatg gttatggccc aggtgttgta acttcacgta caatctcagc tagccaagct   780
   gctggttata acttcattca ctaa                                          804
<210> 57
   <211> 438
   <212> DNA
   <213> Staphylococcus aureus
<400> 57
   atgcgtcaaa catttatggc aaatgaatca aacattgagc gcaaatggta tgttatcgat    60
   gctgaaggcc aaacattagg tcgtttatca tcagaagtag catctatctt acgcggtaaa   120
   aataaagtaa cttacacacc acacgttgat actggtgatt atgtaatcgt tattaatgca   180
   tcaaaaatcg aatttactgg taacaaagaa actgacaaag tttactaccg tcactcaaat   240
   catccaggtg gtatcaaatc aatcactgct ggtgaattaa gaagaactaa cccagaacgt   300
   ttaattgaaa actcaattaa aggtatgtta ccaagcactc gtttaggcga aaaacaaggt   360
   aaaaaattat ttgtatatgg tggcgctgaa catccacacg ctgcacaaca accagaaaac   420
   tacgaattac gtggttaa                                                 438
<210> 58
   <211> 918
   <212> DNA
   <213> Staphylococcus aureus
<400> 58
   atggagactt atgaatttaa cattacagat aaagaacaaa caggtatgcg tgtagataag     60
   ttgctgcctg aattaaataa tgattggtct cgtaaccaga tacaagattg gattaaagca    120
   ggtttagtcg ttgcaaacga taaagttgtt aaatctaatt ataaagtgaa acttaatgat    180
   catatagttg tcactgaaaa agaagtggtt gaagctgata ttctacctga aaatttaaat    240
   ttagatattt attatgaaga tgacgatgtt gcagttgtat ataaaccgaa aggcatggta    300
   gttcatccat caccagggca ttataccaat acattagtta atggtttaat gtatcaaatt    360
   aaaaatttgt caggtattaa tggagaaatt cgtccaggta ttgttcaccg tatagatatg    420
   gatacttctg gtttattaat ggttgctaaa aatgatattg ctcatcgtgg gcttgtagaa    480
   caattaatgg ataaatctgt taaaagaaaa tatatcgctt tagttcacgg gaatattcct    540
   catgattacg gtacaatcga tgcgccaatt ggtagaaaca aaaatgatcg tcaatctatg    600
   gctgttgttg atgatggtaa ggaagcagtg acacatttta acgtactaga acattttaaa    660
   gattatacgc ttgttgaatg tcaacttgaa acaggacgta cgcatcaaat ccgtgtgcac    720
   atgaaatata ttggcttccc attagttggt gatccaaagt atggaccgaa aaagacattg    780
   gatattggtg gtcaagctct acatgctgga cttattggat tcgaacatcc agtaacaggt    840
   gaatatattg aaagacatgc tgaattacca caagactttg aagatttatt agatacaatt    900
   cgaaaaagag atgcataa                                                  918
<210> 59
   <211> 1185
   <212> DNA
   <213> Staphylococcus aureus
<400> 59
   atggcaaaag aaaaattcga tcgttctaaa gaacatgcca atatcggtac tatcggtcac 60
   gttgaccatg gtaaaacaac attaacagca gcaatcgcta ctgtattagc aaaaaatggt 120
   gactcagttg cacaatcata tgacatgatt gacaacgctc cagaagaaaa agaacgtggt 180
   atcacaatca atacttctca cattgagtac caaactgaca aacgtcacta cgctcacgtt 240
   gactgcccag gacacgctga ctacgttaaa aacatgatca ctggtgctgc tcaaatggac 300
   ggcggtatct tagtagtatc tgctgctgac ggtccaatgc cacaaactcg tgaacacatt 360
   cttttatcac gtaacgttgg tgtaccagca ttagtagtat tcttaaacaa agttgacatg 420
   gttgacgatg aagaattatt agaattagta gaaatggaag ttcgtgactt attaagcgaa 480
   tatgacttcc caggtgacga tgtacctgta atcgctggtt cagcattaaa agctttagaa 540
   ggcgatgctc aatacgaaga aaaaatctta gaattaatgg aagctgtaga tacttacatt 600
   ccaactccag aacgtgattc tgacaaacca ttcatgatgc cagttgagga cgtattctca 660
   atcactggtc gtggtactgt tgctacaggc cgtgttgaac gtggtcaaat caaagttggt 720
   gaagaagttg aaatcatcgg tttacatgac acatctaaaa caactgttac aggtgttgaa 780
   atgttccgta aattattaga ctacgctgaa gctggtgaca acattggtgc attattacgt 840
   ggtgttgctc gtgaagacgt acaacgtggt caagtattag ctgctcctgg ttcaattaca 900
   ccacatactg aatttaaagc agaagtatac gtattatcaa aagacgaagg tggacgtcac 960
   actccattct tctcaaacta tcgtccacaa ttctatttcc gtactactga cgtaactggt 1020
   gttgttcact taccagaagg tactgaaatg gtaatgcctg gtgataacgt tgaaatgaca 1080
   gtagaattaa tcgctccaat cgcgattgaa gacggtactc gtttctcaat ccgcgaaggt 1140
   ggacgtactg taggatcagg cgttgttact gaaatcatta aataa 1185
<210> 60
   <211> 2932
   <212> DNA
   <213> Staphylococcus aureus
<400> 60
<210> 61
   <211> 1695
   <212> DNA
   <213> Staphylococcus aureus
<400> 61
   atgactgatt atgttactaa atacgcaaaa aaggtagttt caggagaaat tttggcaagt    60
   ttgaagaata ttcaagtatg caaacgtcac ctatctttta tggagaaccc gccgaatggt   120
   tgccattggg ataatcattt gtctaacaaa gcaattaaat ttgtggaaat gcttccagac   180
   cctaaaacaa accagcccat gcctcttatg gagtttcaga aattcattgt tgggagctta   240
   tacggctggc gtagaggtca atacagaatg tttactaaag cttatataag tatggctaga   300
   aagcaaggta agtctctaat cgtatcggga atgtccgtta acgaactgtt gtttggacaa   360
   taccctaaat ttaatagaca aatttatgta gcttcatcta cttataagca agcgcaaaca   420
   atattcaaga tggcaagcca acaagtaaac ctaatgcgaa gtaaaagcaa gtttatccgt   480
   gaaaaaacag acgtaagaaa gacagacatt gaagatgtat taagtagttc agtgtttgca   540
   cctctttcca ataacccaga tgcggttgat ggtaaagatc ctacagttgc tattttggac   600
   gaattggcaa gtatgcctga tgatgagatg tactcaaggt ttaaaacagg tatgacatta   660
   caaaaaaatc ctttaaccct acttgtttca acggccggag acaatttaaa tagtcaaatg   720
   taccaagagt ataagtatat taaacgtatt ttaaatgaag aagtaagagc tgataattac   780
   tttgtatatt gtgctgaaat ggattcacaa gaagaagttc aagatgaaac aaagtggatt   840
   aaagcaatgc cgcttttaga atcaaaagaa catagaaaaa ctatacttca aaatgtaaaa   900
   gctgatatac aagacgaatt agaaaaaggg acatcgtatc ataagatttt gattaaaaac   960
   ttcaatttat ggcaagcgca aagagaagat agcttgctag atatttcaga ttgggaacaa  1020
   gtaataacgc ctatgcctaa tatcaatggt aaagatgtgt atataggtgt cgacttatcg  1080
   agattggatg acttaacatc tgtagggttt attttcccta acgacgataa aaaagtgttt  1140
   ttacatagtc attctttcat tggattaaga acaaacttag aacaaaaatc taagagagac  1200
   aaaataaatt atgaattagc gattgaacgt ggagaagctg agactacaca atcagatagc  1260
   ggcatgattg attataaaca agttatcgat tttatagtga aatttataac gacgcatgac  1320
   ctgaatgtac aggctgtttg ctatgaccct tggaatgcgc aaagttttat aacaacaatc  1380
   gaatcaatgg ctttagattg gccactcatt gaagtgggac aaagttttaa ggcgttatca  1440
   caatctatta aagaatttag aatgtgggtt gcagatgaaa gaatacagca taacgataat  1500
   atgttactta caacatcagt taataatgcc gttttgattc gtgacggaga agacaatgtg  1560
   aaaataaata aaaaaatgaa tcgtcaaaaa atagatccga ttatttcgat tatcacagct  1620
   ttcactgaag ctagaatgca cgaattccaa gaaaattgga cggagaaata tgaaagcgaa  1680
   gaattcggat tttaa                                                   1695
<210> 62
   <211> 1401
   <212> DNA
   <213> Staphylococcus aureus
<400> 62
   gtgactaaat cagtggctat tataggagcg gggataacag gtttatcaag tgcatatttt     60
   ttaaaacagc aagatcctaa tattgatgta accatctttg aagcatcgaa tcgtccgggg    120
   ggaaagattc aatcgtatcg taaagatggt tatatgattg aactagggcc tgaatcttat    180
   ttaggtagaa aaacgattat gacagaatta gcgaaagata ttggattaga acaagatatt    240
   gttacaaata cgactggaca atcatatatt tttgcgaaaa ataaattata tccgattcca    300
   ggtggttcaa ttatgggtat tccaacagat attaaaccat ttgttactac aaaattaata    360
   tcgccacttg gtaaattaag agcaggatta gatttaatca aaaagcctat acaaatgcaa    420
   gatggtgaca tttctgttgg tgcatttttc agagcaagat taggtaatga ggtacttgag    480
   aacttaattg agcctttaat gggtggtatt tatggtaccg atattgataa attaagtttg    540
   atgagtacgt ttcctaattt taaagaaaaa gaagaggcat tcggaagtct gataaaaggt    600
   atgaaggatg agaaaaataa gcgtctgaaa caaagacaat tatatcctgg cgcaccaaaa    660
   ggacaattca aacaatttaa gcatggttta agctcattta ttgaagcatt agaacaagat    720
   gtgaaaaata aaggtgtgac aatacgctac aatacgtcag tggatgatat tattacatct    780
   caaaagcaat ataaaattgt ttacagtaat caacaagaag atgtattcga tggggtatta    840
   gtgacaacac cgcatcaagt ctttttgaat tggttcggac aagatccagc atttgattac    900
   tttaaaacga tggatagtac gactgttgca actgttgtat tggcatttga tgaaaaagac    960
   attgaaaata cttatgatgg tactggcttc gtgattgcga gaacgagtga tacagacatt   1020
   accgcatgta cttggacatc gaaaaaatgg ccatttacta caccagaagg taaggttttg   1080
   attcgtgcgt atgtaggtaa accaggtgat actgtggttg atgatcatac agataatgaa   1140
   ttagtatcga ttgtacgtag agatttaagt caaatgatga catttaaagg tgatcctgaa   1200
   tttacaattg tcaatcgttt gccgaaaagt atgccacagt accatgtcgg tcatattcaa   1260
   caaattagac agattcaagc acatattaaa caaacatatc cacgacttag agtaactggt   1320
   gcatcttttg aagcggttgg actacctgat tgtattacac aaggtaaagt tgctgctgaa   1380
   gaagtaatcg cagagttgta a                                             1401
<210> 63
   <211> 1017
   <212> DNA
   <213> Staphylococcus aureus
<400> 63
   atgattaaac aattatacaa aaacatcaca atttgtagtt tagcaatatc tactgcatta      60
   actgtatttc cggcaacttc ttatgcaaaa attaattctg aaattaaagc tgtttctgag     120
   aagaatcttg atggtgatac taaaatgtat acacgtacag ctacaacaag tgatagtcaa     180
   aaaaatatta ctcaaagctt acaatttaat ttcttaactg aacctaatta tgataaagaa     240
   acagtattta ttaaagcaaa aggtacaatt ggtagtggtt tgagaatttt agacccaaat     300
   ggttattgga atagtacatt aagatggcct ggatcttatt cagtttcaat tcaaaatgtt     360
   gatgacaaca acaatacaaa tgtgactgac tttgcaccaa aaaatcagga tgaatcaaga     420
   gaagttaaat atacgtatgg ttataaaaca ggtggagatt tttcgattaa tcgtggaggc     480
   ttaactggaa atattacaaa agagagtaat tattcagaga cgattagtta tcaacaacca     540
   tcatatcgta cattacttga tcaatctacg tcacataaag gtgtaggttg gaaagtagaa     600
   gcacatttga taaataatat gggacatgac catacgagac aattaactaa tgatagtgat     660
   aatagaacta aaagtgaaat cttttcttta acacgaaatg gaaatttatg ggcgaaagat     720
   aatttcacac ctaaagacaa aatgcctgta actgtgtctg aagggtttaa tccagaattt     780
   ttagctgtta tgtcacatga taaaaaagac aaaggtaaat cacaatttgt tgttcattat     840
   aaaagatcaa tggatgagtt taaaatagat tggaatcgcc atggtttctg gggctattgg     900
   tctggtgaaa accatgtaga taaaaaagaa gaaaaattat cagcattata tgaagttgat     960
   tggaagacac atgatgtgaa gtttgtaaaa gtacttaatg ataatgaaaa gaaataa       1017
<210> 64
   <211> 1458
   <212> DNA
   <213> Staphylococcus aureus
<400> 64
   atgagcattc gctacgaatc ggttgagaat ttattaactt taattaaaga caaaaaaatc      60
   aaaccatctg atgttgttaa agatatatat gatgcaattg aagagactga tccaacaatt     120
   aagtcttttc tagcgctgga taaagaaaat gcaattaaaa aagcgcaaga attggatgaa     180
   ttacaagcaa aagaccaaat ggatggcaaa ttatttggta ttccaatggg tataaaagat     240
   aacattatta caaacggatt agaaacaaca tgtgcaagta aaatgttaga aggttttgtg     300
   ccaatttacg aatctactgt aatggaaaaa ctacataatg agaatgccgt tttaatcggt     360
   aaattaaata tggatgagtt tgcaatgggt ggttcaacag aaacatctat ttcaaaaaaa     420
   acagttaacc catttgacca taaagcagta ccaggtggtt catcaggtgg atctgcagca     480
   gcagttgcag ctggcttagt accatttagc ttaggttcag acactggtgg ttcaattaga     540
   caaccggctg catattgtgg tgttgtcggt atgaaaccaa catacggtcg tgtatctcga     600
   tttggattag ttgcatttgc atcttcatta gaccaaattg gtccattgac tcgaaatgta     660
   aaagataatg caatcgtatt agaagctatt tctggtgcag atgctaatga ctctacaagt     720
   gcacctgttg atgatgtaga ctttacatct gaaattggta aagatattaa aggattaaaa     780
   gttgcattac ctaaagaata cttaggtgaa ggtgtagctg atgacgtaaa agaagcagtt     840
   caaaacgctg tagaaacttt aaaatcttta ggtgctgtcg ttgaggaagt atcattgcca     900
   aatactaaat ttggtattcc atcatattac gtgattgcat catcagaagc ttcgtcaaac     960
   ctttctcgtt ttgacggaat tcgttatggt tatcattcta aagaagctca ttcattagaa    1020
   gaattatata aaatgtcaag atctgaaggt ttcggtaaag aagtaaaacg tcgtattttc    1080
   ttaggtacat ttgcattaag ttcaggttac tacgatgctt actataaaaa atctcaaaaa    1140
   gttagaacat tgattaaaaa tgactttgat aaagtattcg aaaattatga tgtagtagtt    1200
   ggtccaacag cgcctacaac tgcgtttaat ttaggtgaag aaattgatga tccattaaca    1260
   atgtatgcca atgatttatt aacaacacca gtaaacttag ctggattacc tggtatttct    1320
   gttccttgtg gacaatcaaa tggccgacca atcggtttac agttcattgg taaaccattc    1380
   gatgaaaaaa cgttatatcg tgtcgcttat caatatgaaa cacaatacaa tttacatgac    1440
   gtttatgaaa aattataa                                                  1458
<210> 65
   <211> 1242
   <212> DNA
   <213> Staphylococcus aureus
<400> 65
   gtggccgaac actcatttga cgttaatgaa gtaatcaagg attttccgat attagatcaa      60
   aaagtcaatg gcaaacgttt agcatatctt gattcaacag cgacaagtca aacgcctatg     120
   caagtgttaa atgttttaga ggattactac aagcgttata attcaaacgt tcatcgtggt     180
   gttcatacat taggatcatt ggcaactgat ggttatgaaa atgcccgtga aaccgttcgt     240
   cgttttatta atgcgaagta ttttgaagaa atcattttca cacgcggaac aactgcgtcg     300
   attaaccttg tagcacatag ctatggtgat gcaaatgttg aagagggcga tgaaattgtt     360
   gtcactgaaa tggagcatca tgccaatatt gttccttggc aacagttagc aaagcgtaaa     420
   aatgcgacat tgaaatttat accaatgaca gctgacggtg aattaaacat cgaagatatt     480
   aagcaaacga ttaatgataa aacaaagatc gttgctattg cacatatttc taatgtactc     540
   ggtacaatta atgatgttaa aaccattgca gaaatagctc atcaacatgg cgcaattatc     600
   agtgttgatg gggcgcaagc agcaccacat atgaaacttg atatgcaaga aatgaatgct     660
   gatttttata gttttagtgg tcataaaatg cttggaccaa caggtattgg cgtattattt     720
   ggtaaacgtg agttactaca aaaaatggaa ccgattgagt tcggtggtga catgattgat     780
   tttgtaagta agtatgatgc aacatgggct gatttaccta ctaaatttga ggcgggtact     840
   ccattaattg ctcaagcaat tgggcttgca gaagctattc gctatttaga acgcataggt     900
   tttgatgcaa ttcataaata tgaacaagaa ttaacgatat atgcttatga gcaaatgtct     960
   gcaattgaag gaattgaaat ttatggcccg cctaaggatc gtcgtgcagg tgtaataacg    1020
   tttaatttac aagatgtaca tccacacgat gttgctacag ccgtagatac agaaggtgta    1080
   gcggttagag ctgggcatca ttgtgcgcaa ccgttaatga aatggttaaa tgtgtcttca    1140
   acagctagag cgagttttta tatatacaac acgaaagaag acattgatca gttaataaat    1200
   gccttgaaac aaacgaagga gtttttctct tatgaatttt aa                       1242
<210> 66
   <211> 1185
   <212> DNA
   <213> Staphylococcus aureus
<400> 66
   atggcaaaag aaaaattcga tcgttctaaa gaacatgcca atatcggtac tatcggtcac      60
   gttgaccatg gtaaaacaac attaacagca gcaatcgcta ctgtattagc aaaaaatggt     120
   gactcagttg cacaatcata tgacatgatt gacaacgctc cagaagaaaa agaacgtggt     180
   atcacaatca atacttctca cattgagtac caaactgaca aacgtcacta cgctcacgtt     240
   gactgcccag gacacgctga ctacgttaaa aacatgatca ctggtgctgc tcaaatggac     300
   ggcggtatct tagtagtatc tgctgctgac ggtccaatgc cacaaactcg tgaacacatt     360
   cttttatcac gtaacgttgg tgtaccagca ttagtagtat tcttaaacaa agttgacatg     420
   gttgacgatg aagaattatt agaattagta gaaatggaag ttcgtgactt attaagcgaa     480
   tatgacttcc caggtgacga tgtacctgta atcgctggtt cagcattaaa agctttagaa     540
   ggcgatgctc aatacgaaga aaaaatctta gaattaatgg aagctgtaga tacttacatt     600
   ccaactccag aacgtgattc tgacaaacca ttcatgatgc cagttgagga cgtattctca     660
   atcactggtc gtggtactgt tgctacaggc cgtgttgaac gtggtcaaat caaagttggt     720
   gaagaagttg aaatcatcgg tttacatgac acatctaaaa caactgttac aggtgttgaa     780
   atgttccgta aattattaga ctacgctgaa gctggtgaca acattggtgc attattacgt     840
   ggtgttgctc gtgaagacgt acaacgtggt caagtattag ctgctcctgg ttcaattaca     900
   ccacatactg aatttaaagc agaagtatac gtattatcaa aagacgaagg tggacgtcac     960
   actccattct tctcaaacta tcgtccacaa ttctatttcc gtactactga cgtaactggt    1020
   gttgttcact taccagaagg tactgaaatg gtaatgcctg gtgataacgt tgaaatgaca    1080
   gtagaattaa tcgctccaat cgcgattgaa gacggtactc gtttctcaat ccgcgaaggt    1140
   ggacgtactg taggatcagg cgttgttact gaaatcatta aataa                    1185
<210> 67
   <211> 780
   <212> DNA
   <213> Staphylococcus aureus
<400> 67
   atgcaagcat tacaaacatt taattttgaa gaattaccag taagaacatt agaggttgac      60
   ggagaaccat attttatagg aaaagatgtt gctgacattt taggatatgc aaacggacga     120
   gatgctttgt caaaacatgt tgatgaagac gacaagaaag ttctaacgtc gcgaaatacg     180
   actttagaaa atttaccaaa tcgaggactt actgcagtca acgaatcggg tttatacagc     240
   ctaatcttct catcaaaact agaatcagct aaacgattca aacgctgggt aacatcagat 300
   gtcctaccag ccattcgcaa atatggtatc tacgcaacgg acaacgtaat tgaacaaaca 360
   ttaaaagatc cagactacat cattacagtg ttgactgagt ataagaaaga aaaagagcaa 420
   aacttacttt tgcaacaaga aatcggagag ctaaaaccca aagcagatta tgttgatgaa 480
   atcttaaaat caactggcac attagctaca actcaaatcg cggcagacta cggtatatca 540
   gcacaaaagt taaacaaact actacacgaa gctagactac aacgaaaagt aaataaacag 600
   tgggtgcttt attcagaaca catgggcaag agttacacag attcagacac tataacaatt 660
   gtgcgttctg atggcagaga agacacagtt ttacaaacta gatggacaca aaaaggcaga 720
   ttgaaaatac atgaaatcat gactgaattc ggttatgaag ctaatttagg gggagcgtaa 780
<210> 09
   <211> 702
   <212> DNA
   <213> Staphylococcus aureus
<400> 69
<210> 69
   <211> 1413
   <212> DNA
   <213> Staphylococcus aureus
<400> 69
   ttatacttca acacccatat ctttagcttt tgcaataaca tcatccatgc taccaactaa      60
   acggaatgca tcttctggaa tatggtcata tttaccatct aagatatctt taaagtttgc     120
   aactgttgtc ttaacaggta cataagaacc tttttgacca gtaaattgtt ccgctacgtg     180
   gaagttttga gataagaaga attgaattct acgtgcgcgt tcaactgttt gtttatcttc     240
   atcagataat tcgtccatac ctaagatagc aatgatatct tgcaattcac ggtatttttg     300
   aagtgttgat tgtacatcac gagctacttc ataatgttct tgacctacaa ttgatggttc     360
   caatgctctt gatgtagacg ctaatggatc cacggctgga taaataccca tttcagttaa     420
   tttacgttct aagttagtag ttgcatctaa atgggcaaac gctgtcgcag gcgctgggtc     480
   agtatagtca tcggcaggta cgaataccgc ttgaatagaa gtaactgatc cttttgttgt     540
   agacgtaata cgttcttgta attgtcccat ttcagtagca agtgttggtt ggtaacctac     600
   tgcagaaggc atacgaccta ataatgcaga tacctcagaa ccagcttgtg taaatctgaa     660
   aatgttatcg atgaataata atacgtcttg accttgttcg tcacggaaat attcagccat     720
   tgttaaacca gataatgcaa cacgcatacg tgcaccaggt ggctcattca tttgcccgaa     780
   taccatggct gttttcttaa ttacaccact gtcactcatt tcgaagtata aatcgttacc     840
   ttcacgagta cgttcaccta caccggcgaa tacagaaata ccaccgtgct cttgagcgat     900
   gttgttaatt aattcttgga ttaatactgt tttacctaca ccggcaccac cgaacaatcc     960
   gattttacca cctttaatat aaggtgctag taaatctact actttaatac ctgtttctaa    1020
   aatttgaact tctgttgaaa gttcatcgaa tgctggtgct tgacgatgga taggatcgcg    1080
   gcgaacagaa tcactaattt cttctttaag gtcaattgtt tcacctagta cattaaatac    1140
   acgacctaat gtttcgtcac caacaggtac actaatttct ttgcctgtat cttttacatc    1200
   catgcctctt tggacaccat cagttgaatc catcgcaatt gtacgaacaa cgtcgtcacc    1260
   taattgcagc gcaacttcta atgttagttg tattgtacct tcttctttag gcacatcaat    1320
   aaccaaggcg ttattaattt taggaacttc gttatgttca aatcgaacat caattacagg    1380
   acccataact tgagttacac ggccaattcc cat                                 1413
<210> 70
   <211> 1119
   <212> DNA
   <213> Staphylococcus aureus
<400> 70
   ttataattgt aatgcttctt ctacagattt atattccatt tcaaatgcct ctgcaacgcc      60
   tttattggtt acgtgacctt tgtaagtatt taaacctaat gataatggtt gatttgattt     120
   aaatgcttct ctataccctt tattagctag catgagcgca taaggtagcg tagcattatt     180
   taaagctaac gtcgaagtac gcggtactgc acctggcata tttgcaactg cataatgaac     240
   cacaccatgc ttaatatatg taggatcatc atgtgtcgta attttatcag ttgtttcaaa     300
   aataccgcct tgatcaatag caatgtcaat aataactgac ccatttttca tttgtttaat     360
   catgtcttct gttacaagtc ttggcgcttt agcacctgga attaaaactg cacctattac     420
   taaatcactt tgtttaacat acaactcaat attcaacgga tttgacataa ttgtatgtac     480
   acgtccaccg aataaatcat ctaattgttg taaacgcttt ggattaacat ctaaaatcgt     540
   aacatctgca cctagtccta gtgcaatttt agctgcattt gttcctgctt gaccaccacc     600
   gataatagtt actttaccct taggtactcc tgggacacca cctagtagaa ttcccatacc     660
   accattaagt ttttgtagga actctgcgcc aacttgagct gacattcttc ctgctacctc     720
   actcattggt gataacaatg gtaaagatcg gtctggtaac tgcacagtct catatgcaat     780
   actaattact tttctatcta tcaaagcttg tgttaatttt tcttcatttg ctaaatgaag     840
   ataagtgaat aatacaagcc cttctttaaa atatggatat tcagattcaa gtggttcttt     900
   aactttaata accatatcca catcccaaac ttttgcttgt tcagcaacaa tctcagcacc     960
   tgcttctttg taatctacat cttcaaagaa tgatcctgaa cccgcatttg tttccactaa    1020
   aacagtatgc ccactttcta ctaaagcgtg cacaccactt ggtgataaac caacacgatt    1080
   ttcattattt ttaatctccc ttggtatacc aattttcat                           1119
<210> 71
   <211> 1179
   <212> DNA
   <213> Staphylococcus aureus
<400> 71
   atgacaagac catttaatcg tgtacattta atcgtaatgg attcagtagg tattggtgaa     60
   gcgccagacg cagctgattt taaagatgaa ggttcacata ctttaagaca taccttagaa    120
   ggtttcgatc aaactttacc aaaccttgaa aagttaggtc tagggaacat cgataaatta    180
   ccagtagtaa atgcagttga acaaccagaa gcatactata ctaaattgag tgaagcttca    240
   gttggtaaag atacaatgac tggtcactgg gaaattatgg gattaaatat tatgcaacct    300
   tttaaagtat accctaatgg attccctgaa gagttaattc aacaaattga agaaatgaca    360
   ggtcgtaaag ttgttgctaa caaaccggca tcgggtacgc aaattatcga tgagtggggc    420
   gagcaccaaa tgaaaactgg tgacttaatt gtttatacaa gtgcagaccc agtattgcaa    480
   attgctgcac atgaagacat tatcccatta gaagagttat atgatatttg tgaaaaggtt    540
   cgtgagttga caaaagaccc taaatattta attggtcgta ttatcgcacg tccatatgtt    600
   ggtgaaccag gaaactttac acgtacatct aatcgacatg actatgcgtt aaaaccattt    660
   ggtaaaactg tcttagatca tttgaaagac ggtggttatg atgttattgc catcggtaaa    720
   attaatgaca tttatgatgg tgaaggtgta acagaagcgg ttcgtacgaa gagtaacatg    780
   gacggtatgg atcaattgat gaaaattgtt aagaaagatt tcacaggtat tagcttctta    840
   aacttagtag actttgatgc attatacggt catcgtcgtg ataaaccagg ttatgcacaa    900
   gcaattaaag atttcgatga tcgcttgcca gaactgttta gcaacttaaa agaagacgat    960
   ttagtaatta ttacagcaga ccatggtaat gacccgacag cgccaggtac ggaccatacg   1020
   agagaatata tcccagtaat tatgtacagt ccgaaattta aaggtggtca tgcactagaa   1080
   agtgatacta cattcagttc tatcggtgca actatagcag ataatttcaa cgtaacatta   1140
   ccagagttcg gtaaaagtta tttaaaggaa ttgaaatag                          1179
<210> 72
   <211> 1245
   <212> DNA
   <213> Staphylococcus aureus
<400> 72
   atgactgaga acaataattt agtaacttct actcaaggaa ttattaaaga agcattgcat      60
   aaattgggat ttgacgaagg aatgtacgat ttaattaaag aacctttaag aatgttacaa     120
   gtgcgtatcc ctgtacgaat ggatgatggc acagttaaaa cattcacagg ttaccgtgcg     180
   caacataatg atgctgttgg accaacaaaa gggggcgtgc gtttccaccc agatgttgat     240
   gaagaagaag taaaagcatt atcaatgtgg atgactttga aatgtggcat tgtaaactta     300
   ccatacggtg gtggtaaggg tggtatcgtt tgtgatccac gtcaaatgag cattcatgaa     360
   gttgaacgtt tatcacgcgg atatgtaaga gcaatttcac aattcgtagg tccgaacaaa     420
   gatattccag caccagatgt atttacaaac tcacaaatta tggcttggat gatggatgaa     480
   tatagtgcat tagataaatt taattcacca ggtttcatca caggtaaacc aattgtattg     540
   ggtggttctc atggacgcga cagatcaact gcactaggtg tagttattgc aattgaacaa     600
   gctgcaaaac gtcgtaatat gcaaattgaa ggtgccaagg ttgttattca aggtttcggt     660
   aatgccggaa gtttcttagc taaattctta tatgatttag gtgcaaaaat tgtaggtatc     720
   tctgatgctt acggtgcatt acacgatcca aatggcttag atatagatta tttattagac     780
   cgtcgtgata gttttggtac ggtaacaaat ttatttgaag aaacaatctc aaataaagaa     840
   ttgtttgaat tagattgtga cattttagta ccagcggcta tttcaaacca aattacagaa     900
   gacaatgcac atgatattaa agctagtatc gttgttgaag ctgctaatgg acctacaaca     960
   ccagaagcaa cacgtatttt aactgaacgt ggtatattat tagttccaga cgtattagca    1020
   agtgctggtg gtgtaacggt ttcttacttc gaatgggtac aaaataatca aggttattat    1080
   tggtctgaag aagaagtaaa tgaaaagcta cgtgaaaaat tagaagcggc atttgacacg    1140
   atttacgaat tgtctcaaaa tcgaaaaata gatatgagac ttgcagcata tatcataggt    1200
   attaaacgta cagcagaagc agctagatat cgtggttggg cataa                    1245
<210> 73
   <211> 1859
   <212> DNA
   <213> Staphylococcus aureus
<400> 73
   atgcctaaaa ataaaatttt aatttatttg ctatcaacta cgctcgtatt acctacttta      60
   gtttcaccta ccgcttatgc tgatacacct caaaaagata ctacagctaa gacaacatct     120
   catgattcaa aaaaatctaa tgacgatgaa acttctaagg atactacaag taaagatact     180
   gataaagcag acaacaataa tacaagtaac caagacaata acgacaaaaa atttaaaact     240
   atagacgaca gcacttcaga ctctaacaat atcattgatt ttatttataa gaatttacca     300
   caaaccaata taaaccaatt gctaaccaaa aataaatacg atgataatta ctcattaaca     360
   actttaatcc aaaacttatt caatttaaat tcggatattt ctgattacga acaacctcgt     420
   aatggcgaaa agtcaacaaa tgattcgaat aaaaacagtg acaatagcat caaaaatgac     480
   actgatacgc aatcatctaa acaagataaa gcagacaatc aaaaagcacc taaatcaaac     540
   aatacaaaac caagtacatc taataagcaa ccaaattcgc caaagccaac acaacctaat     600
   caatcaaata gtcaaccagc aagtgacgat aaagcaaatc aaaaatcttc atcgaaagat     660
   aatcaatcaa tgtcagattc ggctttagac tctattttgg atcaatacag tgaagatgca     720
   aagaaaacac aaaaagatta tgcatctcaa tctaaaaaag acaaaaatga aaaatctaat     780
   acaaagaatc cacagttacc aacacaagat gaattgaaac ataaatctaa acctgctcaa     840
   tcattcaata acgatgttaa tcaaaaggat acacgtgcaa catcattatt cgaaacagat     900
   cctagtatat ctaacaatga tgatagcgga caatttaacg ttgttgactc aaaagataca     960
   cgtcaatttg tcaaatcaat tgctaaagat gcacatcgca ttggtcaaga taacgatatt    1020
   tatgcgtctg tcatgattgc ccaagcaatc ttagaatctg actcaggtcg tagtgcttta    1080
   gctaagtcac caaaccataa tttattcggt atcaaaggtg cttttgaagg gaattctgtt    1140
   ccttttaaca cattagaagc tgatggtaat aaattgtata gtattaatgc tggattccga    1200
   aaatatccaa gcacgaaaga atcactaaaa gattactctg accttattaa aaatggtatt    1260
   gatggcaatc gaacaattta taaaccaaca tggaaatcgg aagccgattc ttataaagat    1320
   gcaacatcac acttatctaa aacatatgct acagatccaa actatgctaa gaaattaaac    1380
   agtattatta aacactatca attaactcag tttgacgatg aacgcatgcc agatttagat    1440
   aaatatgaac gttctatcaa ggattatgat gattcatcag atgaattcaa acctttccgc    1500
   gaggtatctg atagtatgcc atatccacat ggccaatgta cttggtacgt atataaccgt    1560
   atgaaacaat ttggtacatc tatctcaggt gatttaggtg atgcacataa ttggaataat    1620
   cgagctcaat accgtgatta tcaagtaagt catacaccaa aacgtcatgc tgctgttgta    1680
   tttgaggctg gacaatttgg tgcagatcaa cattacggtc atgtagcatt tgttgaaaaa    1740
   gttaacagtg atggttctat cgttatttca gaatccaatg ttaaaggatt aggtatcatt    1800
   tctcatagaa ctatcaatgc agctgccgct gaagaattat catatattac aggtaaata     1859
<210> 74
   <211> 1092
   <212> DNA
   <213> Staphylococcus aureus
<400> 74
   ttattttaac tttttagcgt tatataaatc agctaaaggc tttaattcat cataaaatgc      60
   ttggaattca tctaaatcca tttgttgacc cgcatcacta agtgcaacag atggatctgg     120
   atgcacctca gccataactc catcagcacc aactgctaat gctgctttcg cagttggtaa     180
   catgatatct ttacgacctg tactatgcgt aacatctacc atgactggta agtgtgtacc     240
   ttgttttaaa attggtactg ctgaaatatc taaagtgtta cgtgtcgcct tttcataagt     300
   tcggattcca cgttcacata aaataatgtt ttgattacct tgtgaagcaa tgtattcagc     360
   tgcataaaca aactcttcga ttgtagcaga taaaccacgt tttaatagaa taggcttttt     420
   cgtacggcca gcttctttta ataactcgaa gttttgcata ttacgtgcac caatttggaa     480
   tacgtctaaa tactcatcag ccacttcaaa atcatttgga tttacgattt cgctgacaac     540
   atttaaatca tatttatctt taatctgttt aagtatttta agtccttcaa cacctaggcc     600
   ttggaaatca tatggtgatg tacgtggttt aaatgcaccg ccacgaataa atttttcacc     660
   tttagcatgt aagtttttag caacagcttc aacttgttca aatgattcaa ctgaacatgg     720
   cccaaataca aatgatttat tgccgtctcc aataatgccc ccattatcaa atgttacaat     780
   cgtatcttca ggtttcaact tacgtgatac gtataagtgt ttttcatttt cagatttttg     840
   taaatctgta gaggctttaa aaatttcttt aaataattgc ttaattgtat tatcgttaaa     900
   tggtcctttg ttgctatcaa ttaagtcgtt aagcatttct ttttcgcgtt gtggatcata     960
   gatacgtgta ccttgtttta atttttcctc cccaattttt tgtgctagtt caccacgttt    1020
   agataataaa tctaaaattt gatgattcag tgagacaatc tcacttctgt atgattctaa    1080
   tttattactc at                                                        1092
<210> 75
   <211> 696
   <212> DNA
   <213> Staphylococcus aureus
<400> 75
   ttatgcagta acccaatgtc cagcgccacc agtgttatat aattttactg ctgcggcatc     60
   ttgaacactt tcaggagcat ttgctggtga tacaccttta tatttagcag gtgctactga    120
   atcccaagtt gattgtaaga attgatactt accagctgca cctgatgttg gatttacagc    180
   atgaatattg ccacctgatt cacgttgagc aatttgtttt agatgagcat tcacatttac    240
   tgatgaacct tctgatgctt ttgattcagt tggtgttgca gtaacttgtg aattgtttga    300
   tgttgatgct tgtggttgtt gagtttgagc attttgtggt gcttcaactt cttgtgattg    360
   tacttgatta gcttgaacag ctgatggttc aacattatta gttgcaggtg cttgtgcact    420
   catgtttgct ccattagcac ctgttgcatg gtaattccaa gcaaagtgtg taccatctga    480
   ttcaaagtga taagtaaacc cttcatagtc aaatgtataa ttataagccc cagcttcaat    540
   tggtttttga tttaatgttt gatcatttga ttgcgccatt tgcgctaaag atgctttatt    600
   taagtccgct tcacttgcat gggcttcgtg acctgcattt cctgctacga ttcctaaacc    660
   tactgctaat gatgatgcga gtaatgtttt cttcat                              696
<210> 76
   <211> 948
   <212> DNA
   <213> Staphylococcus aureus
<400> 76
   atggacatag aattaacaaa aaaagatggt actgtaatca aattaagtga atacgggttt 60
   atcgttaacg atatagtaat tgatagtatg caaatcaaca caaagtatca agataaagaa 120
   aatatgaacg gtcgtatatt aatggggagc aattatatca gtagagatat agttgttcct 180
   tgtttttgtg tggtaaaaaa tcgttcagac attgcttata tgcgagatat gttgtattcg 240
   ttaacgacag acatagaacc tatgtatttg cgagaaataa gaagaaaaga agagttgaat 300
   tacaggttta ctcaaccaat ttctgatgat tacgtgaaat tagataaaaa caacttcccg 360
   gattatgaat attcaagaca cgatcaacaa aattatgtaa atggtaaaca gtataaagtt 420
   atttttaacg gagttataaa ccctaaacaa aaaggtaata aagtttcttt tgaactaaaa 480
   ttcgaaacta cagaattacc atacggtgaa agtattggaa caagcctaga gttagaagaa 540
   aacaaaaagg ttggattgtg gtcgtttgat tttaatattg attggcatgc aggcggagac 600
   aaaagaaagt atacatttga aaatttgagc aaaggtacag tttactatca tggtagtgct 660
   cctaacgacc aattcaacat gtataaaaag ataacaatta ttttaggcga agatacagaa 720
   tcgtttgtat ggaatttaac gcatgctgaa ataatgaaaa tcgaagggat caaactaaaa 780
   actggagaca gaattgttta tgatagcttc cgagtttata aaaacggtgt tgaaataagt 840
   accgaaacga atatagccca accaaaattt aaatacggag ctaataaatt tgagtttaat 900
   caaacggtac aaaaagttca gtttgatttg aaattttatt ataagtag 948
<210> 77
   <211> 723
   <212> DNA
   <213> Staphylococcus aureus
<400> 77
   atggctcaaa tttctaaata taaacgtgta gttttgaaac taagtggtga agcgttagct 60
   ggagaaaaag gatttggcat aaatccagta attattaaaa gtgttgctga gcaagtggct 120
   gaagttgcta aaatggactg tgaaatcgca gtaatcgttg gtggcggaaa catttggaga 180
   ggtaaaacag gtagtgactt aggtatggac cgtggaactg ctgattacat gggtatgctt     240
   gcaactgtaa tgaatgcctt agcattacaa gatagtttag aacaattgga ttgtgataca     300
   cgagtattaa catctattga aatgaagcaa gtggctgaac cttatattcg tcgtcgtgca     360
   attagacact tagaaaagaa acgcgtagtt atttttgctg caggtattgg aaacccatac     420
   ttctctacag atactacagc ggcattacgt gctgcagaag ttgaagcaga tgttatttta     480
   atgggcaaaa ataatgtaga tggtgtatat tctgcagatc ctaaagtaaa caaagatgcg     540
   gtaaaatatg aaaatttaac gcatattcaa atgcttcaag aaggtttaca agtaatggat     600
   tcaacagcat cttaattctg tatggataat aacattccgt taactgtttt ctctattatg     660
   gaagaaggga atattaaacg tgctgttatg ggtgaaaaga taggtacgtt aattacaaaa     720
   taa                                                                   723
<210> 79
   <211> 774
   <212> DNA
   <213> Staphylococcus aureus
<400> 79
   atgagcttat tatctaaaac gagagagtta aacacgttac ttcaaaaaca caaaggtatt      60
   gcggttgatt ttaaagatgt agcacaaacg attagtagcg taactgtaac aaatgtattt     120
   attgtatcgt gtcgaggtaa aattttaggg tcgagtctaa atgaattatt aaaaagtcaa     180
   agaattattc aaatgttgga agaaagacat attccaagtg aatatacaga acgattaatg     240
   gaagttaaac aaacagaatc aaatattgat atcgacaatg tattaacagt attcccacct     300
   gaaaacagag aattattcat agatagtcgt acaactatct tcccaatttt aggtggagga     360
   gaaagattag gtacattagt acttggtcga gtacatgatg attttaatga aaatgatttg     420
   gtactaggtg aatatgctgc tacagttatt ggtatggaaa tcttacgtga gaagcatagt     480
   gaagtagaaa aagaagcgcg cgataaagct gctattacaa tggcaattaa ttcattatct     540
   tattctgaaa aagaagcgat tgaacatatc tttgaagaac ttggcggtac ggaaggccta     600
   ttaatcgcat caaaagttgc agatagagtt ggtattacta gatctgtaat tgtaaatgca     660
   ctacgtaaat tagaaagtgc tggtgtaatt gaatcacgtt ctttaggaat gaaaggtact     720
   ttcattaaag ttaaaaaaga aaaattctta gatgaattag aaaaaagtaa ataa           774
<210> 79
   <211> 720
   <212> DNA
   <213> Staphylococcus aureus
<400> 79
   tcaactttcg tccttataag atatttctac atctaaatgg aatttttcag tttcgacagt      60
   tttgttgtca ttatatattt ttaagaaact ttcagcttgt cctgttccag tatcaaataa     120
   gtcataagaa aatgatgaac catcattcaa atgaaataat atttttcctg cattaaatcc     180
   agaactaaat tttgacttat taccataatt tcttccttta tttgtatcat taaagccata     240
   aatattatat tcttcttgta gatatcttct taatttagta tcaatttctt gagctgttac     300
   taacttttta ttagttgata ctttgtcagt agatatagtt tgatgttctc cattaaccca     360
   aagattaata ggaatacgtc tagatttctc taaataatca cctgctaatg taactccacc     420
   gtatatatat ttgtttttca aacaatatcc actataactt agaccatata catctacagc     480
   atgatttttg aatttcttta catcatcaac gttcttaaat tcagctgtta ctgtcgaatt     540
   atccatagaa aaaactaatt gatgcgaaag atgattattt tcaggattaa tactttggtg     600
   gtcttcaatt ggatagctac cataatagtt cctaagattc aaaactccga catcagcggt     660
   tgcgatatga ttttgcgaat agcaaaacaa taaaacaaca atgataagta ttcttttcat     720
<210> 80
   <211> 798
   <212> DNA
   <213> Staphylococcus aureus
<400> 80
   atgaatcgtt tgcatggaca acaagttaaa attggttacg gggataacac gattataaat      60
   aaattagatg ttgaaatacc agatggcaaa gtgacgtcaa tcattggtcc taacggctgc     120
   gggaaatcta ctttgctaaa ggcattgtca cgtttattgg cagttaaaga aggcgaagta     180
   tttttagatg gtgaaaatat tcatacacaa tctacgaaag agattgcaaa aaaaatagcc     240
   attttacctc aatcacctga agtagcagat ggcttaactg ttggggaatt agtttcatat     300
   ggtcgttttc cacatcaaaa aggatttggt agattaactg ctgaggataa gaaagaaatt     360
   gattgggcaa tggaagttac aggaactgat acattccgac accgttcaat caatgattta     420
   agtggtggtc aaagacaacg tgtttggatt gcaatggcat tagcacaaag aactgatatt     480
   atctttttag acgaaccaac aacatattta gatatctgtc atcaattaga aatactagaa     540
   ttagttcaga agctaaatca ggaacaaggt tgtacaattg tcatggttct tcatgatatc     600
   aaccaagcga ttcgtttctc agatcatctt attgcgatga aagaagggga tatcatcgct     660
   acaggttcaa cagaagacgt attaacacag gaaatattag aaaaagtttt taatattgat     720
   gttgttttaa gtaaagatcc taaaactgga aaacctttac tggtaactta tgacttatgt     780
   cgcagagctt attcttaa                                                   798
<210> 81
   <211> 957
   <212> DNA
   <213> Staphylococcus aureus
<400> 81
   atgaactatc aagttctttt atattataaa tatacgacga ttgatgaccc tgaacagttt      60
   gctcaggatc acttagcgtt ttgcaaagca caccatttaa aaggtagaat tcttgtttct     120
   acagaaggta ttaacggcac attatctggt acaaaagaag aaaccgaaca atatatggca     180
   catatgcatg ccgatgaacg attcaaagat atggtgttta aaattgatga agctgaagga     240
   catgctttta agaaaatgca tgtacgtcct cgaaaagaaa tcgttgcttt agatttagaa     300
   gatgacgtcg atccaagaca cacaactggc caatatttat cacctgtaga atttagaaaa     360
   gctcttgaag atgatgacac agtcattatt gatgcacgta atgattatga atttgattta     420
   ggtcatttcc gaggtgcaat tcgcccagac atcacacgct ttagagattt acctgactgg     480
   attaaagaga ataaagcgtt atttacagat aaaaaagtgg ttacgtactg tacaggtggc     540
   attcgatgtg aaaaattttc tggatggctt ttaaaagaag gtttcgaaga tgtagcgcag     600
   cttcacggcg gtatagctac atatggtaaa gaccctgaaa caaaaggtca atattgggac     660
   ggtaaaatgt atgtatttga tgatcgtatc agtgttgata tcaaccaagt tgaaaaaaca     720
   attattggta aggattggtt tgatggcaaa ccatgtgaac gttatattaa ttgcgctaac     780
   ccagaatgta ataaacaaat attagtttct gaagaaaacg aaactaaata tttaggtgca     840
   tgctcttatg aatgtgctaa acatgagcgt aatcgttatg ttcaagcaaa taatattagt     900
   gataatgagt ggcaacaacg tttaacaaac tttgatgatt tacatcaaca tgcttag        957
<210> 82
   <211> 654
   <212> DNA
   <213> Staphylococcus aureus
<400> 82
   atggcttcat taaagtcaat catccgtcaa ggtaaacaaa cacgttcaga tcttaaacaa 60
   ttaagaaaat ctggtaaagt accagcagta gtatacggtt acggtactaa aaacgtgtca 120
   gttaaagttg atgaagtaga attcatcaaa gttatccgtg aagtaggtcg taacggtgtt 180
   atcgaattag gcgttggttc taaaactatc aaagttatgg ttgcagacta ccaattcgat 240
   ccacttaaaa accaaattac tcacattgac ttcttagcaa tcaatatgag tgaagaacgt 300
   actgttgaag taccagttca attagttggt gaagcagtag gcgctaaaga aggcggcgta 360
   gttgaacaac cattattcaa cttagaagta actgctactc cagacaatat tccagaagca 420
   atcgaagtag acattactga attaaacatt aacgacagtt taactgttgc tgatgttaaa 480
   gtaactggcg acttcaaaat cgaaaacgat tcagctgaat cagttgtaac agtagttgct 540
   ccaactgaag aaccaactga agaagaaatc gaagctatgg aaggcgaaca acaaactgaa 600
   gaaccagaag ttgttggcga aagcaaagaa gacgaagaaa aaactgaaga gtaa 654
<210> 83
   <211> 762
   <212> DNA
   <213> Staphylococcus aureus
<400> 83
   atgagaacac caattatagc tggtaactgg aaaatgaaca aaacagtaca agaagcaaaa 60
   gacttcgtca atgcattacc aacattacca gattcaaaag aagtagaatc agtaatttgt 120
   gcaccagcaa ttcaattaga tgcattaact actgcagtta aagaaggaaa agcacaaggt 180
   ttagaaatcg gtgctcaaaa tacgtatttc gaagataatg gtgcgttcac aggtgaaacg 240
   tctccagttg cattagcaga tttaggcgtt aaatacgttg ttatcggtca ttctgaacgt 300
   cgtgaattat tccacgaaac agatgaagaa attaacaaaa aagcgcacgc tattttcaaa 360
   catggaatga ctccaattat ttgtgttggt gaaacagacg aagagcgtga aagtggtaaa 420
   gctaacgatg ttgtaggtga gcaagttaag aaagctgttg caggtttatc tgaagatcaa 480
   cttaaatcag ttgtaattgc ttatgagcca atctgggcaa tcggaactgg taaatcatca 540
   acatctgaag atgcaaatga aatgtgtgca tttgtacgtc aaactattgc tgacttatca 600
   agcaaagaag tatcagaagc aactcgtatt caatatggtg gtagtgttaa acctaacaac 660
   attaaagaat acatggcaca aactgatatt gatggggcat tagtaggtgg cgcatcactt 720
   aaagttgaag atttcgtaca attgttagaa ggtgcaaaat aa 762
<210> 84
   <211> 1062
   <212> DNA
   <213> Staphylococcus aureus
<400> 84
   ttatgttaaa actataaggt cttttgtgca tttagtaaag acttgacaac cattttctgt      60
   aattaaaata tcatcttcta ttcttatacc gcccaaacct tctatataaa caccaggttc     120
   tactgtaaca cagttgttaa cttgaagttt atcttgtatc gtacgagcca gcattggccc     180
   ttcatggatt tctaaaccaa taccatgtcc tagtgaatgt ccaaattctt ttccataccc     240
   ttttgactct aaatagtttc ttgaaatggc atcagcttct gcaccagtca tgccaggtct     300
   aatctcatta attgctttca tttgagattc aagtactatt tgatatattt ctttcagttt     360
   aggatctggt tctccaatag caaatgttct agtaatatct gaacaatagc cgttataata     420
   cgcgccaaaa tctaatgtaa tcatgtcgcc tttttcaata attttatcac ttgcaacacc     480
   atgtggtaat gcacctctat gaccagatgc tacaatcgta tcaaatgatg gtccatctgc     540
   tcctaattct agcattttac tttctaatat tgcctttaat tctttttcag tcatacctgc     600
   ttttacaaca gttaaaatat attcatatgt ttcatcaaca atattagctg ctttttgaat     660
   taaagcaatt tcgtcaacat ctttgacgtc tctaatttta tctacagtat tagaaatgct     720
   tattaatgat atacggcttt tatttaattc aaggtatgta tcataactta catgatgccc     780
   ctcaaaacct atattttcaa aattttcttg gtgtagcaat tctttaatct caccaataat     840
   agtagattta cgattaataa tttcataatt tggcgcctgc ttagttgctt gatcaatata     900
   tctaaagtct gttatcaaat attgtttatc tttagatatg ataagtgctc cactggtacc     960
   agtaaaacct gataaatatc ttctattgta atccgaaaga atgataatcg catctaaatg    1020
   tttttgttct aaaatacgat gcacttgtgt tattctgctc at                       1062

## Description

The present invention relates to a pharmaceutical composition or a medicament, notably a protective *Staphylococcus aureus* vaccine, comprising at least one cell wall-associated *Staphylococcus aureus* protein or a fragment or derivative thereof causing an immune response that induces opsonophagocytic activity of human neutrophils for *S. aureus.* The invention further provides particular cell wall-associated *Staphylococcus aureus* proteins and their use.

### Background of the Invention

*Staphylococcus aureus* is a nosocomial as well as a community-acquired pathogen, which causes several diseases, ranging from minor skin infections to serious life-threatening wound infections, bacteraemia, endocarditis, pneumonia and toxic shock syndrome (Lowy F. D., N. Engl. J. Med., 339(8):520-32 (1998)). The worldwide growing incidence of staphylococcal infections is strongly related to the increased use of surgical devices and a growing number of immunocompromised patients. The situation has become more serious, since the increased use of antibiotics led to the emergence of methicillin-resistant *S. aureus* strains (MRSA) (Selvey L. A. et al., Infect. Control. Hosp. Epidemiol., 21(10):645-8 (2000); Peacock J. E. et al., Ann. Intern. Med., 93(4):526-32 (1980)). More recently *S. aureus* isolates with reduced susceptibility to vancomycin (VISA), the antibiotic of choice against MRSA strains, were described (Tenover F. C. et al., Emerg. Infect. Dis., 7(2):327-32 (2001); Tenover F. C. et al., J. Clin. Microbiol., 36(4):1020-7 (1998)), followed by first reports about vancomycin-resistant clinical isolates (VRSA) in 2002 (Staphylococcus aureus resistant to vancomycin--United States, 2002. MMWR Morb Mortal Wkly Rep 2002;51(26):565-7; Palazzo I. C. et al., J. Clin. Microbiol., 43(1):179-85 (2005)). The rising emergence of multidrug-resistant staphylococci led to a growing interest in the development of alternative approaches to prevent and treat staphylococcal infections.

The human humoral (Stranger-Jones Y. K. et al., Proc. Natl. Acad. Sci., U S A, 103(45):16942-7 (2006)) immune response to staphylococcal infection is a crucial component of the host defense (Bredius R. G. et al., J. Immunol., 151(3):1463-72 (1993); Liese J. G. et al., Lancet, 347(8996):220-3 (1996)). Recent investigations on antibody responses to *S. aureus* in patients during the acute phase of infections as well as in healthy individuals showed a high titer of opsonising antibodies against the pathogen in both groups (Dryla A. et al., Clin. Diagn. Lab. Immunol., 12(3):387-98( 2005); Royan S. et al., FEMS Immunol. Med. Microbiol., 29(4):315-21 (2000)). However, antibodies against certain staphylococcal antigens were missing or underrepresented in patient serum, whereas high levels of these antibodies were present in healthy donors (Dryla A. et al., Clin. Diagn. Lab. Immunol., 12(3):387-98( 2005); Clarke S. R. et al., J. Infect. Dis., 193(8):1098-108 (2006)). The humoral immune response elicited during staphylococcal infection does not provide efficient protection against subsequent infections. Likewise, vaccination with whole cells of killed *S. aureus* did not protect animals and humans against staphylococcal infection (Greenberg D. P. et al., Infect. Immun., 55(12):3030-4 (1987); Poole-Warren L. A. et al., Clin. Nephrol., 35(5):198-206 (1991)). Therefore the interest in the design of effective vaccines against *S. aureus* is growing.

Vaccination with capsular polysaccharides (CP) demonstrated reduced severity of staphylococcal infections in animals and humans (Fattom A. et al., Vaccine, 23(5):656-63 (2004); Fattom A. I. et al., Infect. Immun., 64(5): 1659-65 (1996)). Since vaccines based on capsular polysaccharides induce only serotype-specific protection, a bivalent vaccine against *S. aureus* serotype 5 and 8 was developed, which comprises about 75% of the most infectious strains. Due to the poor immunogenicity of polysaccharides, conjugation to a carrier protein was required to induce humoral immune responses. Tested in clinical trials, the CP vaccine elicited high antibody levels in dialysis patients, though not leading to a significant long term protection against bacteraemia (Fattom A. I. et al., Vaccine, 22(7):880-7 (2004)). Alternatively, immunisation with known staphylococcal virulence factors provided a protective immune response against *S. aureus* in animal models. A specific humoral response raised against single extracellular matrix binding proteins like clumping factor A (ClfA), fibronectin-binding protein (FnBP) or collagen-binding protein (Cna) conferred a reduced susceptibility to staphylococcal colonisation (Mamo W. et al., Microbiol. Immunol., 44(5):381-4 (2000); Mamo W. et al., Vaccine, 12(11):988-92 (1994)). Notwithstanding the positive results achieved, vaccination with a single antigen does not suffice to induce full protection against *S. aureus* infection. Additionally, a high variation in the expression pattern of virulence factors among *S. aureus* strains hampers the development of an universal vaccine. Recently is has been shown that a multivalent vaccine consisting of four antigenic determinants provides protection against lethal challenge with *S. aureus* (Stranger-Jones Y. K. et al., Proc. Natl. Acad. Sci., U S A, 103(45):16942-7 (2006)). These data call for the identification of highly antigenic surface proteins conserved among *S. aureus* strains, which could be potential targets for a polyvalent vaccine. Indeed, numerous staphylococcal surface proteins, predicted to be promising antigenic targets, have been identified so far using recently adopted technologies, like proteomics (Brady R. A. et al., Infect. Immun., 74(6):3415-26 (2006); Gatlin C. L. et al., Proteomics, 6(5):1530-49 (2006); Pieper R. et al., Proteomics, 6(15):4246-58 (2006); Vytvytska O. et al., Proteomics, 2(5):580-90 (2002); Nandakumar R. et al., J. Proteome Res., 4(2):250-7 (2005)) or protein selection methods based on expression libraries (Clarke S. R. et al., J. Infect. Dis., 193(8):1098-108 (2006); Etz H. et al., Proc. Natl. Acad. Sci. U S A, 99(10):6573-8 (2002); Weichhart T. et al., Infect. Immun., 71(8):4633-41 (2003); Yang G. et al., Vaccine, 24(8):1117-23 (2006)). Unfortunately, most studies lack functional proof *in vivo.* By serological proteome analysis Vytvytska et al. identified several highly immunogenic proteins using individual sera from patients and healthy donors (Vytvytska O. et al., Proteomics, 2(5):580-90 (2002)).

IVIG preparations are successfully used in the treatment of patients with antibody deficiencies, in order to reduce their high susceptibility to bacterial infections (Lamari F. et al., J. Pharm. Biomed. Anal., 22(6):1029-36 (2000)). Since such preparations consist of purified IgGs derived from not less than thousand plasma donors, they are characterised by a broad spectrum of opsonising antibodies against various pathogens including *S. aureus.* The administration of antibodies against bacterial surface antigens provides opsonisation, complement activation and the phagocytosis of the bacteria (Lamari F. et al., J. Pharm. Biomed. Anal., 22(6):1029-36 (2000); Ono Y. et al., J. Infect. Chemother., 10(4):234-8 (2004)). With regard to *S. aureus* infections, polyclonal IVIG preparations selected for high titers of anti-CIfA IgGs (SA-IGIV) or of anti-CIfA in combination with anti-SdrG IgGs (INH-A21), enhanced bacterial clearance in experimental animals, when used in combination with vancomycin treatment or alone. (Vernachio J. et al., Antimicrob. Agents Chemother., 47(11):3400-6 (2003); Vernachio J. H. et al., Antimicrob. Agents Chemother., 50(2):511-8 (2006)). Even though preparations were selected for high levels of one specific antibody type, they are polyclonal and thus it is unlikely that the protective activity is caused by the interaction of a single type of specific antibodies.

### Summary of the Invention

The world-wide growing incidence of multidrug-resistant *S. aureus* encourages the research for effective vaccination strategies. Since immunisation with vaccines based on single, known components of the staphylococcal virulence apparatus or staphylococcal carbohydrates achieved only partial benefits in prevention of *S. aureus* infections (Mamo W. et al., Microbiol. Immunol., 44(5):381-4 (2000); Mamo W. et al., Vaccine, 12(11):988-92 (1994); Brouillette E. et al., 20(17-18):2348-57 (2002); Zhou H. et al., Vaccine, 24(22):4830-7 (2006)), the identification of novel antigenic proteins remains an important issue. To overcome the limitation of antibody source intravenous immunoglobulin preparations (IVIG) were used that contain a broad spectrum of opsonising antibodies against various pathogens including *S. aureus.* Employing 2-DE gel electrophoresis, subtractive immunoblotting and mass spectrometry several cell wall-associated proteins were identified as novel potential vaccine protein targets. This method for the identification of potential vaccines was coined SUPRA (subtractive proteome analysis). The protective activity of antibodies raised against some of the identified proteins was demonstrated *in vitro* and in a murine sepsis model. The invention thus provides
(1) a pharmaceutical composition or a medicament comprising at least one cell wall-associated *Staphylococcus aureus* protein being the esterase-like protein (hp 2160) of SEQ ID NO: 1, or a fragment or derivative thereof causing an immune response that induces opsonophagocytic activity of human neutrophils for *S. aureus*;
(2) in a preferred embodiment of (1) above, the pharmaceutical composition or medicament further comprises cell wall-associated *Staphylococcus aureus* protein(s) selected from SEQ ID NOs: 2-39 and fragments and derivatives thereof;
(3) in a preferred embodiment of (1) or (2) above the pharmaceutical composition or medicament is a protective *Staphylococcus aureus* vaccine;
(4) the use of the cell wall-associated *S. aureus* protein or a fragment or derivative thereof as defined in (1) above for preparing a protective *Staphylococcus aureus* vaccine;
(5) a diagnostic reagent, a pharmaceutical composition or a medicament comprising at least one antibody against the cell wall-associated *Staphylococcus aureus* protein having SEQ ID NO:1, or a fragment or derivative thereof; and
(6) the use of an antibody against the cell wall-associated *Staphylococcus aureus* protein having SEQ ID NO:1, or a fragment or derivative thereof for preparing a medicament for treating *Staphylococcus aureus* infection in a human or animal.

### Figures

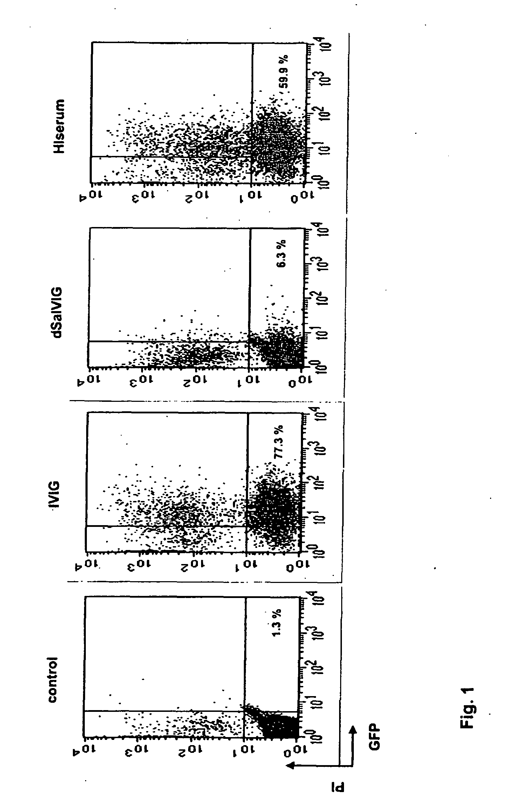

Fig. 1: *In vitro* ops onophagocytosis of GFP-expressing *S. aureus* by human PMNs. Human PMNs, bacteria (MOI 10) and IVIG, dSaIVIG and heat-inactivated serum (HIserum), respectively, were incubated at 37°C for 3 minutes. Flow cytometric analysis revealed the percentage of GFP-positive human PMNs. Dead human PMNs were stained by propidium iodide (PI).

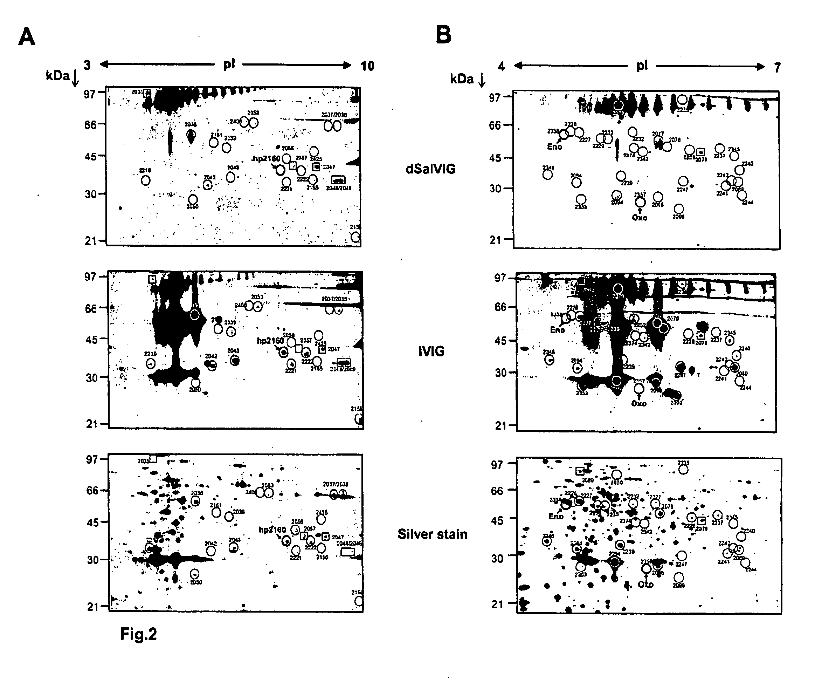

Fig. 2: Differences in the *S. aureus* antigen recognition profiles using IVIG and IVIG depleted of *S. aureus*-specific IgGs (dSaIVIG) for immunoblotting. Cell wall-associated proteins were isolated from *S. aureus* strain ATCC 29213. Proteins were separated according to their isoelectric point (pI) on pI 3-10 IPG strips (A) and on pI 4-7 IPG strips (B). Gels loaded with 100 µg protein were used for subtractive immunoblotting. Spots not immunoreactive with dSaIVIG (top), but with IVIG (middle) represented spots predicted to be promising vaccine candidates (circles). Spots immunoreactive with both, IVIG and dSaIVIG were excluded from further investigation (squares). For MALDI-TOF identification corresponding spots were matched with preparative gels loaded with 500 µg protein (bottom). Circled and numbered spots identified by MALDI-TOF are listed in Table 1.

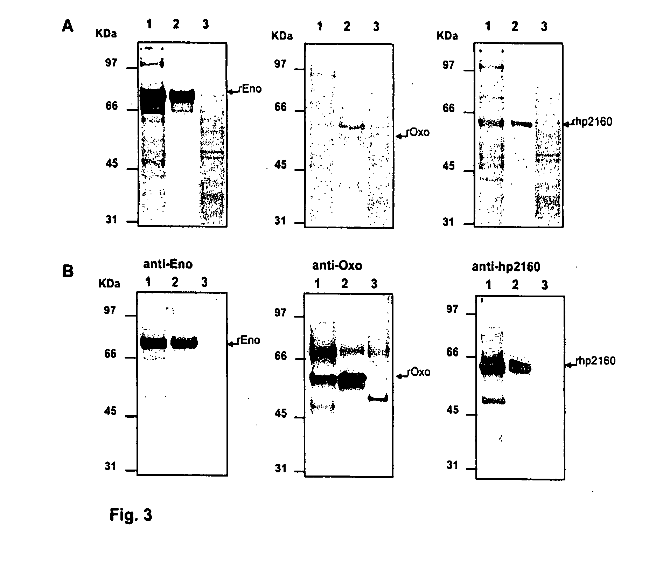

Fig. 3: SDS PAGE of purified recombinant Enolase (rEno), Oxo (rOxo) and hp2160 (rhp2160) stained with silver (A) and specificity control of affinity purified anti-Eno, anti-Oxo and anti-hp2160 antibodies by immunoblotting (B). Cytplasmic protein fraction of *E. coli* expressing recombinant rEno, rOxo or rhp2160 (1), affinity purified rEno, rOxo or rhp2160 protein (2), and cytoplasmic protein fraction of non-transformed *E. coli* (3) were separated by SDS PAGE. 10 µg of cytoplasmic fractions and 2.5 µg of purified proteins were loaded on the gel. Immunoblots were probed with affinity purified anti-Eno, anti-Oxo or anti-hp2160 (1 mg/ml).

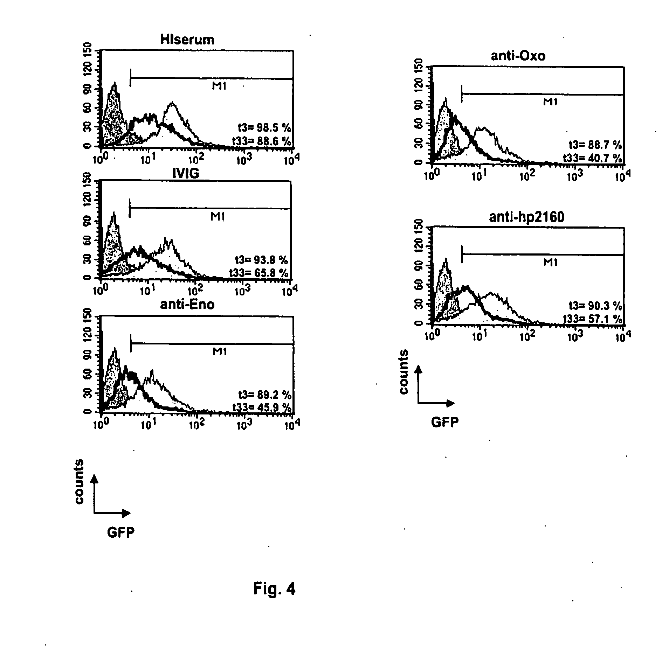

Fig. 4: *In vitro* opsonising activity of purified anti-Eno, -Oxo and -2160 IgGs in comparison to IVIG and heat-inactivated serum (HIserum). Human PMNs, bacteria (MOI 10) and 2.5% HIserum or 500 µg/ml IgGs were incubated for 3 min at 37°C for opsonophagocytosis. Human PMNs and bacteria without opsonins served as negativ control (dark grey). Phagocytosis was stopped by differential centrifugation and the cells were applied to flow cytometric analysis to determine the green fluorescence at t3 (light grey). Incubation was continued at 37°C for further 30 min (t33, transparent), followed by flow cytometric analysis of the human PMNs. The percentage of GFP-positive human PMNs represents cells with internalised GFP-expressing *S. aureus* (M1).

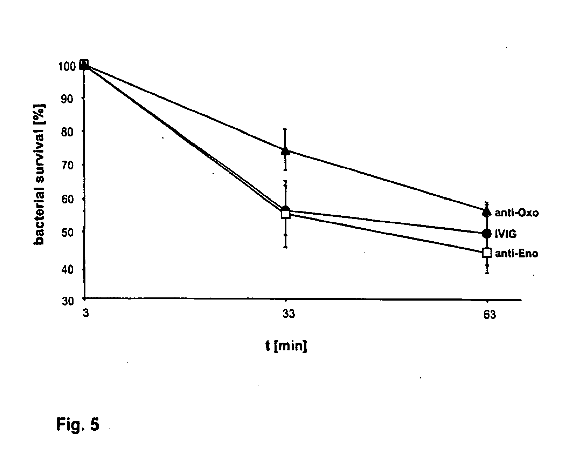

Fig. 5: *In vitro* opsonophagocytic killing of *S. aureus* opsonised with anti-Eno IgGs (open squares), anti-Oxo IgGs (triangles) or IVIG (circles). Human PMNs, bacteria (MOI 1) and 2.5% IgGs (500 µg/ml) were incubated at 37°C for 3 min. Cells were lysed immediately after differential centrifugation (t3) as well as 30 min (t33) and 60 min (t63) later. The mean and SD of triplicates are represented.

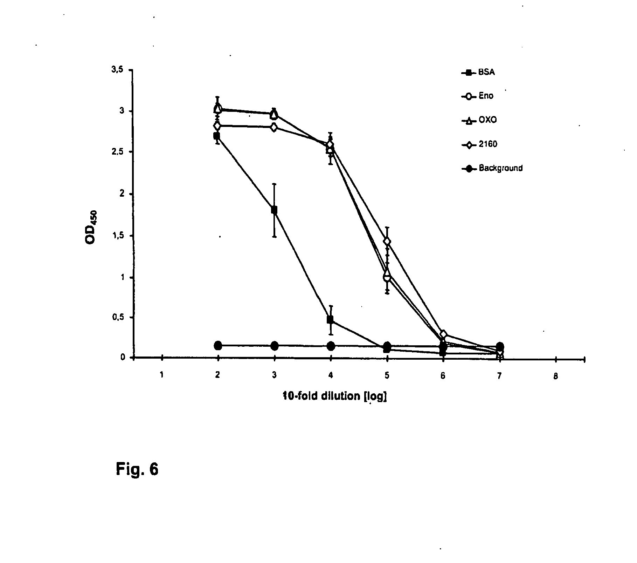

Fig. 6: Detection of antibody levels in serum of mice immunised with rEno (open circles), rOxo (triangles), rhp2160 (diamonds) and BSA (squares) as mock-control using ELISA. 10-fold serial dilutions of sera from each mouse were applied to maxisorb plates coated with 5 µg/ml antigen. As background (circles) signal neg. serum of non-immunised mice was used. Results are expressed as OD at 450 nm. Represented is the mean and SD for a group of 7 mouse sera.

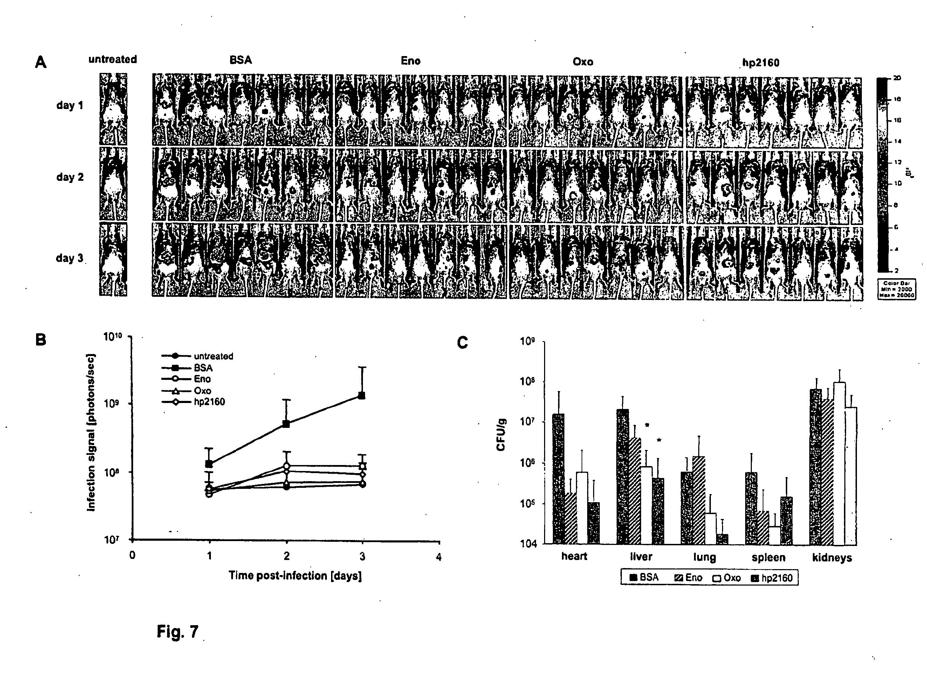

Fig. 7: *In vivo* imaging of *S. aureus* Xen29 infection in C57BL/6 mice immunised with rEno, rOxo, rhp2160 and BSA as mock-control. *S. aureus* Xen 29 (1x108 CFU) was injected i.v. The immunised mice (*n* =7) were shaved, to avoid the loss of photon emission and imaged ventrally. (A). The process of infection was monitored daily by 5-min exposure over a 3-day course by using the IVIS system. (B). Quantitative analysis of the bioluminescence determined as a calculation of a total number of photons/sec within a ROI using the IVIS computer software (untreated, circles; BSA, squares; Eno, open circles; Oxo, triangles; hp2160, diamonds). Represented is mean and SD of a group of seven mice. (C) Bacterial load in organs of immunised mice 3 days after *S. aureus* challenge. Each bar represents mean and SD for a group of 7 mice (BSA, black; Eno, hatched; Oxo, transparent; hp2160, light grey). Asterisks indicate a statistically significant difference compared to BSA controls.

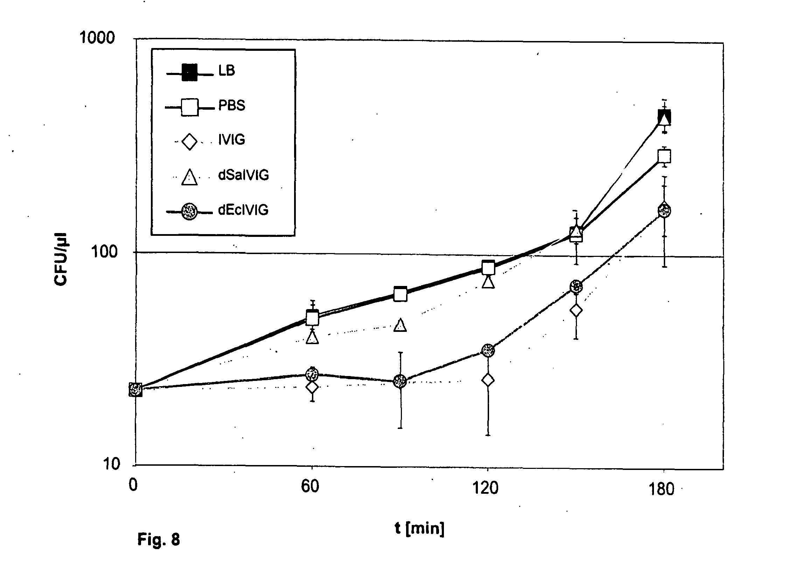

Fig. 8: Bacteriostatic effect of IVIG on *S. aureus* growth. IVIG absorbed with *S. aureus* (dSaIVIG, light grey triangle) did not influence *S. aureus* growth, whereas IVIG absorbed with *E. coli* (dEcIVIG, dark grey circle) still displayed a bacteriostatic effect.

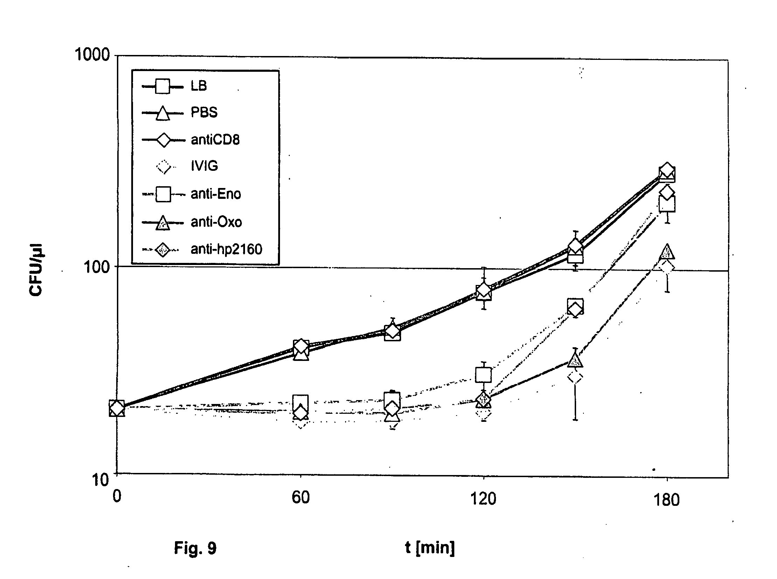

Fig. 9: Bacteriostatic effect of affinity purified anti-Eno (dark grey square), anti-Oxo (dark grey triangle) and anti-hp2160 (dark grey diamond) antibodies on *S. aureus* growth.

### Detailed description of the Invention

As high anti-staphylococcal antibody levels are present in both healthy individuals and acutely infected patients, but with heterogeneous distribution pattern (Dryla A. et al., Clin. Diagn. Lab. Immunol., 12(3):387-98( 2005)), it was assumed that commercially available intravenous immunoglobulin preparation (IVIG) might contain an antibody repertoire of choice for the identification of potential vaccine candidates. Thus, an intravenous immunoglobulin (IVIG) preparation was employed as a source of antibodies directed against *S. aureus* surface proteins for the identification of novel vaccine protein candidates. IVIG induced a strong opsonophagocytic activity of human neutrophils for *S. aureus.* In order to identify proteins that are targeted by IVIG, subtractive proteome analysis (SUPRA) of *S. aureus* surface proteins was performed. Proteins solely reacting with IVIG, but not with IVIG depleted of *S. aureus*-specific opsonising antibodies (dSaIVIG), were identified as vaccine candidates using MALDI-TOF analysis. Three out of 40 identified candidates, enolase (Eno), oxoacyl reductase (Oxo) and hypothetical protein (hp2160), were expressed as GST-fusion proteins, purified and used for the enrichment of corresponding IgGs from IVIG by affinity chromatography. Affinity purified anti-Eno, anti-Oxo and anti hp2160 antibodies showed significant opsonising activity enabling uptake and killing of *S. aureus* by human neutrophils. Significant antibody responses were elicited in mice immunised with recombinant antigens. After challenge with *S. aureus,* reduced staphylococcal spread was detected in immunised mice by *in vivo* imaging system. The recovery of *S. aureus* CFUs from organs of immunised mice was diminished by 10-100-fold. The results of this study suggest our approach to be a valuable tool for the identification of novel vaccine candidates and therapeutic antibodies. Thus, each of these *S. aureus* proteins proved to be able to induce an efficient immune response, establishing protective immunity to *S. aureus.*

In the pharmaceutical composition or medicament as defined in (1) to (3) above the cell wall-associated protein is preferably derived from *S. aureus* strain ATCC 29213 (American type culture collection) (commercially available at ATCC, Manassas, VA (USA)).

Furthermore it is preferred that the cell wall associated protein solely reacts with an intravenous immunoglobulin (iVIG) preparation but not with an iVIG preparation depleted of *S. aureus*-specific opsonising antibodies.

It is particularly preferred that the cell wall-associated protein is selected from the esterase-like protein (hp 2160) of SEQ ID NO: 1, the enolase (Eno) of SEQ ID NO: 2, the 3-oxoacyl-reductase (Oxo) of SEQ ID NO: 3, the NADH dehydrogenase like protein of SEQ ID NO: 4, the anion-binding protein like protein of SEQ ID NO: 5, the peptidoglycan hydrolase of SEQ ID NO: 6, the conserved hypothetical protein of SEQ ID NO: 7, the immunodominant antigen A of SEQ ID NO: 8, the aldehyde dehydrogenase of SEQ ID NO: 9, the truncated beta-hemolysin of SEQ ID NO: 10, the secretory antigen precursor SsaA homolog of SEQ ID NO: 11, the 50S ribosomal protein L13 of SEQ ID NO: 12, the conserved hypothetical protein of SEQ ID NO: 13, the translational elongation factor TU of SEQ ID NO: 14, the autolysin of SEQ ID NO: 15, the hypothetical protein of SEQ ID NO: 16, the protoporphyrinogen oxidase of SEQ ID NO: 17, synergohymenotropic toxin precursor-like protein of SEQ ID NO: 18, the glutamyl-t-RNAGln amidotransferase subunit A of SEQ ID NO: 19, the aminotransferase NifS homologue of SEQ ID NO: 20, the translational elongation factor TU of SEQ ID NO: 21, the hypothetical protein of SEQ ID NO: 22, the immunodominant antigen A of SEQ ID NO: 23, the ATP synthase beta chain of SEQ ID NO: 24, the alanine dehydrogenase of SEQ ID NO: 25, the phosphopentomutase of SEQ ID NO: 26, the NAD-specific glutamate dehydrogenase of SEQ ID NO: 27, autolysin precursor-like protein of SEQ ID NO: 28, the chorismate mutase homolog of SEQ ID NO: 29, the SceD precursor-like protein of SEQ ID NO: 30, the hypothetical protein of SEQ ID NO: 31, the uridylate kinase of SEQ ID NO: 32, the transcription pleiotropic repressor codY of SEQ ID NO: 33, the enterotoxin SEM of SEQ ID NO: 34, the ferrichrome transport ATP-binding protein of SEQ ID NO: 35, the conserved hypothetical protein of SEQ ID NO: 36, the 50S ribosomal protein L25 of SEQ ID NO: 37, the triosephosphate isomerase of SEQ ID NO: 38, the Xaa-Pro dipeptidase of SEQ ID NO: 39 and fragments and derivatives thereof. Particularly preferred among those proteins is the Eno of Seq ID NO: 2, the Oxo of Seq ID NO: 3 , hp2160 of Seq ID NO: 1 or a fragment or derivative thereof.

The term "fragments" within the meaning of the invention comprises a continuous segment out of the above proteins having at least 5 amino acid residues, preferably at least 10 amino acid residues.

The term "derivative" within the meaning of the invention comprises deletion, substitution and addition muteins of the above proteins and combinations thereof. A deletion mutein lacks up to 10, preferably up to 5 continuous or separate amino acid residues, relative to the starting protein sequence. A substitution mutein has substituted up to 10, preferably up to 5 continuous or separate amino acid residues, relative to the starting protein sequence. An addition mutein has added up to 10, preferably up to 5 continuous or separate amino acid residues, relative to the starting protein sequence. The term "derivative" also covers chemical modifications of the protein (e.g. the modification of functional groups, linking of functional groups (such as alkylation, hydroxylation, phosphatation, thiolation, carboxilation and the like), linkage to at least one further functional protein domain (such as marker proteins, carrier proteins, proteins holding adjuvant properties and the like; the linkage being directly or via a linker molecule) and to other biologically active molecules (toxins, antibiotics, lipids, carbohydrates, nucleic acids and the like).

All of the modifications do, however, not reduce the immunogenic activity of the protein.

The pharmaceutical composition or medicament as defined in (1) to (3), (4), (5) and (6) above may further comprise pharmaceutically acceptable carriers (as well as pharmaceutically acceptable binders, stabilizers, excipients, etc.) which are required for the administration of the pharmaceutical composition or medicament to the human or animal patients.

The proteome analysis revealed nearly 40 cell wall-associated vaccine candidates, which were specifically recognised by opsonising IgGs. A number of the identified proteins are already known as staphylococcal virulence factors, like the fibrinogen-binding, bone sialoprotein-binding protein (Yacoub A. et al., Eur. J. Biochem., 222(3):919-25 (1994)), β-hemolysin (Hedstrom S. A. et al., Acta. Pathol. Microbiol. Immunol. Scand. [B], 90(3):217-20 (1982)) or enterotoxin M (SEM) (Jarraud S. et al., J. Immunol., 166(1):669-77 (2001)). Other proteins, like staphylococcal enolase or alanine dehydrogenase, are known as cytosolic proteins, but are also located on the surface of *S. aureus* or other bacterial pathogens (Carneiro C. R. et al., Microbes. Infect., 6(6):604-8 (2004); Rosenkrands I. et al., J. Bacteriol., 184(13):3485-91 (2002)). IsaA and EF-Tu are proteins, already identified by serological proteome analysis (SERPA) (Vytvytska O. et al., Proteomics, 2(5):580-90 (2002)). It is important to note that our selection of *S. aureus* potential vaccine candidates is distinct from 15 *S. aureus* proteins previously identified by Vytvytska et al. employing SERPA (Vytvytska O. et al., Proteomics, 2(5):580-90 (2002)). Unfortunately, these authors provided little information about the potential immunochemical or protective properties of these candidates.

In the study which lead to the invention enolase (Eno), oxoacyl-reductase (Oxo) and the hypothetical protein (hp2160) were repeatedly identified by 2-DE immunoblots. Affinity purified antibodies against each of these proteins were shown to be able to opsonise bacteria, leading to a potent opsonophagocytosis and killing effect *in vitro.* Strong humoral immune response was elicited in mice immunised with the individual proteins. The high level of specific antibodies, detected in mice sera, correlated with the reduction of bacterial infection in vaccinated animals. With regard to these results, the combination of several *S. aureus* antigens is expected to produce even better and more robust immunity. Lately, Enolase was shown to be located on the cell surface of *S. aureus* and to bind extracellular matrix components like laminin (Carneiro C. R. et al., Microbes. Infect., 6(6):604-8 (2004)) and plasminogen (Molkanen T. et al., FEBS Lett., 517(1-3):72-8 (2002)); its important role as staphylococcal virulence factor seems indisputable. Immunisation with recombinant enolase successfully reduced staphylococcal colonisation of *S. aureus.* The protective activity of enolase seems to be not limited to *S. aureus.* Recently it was demonstrated that mice immunised with enolase were protected against S. epidermidis colonisation (Sellman B. R. et al., Infect. Immun., 73(10):6591-600 (2005)). The two other proteins, Oxo and hp2160 are not yet described. Based on sequence homologies, Oxo displays characteristics of enzymes, involved in fatty acid biosynthesis and the hp2160 sequence shows similarity to esterase, an enzyme with lipolytic activity.

It is noteworthy that enolase and other *S. aureus* vaccine candidates have been missed by a recent elegant search for protective vaccine candidates by Stranger-Jones et al. and co-workers employing a bioinformatics-based reverse vaccinology approach (Stranger-Jones Y. K. et al., Proc. Natl. Acad. Sci., U S A, 103(45):16942-7 (2006)). These authors screened multiple staphylococcal genomes for genes containing the conserved LPXTG motif that is common for surface proteins covalently linked to the cell wall. The assumption was that many proteins involved in *S. aureus* pathogenesis are anchored to the bacterial cell wall by a transpeptidation mechanism that requires the C-terminal sorting signal with a conserved LPXTG motif (Navarre W. W. et al., Microbiol. Mol. Biol. Rev., 63(1): 174-229 (1999)). Indeed, mice immunised with a combination of four such antigens, previously tested for their distinct immunogenic properties, were protected against lethal challenge with clinical *S. aureus* isolates. However, numerous novel, anchorless proteins with adhesive and invasive properties that do not contain either a signal peptide or the LPXTG motif were identified in the past few years. The mechanism, by which these proteins are secreted and reassociated with the surface is still unknown (Chhatwal G. S. et al., Trends Microbiol., 10(5):205-8 (2002)). Next to Enolase, a protein exhibiting GAPDH activity and called Tpn was identified on the staphylococcal surface. Tpn was demonstrated to possess transferrin-binding activity (Modun B. et al., Infect. Immun., 67(3):1086-92 (1999)). Another anchorless, broad-spectrum adhesin Eap (Hansen U. et al., Matrix Biol., 25(4):252-60 (2006); Palma M. et al., J. Bacteriol., 181(9):2840-5 (1999)) binds to a large variety of host components, recognising not only fibronectin, fibrinogen or prothrombin, but also collagens, laminin and other extracellular matrix proteins (Palma M. et al., J. Bacteriol., 181(9):2840-5 (1999); McGavin M. H. et al., Infect. Immun., 61(6):2479-85 (1993)). Each of these anchorless proteins was shown to play an important role in the pathogenesis of *S. aureus,* which underscores the continued necessity to search for vaccine candidates lacking the LPXTG motif.

The antibodies utilized in embodiments (5) and (6) above may be polyclonal or monoclonal. Such antibodies employed in the diagnostic reagent, pharmaceutical composition or medicament can be prepared (i.e. raised against the antigen) by suitable methods known to a skilled person.

In the diagnostic reagent of embodiment (5) the antibody may be linked to a suitable detection moiety (such as dye or enzyme), allowing the protein to be deleted. Alternatively, it may also be linked to suitable carrier.

The invention will be further explained in the following Examples.

### Examples

Bacterial strains: *S. aureus* strain ATCC 29213 was obtained from the American Type Culture Collection (ATCC) and was used for the extraction of cell wall-associated proteins. For opsonophagocytosis assays GFP-expressing *S. aureus* (ATCC 29213), generated in our laboratory (Schnaith A. et al., J. Biol. Chem. 282(4):2695-706 (2007)), was used. The bioluminescent *S. aureus* Xen29, applied for *in vivo* imaging studies, was purchased from Xenogen Corp. (Alameda, USA). This strain was derived from the virulent *S. aureus* ATCC 12600 strain and is characterised by its possibility to synthesise the luciferase enzyme as well as its substrate constitutively (Francis K. P. et al., Infect. Immun., 68(6):3594-600 (2000); Kadurugamuwa J. L. et al., Infect. Immun., 71(2):882-90 (2003)). For cloning and expression of recombinant proteins Escherichia coli strains Top 10, DH5α and BL21 (Invitrogen Corp., Karlsruhe, Germany) were used.

Isolation of cell wall-associated proteins: *S. aureus* ATCC 29213 from agar plate was precultured in Luria-Bertani (LB) broth at 37°C with constant shaking to log growth. An aliquot of the preculture was inoculated into 200 ml of LB broth to obtain a starting optical density (OD₆₀₀) of 0.05. The bacteria were cultured with constant shaking at 37°C under aerobic conditions until early exponential growth phase was reached. Bacteria were pelleted by centrifugation at 17,000 x g for 20 min at 4°C and the pellets washed twice in 10 mM TE-buffer, pH 8.0. The cell wall-associated proteins were extracted according to the method of Antelmann (Antelmann H. et al., Proteomics, 2(5):591-602 (2002)) with modifications. The cell pellets were resuspended in 10 ml 1.5 M LiCl, 25 mM Tris-HCl, pH 7.2 containing protease inhibitors (Roche Diagnostics, Mannheim, Germany) and incubated on ice for 30 min. After centrifugation at 17,000 x *g* for 20 min at 4°C, supernatants were pooled and precipitated with 10% w/v trichloroacetic acid (Sigma, Munich, Germany) overnight (ON) at 4°C. The precipitate was collected by centrifugation at 17,000 x *g* for 60 min, and 4°C, washed three times with ice-cold ethanol and dried under vacuum. Extracted proteins were dissolved in 8M urea for two-dimensional gel electrophoresis (2-DE). The protein concentration was determined using the Bio-Rad DC protein assay kit (Bio-Rad, Germany) according to the instructions of the manufacturer.

SDS-PAGE: One-dimensional gel electrophoresis (1-DE) was performed with 10% polyacrylamide gels according to the method of Schägger and Jagow (Schagger H. et al., Anal. Biochem., 166(2):368-79 (1987)) using the Criterion Cell (Bio-Rad). The gels were stained either with Coomassie brilliant blue R250 (CBB) dissolved in 45% ethanol and 15% acetic acid or with silver according to the method described by Shevchenko *et al.* (Shevchenko A. et al., Anal. Chem., 68(5):850-8 (1996)). Western blots were performed using the Criterion Blotter (Bio-Rad) according to the instruction of the manufacturer.

Depletion of *S. aureus* specific IgGs from IVIG: Human intravenous polyclonal immunoglobulin G (IVIG) was purchased from Octapharma (Octagam^{®}, Langenfeld, Germany). To deplete *S. aureus*/*E. coli* specific antibodies from IVIG (dSaIVIG and dEcIVIG, respectively), *S. aureus* (ATCC 29213) or *E. coli* (DH5α) from overnight culture were pelleted by centrifugation at 17,000 x g for 20 min, 4°C, washed twice in 1x PBS and the pellet was resuspended in IVIG. The suspension was rotated slowly overnight at 4°C. Bacteria were removed by centrifugation and the supernatant was passed through a 0.2 µm membrane filter, followed by the determination of the protein concentration.

Subtractive proteome analysis (SUPRA): 2-DE was performed according to the method described by Bernardo *et al.* (Bemardo K. et al., Antimicrob. Agents Chemother., 48(2):546-444 (2004)) using the Multiphor II (Pharmacia-FRG) system according to the instructions of the manufacturer. Proteins dissolved in 8 M urea were separated on 18 cm immobilised pH gradient (IPG) strips in a nonlinear pH range of 3-10 and 4-7 (GE Healthcare, Munich, Germany). Isoelectric focusing (IEF) was performed using 500 µg protein for coomassie and 100 µg for silver gels and western blots. Proteins were solubilised in rehydration buffer (8 M urea, 2 M thiourea, 40 mM DTT, 1% CHAPS, 0.5% Pharmalyte and separated according to their pIs (isoelectric point) using the Multiphor II. After IEF, rod gels were equilibrated first in equilibration buffer (50 mM Tris-HCL [pH 8.8], 8 M urea, 2 M thiourea, 30% glycerol, 2% SDS, 0.05% bromophenol blue) containing 3.5 mg/ml DTT, followed by equilibration in equilibration buffer containing 45 mg/ml iodoacetamide and applied to SDS-PAGE. The proteins were separated on 12.5% Tris-glycine SDS gels (25 cm, 20 cm, 1.0 mm) using the Ettan Dalt II system (GE Healthcare).

Immunoblotting: Proteins separated by 2-DE were transferred to nitrocellulose membranes using the Trans-Blot Cell (Bio-Rad) according to the instruction of the manufacturer. After blocking in 5% low-fat dry milk and 2% BSA dissolved in TBST (Tris-buffered saline-Tween 20, pH 7.5) for 1h at room temperature, membranes were probed with IVIG (1:500) or with IVIG depleted of *S. aureus* specific IgGs (dSaIVIG) using an equal antibody concentration, overnight at 4°C. Membranes were washed in TBST and incubated with anti-human IgG peroxidase conjugate (1:2500) for specific detection of immune complexes. After subsequent washing in TBST, signals were developed using the ECL detection system (GE Healthcare). Each membrane was used twice. After treatment with dSaIVIG, membrane was stripped at 50°C using stripping buffer containing 62.5 mM Tris-HCl pH 6,8; 2% SDS; 100 mM β-Mercaptoethanol, and incubated with IVIG.

Signals of interest were matched with corresponding spots on the CBB stained preparative gel, excised and digested with trypsin (Bernardo K. et al., Antimicrob. Agents Chemother., 48(2):546-444 (2004)). MALDI-TOF/MS analysis was performed using the Bruker REFLEX IV mass spectrometer (Bruker-Daltonik, Leipzig, Germany). Further data processing was done using the XMASS 5.1.1 postanalysis software. Protein mass spectra were analysed using the MASCOT software against a public database (NCBI)

Cloning of GST-fusion proteins: The recombinant proteins, Enolase (rEno; SEQ ID NO: 2), Oxoacyl-Reductase (rOxo; SEQ ID NO: 3) and the hypothetical protein hp2160 (rhp2160; SEQ ID NO: 1) were cloned as N-terminal GST-fusion proteins using the Gatetway Technology (Invitrogen) according to the instructions of the manufacturer. The open reading frame (ORF) of each gene was amplified from S. *aureus* (ATCC 29213) chromosomal DNA by PCR using 5'-CACCATGCCAATTATTA CAGATG-3' (SEQ ID NO: 40) and 5'-TTATTTATCT AAGTTATAGAA TGATTTG-3' (SEQ ID NO: 41), 5'-CACCATGAAA ATGACTAAGA GTGCT-3' (SEQ ID NO: 42) and 5'-TACATGTACA TTCCACCATT TACATG-3' (SEQ ID NO: 43), and 5'-CACCTTGATT AGAAACCGTG TTATG-3' (SEQ ID NO: 44) and 5'-TTAGTTATTT TGTGTTACAT CCTCATC-3' (SEQ ID NO: 45) for Eno, Oxo and hp2160, respectively. The reaction products were cloned into pENTR/D-TOPO and transformed into *E. coli* TOP10 (Invitrogen). By LR recombination the genes of interest were transferred from entry clone into pDEST15 destination vector, in order to generate a vector coding for the N-terminal GST-fusion protein. After transformation of the destination vector into *E. coli* DH5α, positive clones were identified by restriction analysis and transformed into the *E. coli* BL21 strain for expression.

Expression and purification of staphylococcal antigens: *E. coli* BL21 harbouring the recombinant plasmids were grown in LB medium containing ampicillin at 30°C until the cultures reached an OD₆₀₀ of ∼ 0.6. The protein expression was induced with 0.5 mM IPTG for 4 h at 27°C. Bacterial cells were harvested by centrifugation, washed with 1xPBS and resuspended in 1xPBS containing protease inhibitors and lysed mechanically using the French Press (Thermo Scientific, Waltham, US). The bacterial debris was removed by subsequent centrifugation at 17,000 x g. The expression of soluble protein was assessed by SDS-PAGE. The recombinant proteins were purified by affinity chromatography using glutathione sepharose prepacked GSTrap FF columns on the *ÄKTApurifier* liquid chromatography system (GE Healthcare) according to the manufacturers instructions.

Affinity absorption of specific IgGs from IVIG: In order to enrich specific IgGs, 5-10 mg purified recombinant protein (rEno, rOxo, rhp2160) were covalently linked to the NHS-activated Sepharose according to the manufacturers instructions (GE Healthcare). The absorption of IgGs was performed ON at 4°C by slow recirculation of IVIG previously transferred into PBS buffer (pH 7.3) using the HiPrep 26/10 Desalting column (GE Healthcare).

Specifically bound IgGs were eluted from the column using 0.1 M Glycine-HCL, pH 2.7 on the ÄKTA*purifier* liquid chromatography system. The pH of the eluted fractions was neutralised by collecting them into a sufficient amount of 1M Tris-HCL, pH 9. IgG containing fractions were pooled and after buffer exchange into 1 x PBS, pH 7.3 concentrated using Centricon Plus-70 centrifugal filter units with a 30.000 MWCO membrane (Millipore, Schwalbach, Germany).

*In vitro* opsonophagocytosis and bactericidal assay: Human polymorphonuclear cells (PMNs) were isolated by dextran sedimentation and Ficoll-Hypaque gradient centrifugation using 50 ml of heparinised blood from a healthy donor according to standard protocols. Extracted PMNs were resuspended in PBS containing 10 mM glucose, pH7.3, followed by the determination of cell number by trypan blue counting.

For the opsonophagocytosis assay GFP-expressing *S. aureus* ATCC 29213 from ON culture was inoculated into 25 ml of LB broth and grown at 37°C to an OD₆₀₀ of 0.3. Bacteria were harvested, washed and adjusted to 1x10⁹ CFU/ml.

IVIG, dSaIVIG or enriched specific IgGs (anti-Eno, -Oxo and -hp2160) were applied in a concentration of 500 µg/ml, which corresponds to the average IgG concentration present in 2.5% human serum.

IgGs, 2.5 x 10⁶ human PMNs and bacteria in a MOI (multiplicity of infection) of 10 were incubated for 60 s at 37°C with slow rotation, pelleted by centrifugation for 60 s at 400 x g at 37°C and further incubated for 60 s at 37°C, in order to synchronise phagocytosis. Pellets were resuspended and the phagocytosis was stopped by differential centrifugation for 5 min at 150 x g at 4°C. Immediately after centrifugation (t3) an aliquot was removed and stored in a 1:5 dilution in 0.5% BSA/PBS, pH7.3 at 4°C until FACS analysis was performed. Incubations were continued for further 30 min at 37°C. As a reference value, bacterial uptake in presence of 2.5% heat-inactivated human serum was investigated. Heat-labile complement was destroyed by incubation of the serum for 30 min at 56°C. Triplicates were analysed using the FACSCalibur immunocytometry system and the CELLQuestPro software (BD Biosciences).

To investigate the bactericidal effect induced by IVIG and enriched specific IgGs, 2.5 x 10⁶ human PMNs and 2.5 x 10⁶ bacteria (MOI 1) were applied for the killing assay. PMNs, bacteria and IgGs were treated, as described above, until samples immediately (t3) as well as 30 (t33) and 60 minutes after differential centrifugation (t63) were collected. An aliquot of each sample was diluted 1:10 in sterile H₂O and 0.1% Triton X 100 and incubated on ice for 5 min to lyse PMNs. Dilutions of triplicates were spread via spiral plater (Eddy Jet, IUL Instr., Königswinter, Germany) on Miller Hinton (MH) agar plates and the CFU number was determined according to the instructions of the manufacturer. To calculate the percentage of surviving bacteria after 33 and 63 min, CFUs present at t33 or t63 were divided by CFUs present at t3, multiplied by 100.

Immunisation: Female C57/ BL6 mice (6-8 weeks) were purchased from Charles River Laboratories (Sulzfeld, Germany). Groups of 7 mice were administered intraperitoneally (i.p.) with an 1:1 emulsion of 100 µg recombinant protein (rEno, rOxo or rhp2160) and complete Freund's Adjuvant (Sigma) in a total volume of 200 µl on day 0, followed by subcutaneous (s.c.) administration of three booster doses using an emulsion of 50 µg antigen and incomplete Freund's Adjuvant (1:1) on day 14, day 28 and day 42. Mice immunised with an emulsion of BSA and adjuvant served as controls. Blood samples were taken from each mouse one week after the last booster injection, in order to determine the antibody titer.

Detection of antibody levels: Enzyme-linked immunoabsorbent assay (ELISA) was used to determine levels of antibodies directed against Eno, Oxo and hp2160 protein in sera of immunised mice. 96-well polystyrene maxisorb plates (Nunc, Wiesbaden, Germany) were coated overnight at 4°C with 5 mg/ml of the recombinant antigen (rEno, rOxo, rhp2160), BSA or GST in PBS, pH 7.3. The wells were washed three times with PBS containing 0.05% Tween 20 (PBST) and blocked using StartingBlock T20/PBS (Pierce) for 30 min at RT. Triplicates of ten-fold serial dilutions of serum in blocking buffer were incubated for 2 h at RT. After subsequent washing, the bound antibodies were detected with the peroxidase-conjugated goat anti-mouse IgG (Sigma) incubating the samples for 2h at RT and using the tetramethylbenzidine system (BD Biosciences, Heidelberg, Germany) as substrate according to the manufacturers instructions. The enzyme reaction was stopped by addition of 2N H₂SO₄, followed by measuring the absorbance at OD₄₅₀ using the ELISA plate reader (MRX TC, Dynex Technology, Kaiserslautern, Germany).

Mice sepsis model: The infection of mice was performed using the bioluminescent *S. aureus* Xen29 strain (Francis K. P. et al., Infect. Immun., 68(6):3594-600 (2000); Kadurugamuwa J. L. et al., Infect. Immun., 71(2):882-90 (2003)). Prior to infection the mice were shaved ventrally to enable light detection. The animals were challenged intravenously (i.v.) with an inoculum of 1 x 108 CFU *S. aureus* Xen29 two weeks after the last booster injection. Bacterial inoculum was assessed by plating a serial dilution on MH agar plates and counting the CFU. The infected mice were monitored daily over a time course of 3 days post infection in an anaesthetised state (isofluoran-oxygen gas mixture) using the IVIS imaging system (Xenogen Corp.). Mice were imaged in ventral position for 5 min exposure. An untreated control mouse was included in every image to detect the background photon emission. The infection rate was displayed in a pseudocolour scale representing the number of photons per second emitted from the mice. Highly intensive bioluminescence signals were displayed as red and low intensity signals as blue. The light intensity within defined regions of interest (ROIs) was quantified using the LivingImage software (Xenogen Corp.). Bioluminescent signals were quantified by setting the ROIs over the ventral side of the animal body. Extremities, tail and head were excluded from the analysis.

Bacterial load in organs: To determine the number of bacteria within the organs, mice were sacrificed on day 3 subsequent to the IVIS image. Target tissues (liver, kidney, spleen, heart and lung) were weighed and homogenised in 2 ml PBS containing 0.05% Triton X 100. Ten-fold serial dilutions were spread on MH agar plates and the CFU number was determined.

Statistical analysis: Student's t-tests were performed to analyse the statistical significance of bacterial load in organs. Differences with p < 0.05 were considered as statistically significant.

*In vitro* Bacteriostatic effect: *S. aureus* ATCC 29213 from ON culture was inoculated into 25 ml of LB broth and grown at 37°C to an OD₆₀₀ of 0.3. Bacteria were harvested, washed and adjusted to 1x10⁹ CFU/ml. IVIG, dSaIVIG, dEcIVIG or enriched specific IgGs (anti-Eno, -Oxo and -hp2160) were added to LB in a concentration of 200 µg/ml in 500µl PBS. The prewarmed media were inoculated with 8x103 CFU/ml and the cultures were incubated for 180 min at 37°C. Samples were taken at 0, 60, 90, 120, 150 and 180 minutes post inoculation. Assays without IgGs but LB or PBS or with unspecific murine antiCD8 IgGs served as controls. An aliquot of each sample was diluted 1:2 in 0.1% Triton^{®} X 100 in sterile H₂O and incubated in a sonicator bath for 5 min. Dilutions of triplicates were spread via spiral plater (Eddy Jet, IUL Instr., Königswinter, Germany) on Miller Hinton (MH) agar plates and the CFU number was determined according to the instructions of the manufacturer.

### Results

IVIG contains opsonising antibodies to *S.aureus:* To determine whether commercial IVIG preparations are suitable antibody sources for the identification of immunoreactive antigens, an IVIG preparation (Octagam^{®}) was tested *in vitro* for opsonising antibodies to *S. aureus*. The presence of opsonising antibodies was investigated in a PMNs-mediated opsonophagocytosis assay. The efficacy of complement-independent IVIG-mediated phagocytosis was compared to phagocytosis induced by 2.5% heat-inactivated human serum (HIserum). Flow cytometry revealed that IVIG induces strong phagocytic activity of 77.3% human PMNs, which was slightly higher than that mediated by HIserum (59.9%). Phagocytosis was nearly completely abolished, when IVIG was preabsorbed with *S. aureus* (dSaIVIG) (Fig. 1). These observations provide clear evidence, that opsonising antibodies are abundant in IVIG.

Detection and identification of immunogenic staphylococcal surface proteins by 2-DE analysis: In order to identify antigenic proteins, recognised by IgGs present in IVIG, cell wall associated proteins from *S. aureus* (clinical isolate ATCC 29213) were separated by 2-DE electrophoresis, followed by Western blot analysis using IVIG and dSaIVIG. To achieve high-quality spot resolution and to ensure adequate spot matching and identification, proteins were separated in two different pH ranges, pH 3-10 (Fig. 2A) and pH 4-7 (Fig. 2B), in series of three gels in parallel for each pH range. One of the gels was used for immunoblotting, probing the same blot twice, first with dSaIVIG (Fig. 2, top) and then IVIG (Fig. 2, middle). IVIG and dSaIVIG produced reproducibly distinct immunoblot profiles of 2-DE separated *S. aureus* surface proteins. The depletion of *S. aureus*-specific, opsonising IgGs by absorption of IVIG with *S. aureus* resulted in a considerably lower number of spots compared to immunoblots treated with IVIG (Fig. 2, top and middle). We assumed that proteins solely recognised by complete, but not with dSaIVIG may serve as vaccine candidates. Vice versa, proteinspots strongly reacting with both, IVIG and dSaIVIG, were supposed to be recognised by IgGs, that lack specificity for *S. aureus* antigens, and were therefore excluded from further investigation. For spot matching and identification, the two other gels were stained either with silver (Fig. 2, bottom) or coomassie (not shown). Spots of interest were cut from the coomassie stained gel and used for MALDI-TOF analysis. Eventually, about 40 proteins were identified using SUPRA (Table 1).

Expression of recombinant proteins and purification of specific antibodies: Three highly immunogenic proteins Enolase (Eno), oxoacyl-reductase (Oxo) and the hypothetical protein 2160 (hp2160) were repeatedly identified in 2-DE analysis. In order to prove their ability to trigger protective immune responses, these proteins were cloned, expressed as soluble GST-fusion proteins in *E. coli* and purified by affinity chromatography. The purity of the recombinant proteins was confirmed by SDS-PAGE (Fig. 3A).

To demonstrate the opsonic activity of antibodies against rEno, rOxo and rhp2160, corresponding antibodies from IVIG were purified by affinity chromatography. The specificity of enriched antibodies was analysed by immunoblotting. Western blots of purified antigens, cytoplasmic fractions of recombinant protein expressing *E. coli* as well as cytoplasmic fractions of non-transformed *E. coli* (BL21) were probed with affinity purified antibody fractions. As shown in Fig. 3B, each antibody fraction was able to recognise the corresponding antigen specifically: rEno (75.5 kDa), rOxo (54.5 kDa) and rhp2160 (64.0 kDa)

Opsonic effect of anti- Eno, -Oxo and -hp2160 antibodies: The opsonic activity of the affinity purified antibodies against rEno, rOxo and rhp2160 (anti-Eno, -Oxo and -hp2160) was analysed in a human PMNs-mediated opsonophagocytosis assay. The uptake of GFP-expressing *S. aureus* by human PMNs in presence of specific antibodies was monitored by flow cytometry. The opsonophagocytic activity mediated by the individual antibodies was compared to opsonophagocytic activity induced by IVIG and HIserum. As shown in Fig. 4, antibodies to any of the selected antigens were able to induce strong bacterial uptake by human PMNs. Within 3 min, 88-90% human PMNs stained positive for *S. aureus* indicating robust opsonising activity triggered by the affinity purified antibodies. Comparable phagocytosis rates were obtained for IVIG (∼93.8%) as well as for HIserum (98.5%). Phagocytosis was not observed in absence of IgGs.

The percentage of GFP-positive human PMNs was significantly reduced in all samples, when measured 30 minutes after termination of phagocytosis (t33). To elucidate whether the reduction of green fluorescent PMNs was caused by the elimination of bacteria, viable intracellular *S. aureus* were quantified by CFU counting. As shown in Fig. 5, IVIG as well as specific antibodies are able to induce bacterial killing by PMNs. Using IVIG and anti-Eno as opsonins, 60% of the initial CFUs were recovered after 30 min (t33), further decreasing to 45% (anti-Eno) and 50% (IVIG) surviving bacteria after additional 30 min incubation (t63). When anti-Oxo served as opsonin, 75% of the initial CFU number was recovered at t33 and the PMNs mediated killing effect resulted in 58% surving bacteria 30 min later (t63).

Immunisation with rEno, rOxo and rhp2160 induces significant levels of specific anti-*S.aureus* antibodies: Encouraged by the fact that anti-Eno, Oxo and hp2160 were able to induce opsonophagocytic killing of *S. aureus* by human PMNs *in vitro,* we used the recombinant proteins to induce a protective immune response to *S. aureus* infection in mice. Groups of 7 mice were immunised either with rEno, rOxo or rhp2160 and BSA as control. One week after the third administration of the booster injection the level of specific antibodies in serum was determined using ELISA. High levels of anti-Eno, -Oxo, -hp2160 and -BSA antibodies were detected in the serum of immunised mice. The anti-BSA antibody titer in mock-immunised mice was nearly 100-fold lower (Fig. 6).

Protection of rEno, rOxo and rhp2160 immunised mice against *S. aureus* infection: To determine whether the immune response elicited by rEno, rOxo and rhp2160 was able to protect mice against *S. aureus*, immunised mice were challenged intravenously (i.v.) with the virulent, bioluminescent *S. aureus* Xen 29 strain with an inoculum of 10⁸ CFU. Mice were monitored by IVIS (*in vivo imaging system*) to follow the spreading of bacteria from day 1 to day 3 of infection. The *in vivo* bioluminescence images of the ventral side of an uninfected control, mock-immunised and recombinant protein-immunised mice are shown in Fig. 7A. Whereas a progressive increase of bioluminescence was observed in mice immunised with BSA, no significant increase of bioluminescence was detected in mice treated with rEno, rOxo or rhp2160. Quantitative analysis of emitted photons per second counted within the "region of interest" (ROI) for each imaged mouse is shown in Fig. 7B. Nearly constant bioluminescence values were observed in vaccinated mice over the course of infection, whereas a significant proliferation of bacteria, resulting in up to 10-fold increase of the bioluminescent signals, was measured in the group of mock-immunised mice.

In order to quantify the bacterial load in different organs by an independent method, CFUs of *S. aureus* in liver, kidneys, heart, lung and spleen were determined.

Vaccination with rEno, rOxo or rhp2160 resulted in remarkably lower *S. aureus* densities in all target tissues compared to the bacterial load in animals receiving BSA (Fig. 7C). The most obvious difference was observed in liver and heart tissue. In the liver the hp2160 immunised group revealed a 100-fold lower colonisation rate compared to the controls, followed by Oxo with a 10-fold lower bacterial load and finally Eno with 5-fold less bacteria. The *in vivo* data demonstrate that the immunisation with all three candidates (Eno, Oxo and hp2160) induces a protective immune response, which reduces an uncontrolled spread of *S. aureus* infection in mice.

**Table 1: Cell wall-associated immunogenic antigens from S. aureus ATCC 29213 identified by MALDI-TOF**

| SEQ ID NO | GenBank Acc. No. | Spot ID | Putative identification | p*I*^{a} | MW^{b}(kDa) | MAS COT^{c}Tot. score | Seq. coverage^{d} (%) |
|---|---|---|---|---|---|---|---|
| 1 | gil15927930 | 2160 | esterase-like protein (hp2160) | 7.2 | 35.6 | 92 | 28 |
| 2 | gi\|15926453 | 2338 | enolase (Eno) | 4.6 | 47.1 | 206 | 47 |
| 3 | gi\|15926814 | 2357 | 3-oxoacyl-(acyl-carrier protein) reductase (Oxo) | 5.6 | 26.2 | 103 | 35 |
| 4 | gi\|15926530 | 2036/2078 | hypothetical protein, | 5.4 | 44.4 | 79 | 29 |
| 5 | gi\|15926396 | 2037/2038 | hypothetical protein, | 9.0 | 74.4 | 248 | 38 |
| 6 | gi\|2239274 | 20392345 | peptidoglycan hydrolase | 6.1 | 35.2 | 63 | 22 |
| 7 | gi\|15926560 | 2042 | conserved hypothetical protein | 5.6 | 31.8 | 121 | 48 |
| 8 | gi\|15928148 | 2043/2050 | immunodominant antigen A | 6.1 | 24.2 | 80 | 23 |
| 9 | gi\|14247692 | 2053 | aldehyde dehydrogenase | 6.6 | 51.9 | 56 | 15 |
| 10 | gi\|21205110 | 2056 | truncated beta-hemolysin | 8.8 | 37.5 | 105 | 42 |
| 11 | gi\|15927879 | 2155 | secretory antigen precursor SsaA homolog | 9.0 | 29.4 | 112 | 40 |
| 12 | gi\|15927798 | 2158 | 50S ribosomal protein L13 | 9.3 | 16.3 | 228 | 66 |
| 13 | gi\|15926780 | 2161 | conserved hyp. protein | 5.7 | 34.6 | 186 | 45 |
| 14 | gi\|15926226 | 2219/2227 | translational elongation factor TU | 4.7 | 43.1 | 98 | 33 |
| 15 | gil15924043 | 2221 | autolysin | 9.7 | 102.6 | 77 | 11 |
| 16 | gi\|21205078 | 2222 | hypothetical protein | 5.8 | 65.5 | 63 | 13 |
| 17 | gi\|14247604 | 2406 | protoporphyrinogen oxidase | 6.3 | 52.2 | 126 | 23 |
| 18 | gil15927580 | 2425 | synergohymenotropic toxin precursor-like protein | 8.6 | 38.7 | 78 | 14 |
| 19 | gil6578924 | 2070 | glutamyl-t-RNAGln | 5.0 | 52.3 | 143 | 36 |
| 20 | gi\|15926504 | 2077 | aminotransferase NifS homolog | 5.3 | 46.4 | 346 | 60 |
| 21 | gi\|15926226 | 2084 | translational elongation factor TU | 4.4 | 29.6 | 153 | 29 |
| 22 | gi\|15923845 | 2089 | hypothetical protein | 5.7 | 29.4 | 113 | 38 |
| 23 | gi\|15928148 | 2094/2099 | immunodominant antigen A | 5.9 | 24.2 | 84 | 23 |
| 24 | gi\|15927677 | 2226 | ATP synthase beta chain | 4.7 | 51.4 | 317 | 55 |
| 25 | gi\|21204821 | 2228 | alanine dehydrogenase | 5.6 | 40.1 | 268 | 55 |
| 26 | gi\|15925843 | 2229/2230 | phosphopentomutase | 5.0 | 43.8 | 229 | 60 |
| 27 | gi\|15926547 | 2232 | NAD-specific glutamate dfehyd rogenase | 5.2 | 45.9 | 255 | 50 |
| 28 | gi\|15928230 | 2235 | autolysin precursor-like protein | 6.0 | 69.2 | 72 | 16 |
| 29 | gi\|14247509 | 2237 | chorismate mutase homolog | 5.8 | 40.7 | 112 | 34 |
| 30 | gi\|15927670 | 2239 | SceD precursor-like protein | 5.5 | 24.1 | 70 | 34 |
| 31 | gi\|15923892 | 2240 | hypothetical protein | 6.5 | 37.1 | 89 | 19 |
| 32 | gi\|15926841 | 2241 | uridylate kinase | 6.0 | 26.3 | 88 | 26 |
| 33 | gi\|15926838 | 2242 | transcription pleiotropic | 5.9 | 28.7 | 178 | 49 |
| 34 | gi\|14247601 | 2244 | enterotoxin SEM | 6.5 | 27.5 | 68 | 28 |
| 35 | gi\|15926324 | 2247 | ferrichrome transport ATP- | 5.6 | 29.7 | 99 | 28 |
| 36 | gi\|15928275 | 2342 | conserved hypothetical protein | 5.3 | 37.5 | 188 | 33 |
| 37 | gi\|15926178 | 2346 | 50S ribosomal protein L25 | 4.4 | 23.8 | 104 | 34 |
| 38 | gi\|15926451 | 2353 | triosephosphate isomerase | 4.8 | 27.4 | 66 | 32 |
| 39 | gi\|13701328 | 2374 | Xaa-Pro dipeptidase | 5.2 | 39.6 | 241 | 36 |

| | | | | | | | |
|---|---|---|---|---|---|---|---|
| a) Theoretical pI b) Theoretical molecular weight (Da) c) Identification probability of the fragment match d) Sequence coverage of the total amino acid number | | | | | | | |

Specific IgGs exhibit a bacteriostatic effect on *S. aureus* growth *in vitro*: In order to analyse whether *S. aureus* specific IgGs could influence bacterial growth CFU numbers were compared over a period of three hours. As shown in figure 8 IVIG exhibits a bacteriostatic effect on *S. aureus* growth during the first two hours post inoculation. A comparable effect was observed when IVIG preabsorbed with *E. coli* (dEcIVIG) was used, whereas IVIG depleted of specific IgGs(dSaIVIG) did not influence the growth of *S. aureus.* These data indicate that the bacteriostatic effect is mediated exclusively by specific IgGs. In the next step enriched individual IgGs were analysed for their ability to influence bacterial growth. Figure 9 shows that each of the specific IgGs inhibits the growth of *S. aureus.* Anti-Eno treated assays start growing after 90 min, whereas those treated with anti-Oxo are comparable to IVIG.

### Sequence Listing - Free Text

SEQ ID NOs: 1 to 39 *S. aureus* cell wall-associated proteins (Table 1)
SEQ ID NOs: 40 to 45 primer
SEQ ID NOs: 46 to 84 DNA sequences coding for SEQ ID NOs: 1-39

## Claims

1. A pharmaceutical composition or a medicament comprising at least one cell wall-associated *Staphylococcus aureus* protein being the esterase-like protein (hp 2160) of SEQ ID NO: 1, or a fragment or derivative thereof causing an immune response that induces opsonophagocytic activity of human neutrophils for *S*. *aureus,* wherein the derivative has a deletion, substitution and/or addition of up to 10 amino acids, or has a chemical modification.

2. The pharmaceutical composition or medicament of claim 1, wherein the cell wall-associated protein
(i) is derived from *S*. *aureus* strain ATCC 29213; and/or
(ii) solely reacts with an intravenous immunoglobulin (IVIG) preparation but not with an IVIG preparation depleted of *S*. *aureus*-specific opsonising antibodies.

3. The pharmaceutical composition or medicament of claim 1 or 2, wherein the composition further comprises *S*. *aureus* cell wall-associated protein(s) selected from the enolase (Eno) of SEQ ID NO: 2, the 3-oxoacyl-reductase (Oxo) of SEQ ID NO: 3, the NADH dehydrogenase like protein of SEQ ID NO: 4, the anion-binding protein like protein of SEQ ID NO: 5, the peptidoglycan hydrolase of SEQ ID NO: 6, the conserved hypothetical protein of SEQ ID NO: 7, the immunodominant antigen A of SEQ ID NO: 8, the aldehyde dehydrogenase of SEQ ID NO: 9, the truncated beta-hemolysin of SEQ ID NO: 10, the secretory antigen precursor SsaA homolog of SEQ ID NO: 11, the 50S ribosomal protein L13 of SEQ ID NO: 12, the conserved hypothetical protein of SEQ ID NO: 13, the translational elongation factor TU of SEQ ID NO: 14, the autolysin of SEQ ID NO: 15, the hypothetical protein of SEQ ID NO: 16, the protoporphyrinogen oxidase of SEQ ID NO: 17, synergohymenotropic toxin precursor-like protein of SEQ ID NO: 18, the glutamyl-t-RNAGIn amidotransferase subunit A of SEQ ID NO: 19, the aminotransferase NifS homologue of SEQ ID NO: 20, the translational elongation factor TU of SEQ ID NO: 21, the hypothetical protein of SEQ ID NO: 22, the immunodominant antigen A of SEQ ID NO: 23, the ATP synthase beta chain of SEQ ID NO: 24, the alanine dehydrogenase of SEQ ID NO: 25, the phosphopentomutase of SEQ ID NO: 26, the NAD-specific glutamate dehydrogenase of SEQ ID NO: 27, autolysin precursor-like protein of SEQ ID NO: 28, the chorismate mutase homolog of SEQ ID NO: 29, the SceD precursor-like protein of SEQ ID NO: 30, the hypothetical protein of SEQ ID NO: 31, the uridylate kinase of SEQ ID NO: 32, the transcription pleiotropic repressor codY of SEQ ID NO: 33, the enterotoxin SEM of SEQ ID NO: 34, the ferrichrome transport ATP-binding protein of SEQ ID NO: 35, the conserved hypothetical protein of SEQ ID NO: 36, the 50S ribosomal protein L25 of SEQ ID NO: 37, the triosephosphate isomerase of SEQ ID NO: 38, the Xaa-Pro dipeptidase of SEQ ID NO: 39 and fragments and derivatives thereof.

4. The pharmaceutical composition or medicament of any one of claims 1 to 3, wherein the fragments comprises at least 10 amino acids.

5. The pharmaceutical composition or medicament of any one of claims 1 to 4, wherein the derivatives include modifications of functional groups, linkage of functional groups, linkage to at least one further functional protein domain and linkage to other biologically active molecules.

6. The pharmaceutical composition or medicament of any one of claims 1 to 5, which is a protective *Staphylococcus aureus* vaccine.

7. Use of the cell wall-associated *S*. *aureus* protein having SEQ ID NO:1, or a fragment or derivative thereof for preparing a protective *Staphylococcus aureus* vaccine, wherein the derivative has a deletion, substitution and/or addition of up to 10 amino acids or has a chemical modification.

8. A diagnostic reagent, a pharmaceutical composition or a medicament comprising at least one antibody against the cell wall-associated *Staphylococcus aureus* protein having SEQ ID NO:1, or a fragment or derivative thereof, wherein the derivative has a deletion, substitution and/or addition of up to 10 amino acids or has a chemical modification.

9. Use of an antibody against the cell wall-associated *Staphylococcus aureus* protein having SEQ ID NO:1, or a fragment or derivative thereof for preparing a medicament for treating *Staphylococcus aureus* infection in a human or animal, wherein the derivative has a deletion, substitution and/or addition of up to 10 amino acids or has a chemical modification.

## Patentansprüche

1. Pharmazeutische Zusammensetzung oder Medikament, umfassend wenigstens ein zellwandassoziiertes *Staphylococcus-aureus-Protein,* bei dem es sich um das esteraseartige Protein (hp 2160) der SEQ ID Nr. 1 oder ein Fragment davon oder Derivat davon handelt, das eine Immunantwort verursacht, die bei humanen Neutrophilen eine opsonophagozytische Aktivität gegenüber *S. aureus* induziert, wobei das Derivat eine Deletion, Substitution und/oder Addition von bis zu 10 Aminosäuren aufweist oder eine chemische Modifikation aufweist.

2. Pharmazeutische Zusammensetzung oder Medikament gemäß Anspruch 1, wobei das zellwandassoziierte Protein
(i) vom *S*.-*aureus*-Stamm ATCC 29213 abgeleitet ist; und/oder
(ii) ausschließlich mit einem intravenösen Immunglobulin(IVIG)-Präparat, aber nicht mit einem IVIG-Präparat, das an *S*.-*aureus*-spezifischen opsonisierenden Antikörpern abgereichert ist, reagiert.

3. Pharmazeutische Zusammensetzung oder Medikament gemäß Anspruch 1 oder 2, wobei die Zusammensetzung weiterhin ein oder mehrere *S*.-*aureus*-zellwandassoziierte Proteine umfasst, die aus der Enolase (Eno) der SEQ ID Nr. 2, der 3-Oxoacyl-Reductase (Oxo) der SEQ ID Nr. 3, dem NADH-Dehydrogenase-artigen Protein der SEQ ID Nr. 4, dem anionbindendes-Protein-artigen Protein der SEQ ID Nr. 5, der Peptidoglycan-Hydrolase der SEQ ID Nr. 6, dem konservierten hypothetischen Protein der SEQ ID Nr. 7, dem immundominanten Antigen A der SEQ ID Nr. 8, der Aldehyd-Dehydrogenase der SEQ ID Nr. 9, dem verkürzten beta-Hämolysin der SEQ ID Nr. 10, dem sekretorisches-Antigen-Vorläufer-SsaA-Homologen der SEQ ID Nr. 11, dem 50S-ribosomalen Protein L13 der SEQ ID Nr. 12, dem konservierten hypothetischen Protein der SEQ ID Nr. 13, dem translationalen Elongationsfaktor der SEQ ID Nr. 14, dem Autolysin der SEQ ID Nr. 15, dem hypothetischen Protein der SEQ ID Nr. 16, der Protoporphyrinogen-Oxidase der SEQ ID Nr. 17, dem synergohymenotropisches-Toxin-Vorläufer-artigen Protein der SEQ ID Nr. 18, der Glutamyl-t-RNAGln-Amidotransferase-Untereinheit A der SEQ ID Nr. 19, dem Aminotransferase-NifS-Homologen der SEQ ID Nr. 20, dem translationalen Elongationsfaktor TU der SEQ ID Nr. 21, dem hypothetischen Protein der SEQ ID Nr. 22, dem immundominanten Antigen A der SEQ ID Nr. 23, der ATP-Synthase-beta-Kette der SEQ ID Nr. 24, der Alanin-Dehydrogenase der SEQ ID Nr. 25, der Phosphopentomutase der SEQ ID Nr. 26, der NAD-spezifischen Glutamat-Dehydrogenase der SEQ ID Nr. 27, dem Autolysin-Vorläufer-artigen Protein der SEQ ID Nr. 28, dem Chorismat-Mutase-Homologen der SEQ ID Nr. 29, dem SceD-Vorläufer-artigen Protein der SEQ ID Nr. 30, dem hypothetischen Protein der SEQ ID Nr. 31, der Uridylat-Kinase der SEQ ID Nr. 32, dem Transcriptionspleiotropen Repressor codY der SEQ ID Nr. 33, dem Enterotoxin SEM der SEQ ID Nr. 34, dem Ferrichrom-Transport-ATP-bindenden Protein der SEQ ID Nr. 35, dem konservierten hypothetischen Protein der SEQ ID Nr. 36, dem 50S-ribosomalen Protein L25 der SEQ ID Nr. 37, der Triosephosphat-Isomerase der SEQ ID Nr. 38, der Xaa-Pro-Dipeptidase der SEQ ID Nr. 39 und Fragmenten und Derivaten davon ausgewählt sind.

4. Pharmazeutische Zusammensetzung oder Medikament gemäß einem der Ansprüche 1 bis 3, wobei die Fragmente wenigstens 10 Aminosäuren umfassen.

5. Pharmazeutische Zusammensetzung oder Medikament gemäß einem der Ansprüche 1 bis 4, wobei die Derivate Modifikationen von funktionellen Gruppen, Verknüpfung von funktionellen Gruppen, Verknüpfung mit wenigstens einer weiteren funktionellen Proteindomäne und Verknüpfung mit anderen biologisch aktiven Molekülen umfassen.

6. Pharmazeutische Zusammensetzung oder Medikament gemäß einem der Ansprüche 1 bis 5, bei der bzw. dem es sich um ein schützendes *Staphylococcus-aureus-Vakzin* handelt.

7. Verwendung des zellwandassoziierten S.-aureus-Proteins mit der SEQ ID Nr. 1 oder eines Fragments oder Derivats davon zur Herstellung eines schützenden *Staphylococcus-aureus-Vakzins,* wobei das Derivat eine Deletion, Substitution und/oder Addition von bis zu 10 Aminosäuren aufweist oder eine chemische Modifikation aufweist.

8. Diagnostisches Reagens, pharmazeutische Zusammensetzung oder Medikament, umfassend wenigstens einen Antikörper gegen das zellwandassoziierte *Staphylococcus-aureus-Protein* mit der SEQ ID Nr. 1 oder ein Fragment oder Derivat davon, wobei das Derivat eine Deletion, Substitution und/oder Addition von bis zu 10 Aminosäuren aufweist oder eine chemische Modifikation aufweist.

9. Verwendung eines Antikörpers gegen das zellwandassoziierte *Staphylococcus-aureus-Protein* mit der SEQ ID Nr. 1 oder ein Fragment oder Derivat davon zur Herstellung eines Medikaments zu Behandlung einer *Staphylococcus-aureus-Infektion* bei einem Mensch oder Tier, wobei das Derivat eine Deletion, Substitution und/oder Addition von bis zu 10 Aminosäuren aufweist oder eine chemische Modifikation aufweist.

## Revendications

1. Composition pharmaceutique ou médicament comprenant au moins une protéine de *Staphylococcus aureus* associée à la paroi cellulaire qui est la protéine de type estérase (hp 2160) de SEQ ID NO: 1, ou un fragment ou dérivé de celle-ci déclenchant une réponse immunitaire qui induit une activité opsonophagocytaire des neutrophiles humains sur S. *aureus,* où le dérivé présente une délétion, une substitution et/ou une addition d'un nombre d'acides aminés allant jusqu'à 10, ou présente une modification chimique.

2. Composition pharmaceutique ou médicament selon la revendication 1, dans laquelle/lequel la protéine associée à la paroi cellulaire
(i) est dérivée de la souche de *S. aureus* ATCC 29213; et/ou
(ii) réagit uniquement avec une préparation d'immunoglobuline intraveineuse (IVIG) mais pas avec une préparation d'IVIG appauvrie en anticorps opsonisants, spécifiques de *S. aureus.*

3. Composition pharmaceutique ou médicament selon la revendication 1 ou 2, ladite composition comprenant en outre une ou plusieurs protéine(s) associée(s) à la paroi cellulaire de *S. aureus,* choisies parmi l'énolase (Eno) de SEQ ID NO: 2, la 3-oxoacyl-réductase (Oxo) de SEQ ID NO: 3, la protéine de type NADH-déshydrogénase de SEQ ID NO: 4, la protéine de type protéine de liaison anionique de SEQ ID NO: 5, la peptidoglycane-hydrolase de SEQ ID NO: 6, la protéine hypothétique conservée de SEQ ID NO: 7, l'antigène A immunodominant de SEQ ID NO: 8, l'aldéhyde déshydrogénase de SEQ ID NO: 9, la bêta-hémolysine tronquée de SEQ ID NO: 10, l'homologue de SsaA précurseur d'antigène sécrétoire de SEQ ID NO: 11, la protéine ribosomique 50S L13 de SEQ ID NO: 12, la protéine hypothétique conservée de SEQ ID NO: 13, le facteur d'élongation traductionnel TU de SEQ ID NO: 14, l'autolysine de SEQ ID NO: 15, la protéine hypothétique de SEQ ID NO: 16, la protoporphyrinogène-oxydase de SEQ ID NO: 17, la protéine de type précurseur de toxine synergohyménotrope de SEQ ID NO: 18, la sous-unité A de la glutamyl-t-RNAGIn-amidotransférase de SEQ ID NO: 19, l'homologue de l'aminotransférase NifS de SEQ ID NO: 20, le facteur d'élongation traductionnel TU de SEQ ID NO: 21, la protéine hypothétique de SEQ ID NO: 22, l'antigène A immunodominant de SEQ ID NO: 23, la chaîne bêta d'ATP-synthase de SEQ ID NO: 24, l'alanine-déshydrogénase de SEQ ID NO: 25, la phosphopentomutase de SEQ ID NO: 26, la glutamate-déshydrogénase NAD-spécifique de SEQ ID NO: 27, la protéine de type précurseur d'autolysine de SEQ ID NO: 28, l'homologue de chorismate-mutase de SEQ ID NO: 29, la protéine de type précurseur de SceD de SEQ ID NO: 30, la protéine hypothétique de SEQ ID NO: 31, l'uridylate-kinase de SEQ ID NO: 32, le répresseur de transcription pléiotrope codY de SEQ ID NO: 33, l'entérotoxine SEM de SEQ ID NO: 34, la protéine de transport de ferrichrome se liant à l'ATP de SEQ ID NO: 35, la protéine hypothétique conservée de SEQ ID NO: 36, la protéine ribosomique 50S L25 de SEQ ID NO: 37, la triosephosphate-isomérase de SEQ ID NO: 38, la Xaa-Pro dipeptidase de SEQ ID NO: 39 et les fragments et dérivés de ceux-ci.

4. Composition pharmaceutique ou médicament selon l'une quelconque des revendications 1 à 3, où les fragments comprennent au moins 10 acides aminés.

5. Composition pharmaceutique ou médicament selon l'une quelconque des revendications 1 à 4, où les dérivés englobent les modifications de groupes fonctionnels, la liaison de groupes fonctionnels, la liaison à au moins un domaine de protéine fonctionnelle supplémentaire et la liaison à d'autres molécules biologiquement actives.

6. Composition pharmaceutique ou médicament selon l'une quelconque des revendications 1 à 5, qui est un vaccin de protection contre *Staphylococcus aureus.*

7. Utilisation de la protéine de *S*. *aureus* associée à la paroi cellulaire ayant SEQ ID NO: 1, ou d'un fragment ou dérivé de celle-ci pour la préparation d'un vaccin de protection contre *Staphylococcus aureus,* où le dérivé présente une délétion, une substitution et/ou une addition d'un nombre d'acides aminés allant jusqu'à 10, ou présente une modification chimique.

8. Réactif de diagnostic, composition pharmaceutique ou médicament comprenant au moins un anticorps dirigé contre la protéine de *Staphylococcus aureus* associée à la paroi cellulaire ayant SEQ ID NO: 1, ou l'un de ses fragments ou dérivés, où le dérivé présente une délétion, une substitution et/ou une addition d'un nombre d'acides aminés allant jusqu'à 10, ou présente une modification chimique.

9. Utilisation d'un anticorps dirigé contre la protéine de *Staphylococcus aureus* associée à la paroi cellulaire ayant SEQ ID NO: 1, ou un fragment ou dérivé de celle-ci, pour la préparation d'un médicament destiné au traitement d'une infection par le *Staphylococcus aureus* chez un humain ou un anomal, où le dérivé présente une délétion, une substitution et/ou une addition d'un nombre d'acides aminés allant jusqu'à 10, ou présente une modification chimique.
